(19) Europäisches Patentamt / European Patent Office / Office européen des brevets

(11) **EP 2 618 727 B1**

(12) **EUROPEAN PATENT SPECIFICATION**

(45) Date of publication and mention of the grant of the patent:
**22.06.2022 Bulletin 2022/25**

(21) Application number: **11827551.0**

(22) Date of filing: **22.09.2011**

(51) International Patent Classification (IPC):
**A61B 5/06** (2006.01)

(52) Cooperative Patent Classification (CPC):
**A61B 5/065; A61B 5/283; A61B 5/349;**
A61B 5/061; A61B 5/316; A61B 5/6852;
A61B 5/6869

(86) International application number:
**PCT/US2011/052793**

(87) International publication number:
**WO 2012/040487 (29.03.2012 Gazette 2012/13)**

(54) **APPARATUS AND METHOD FOR CATHETER NAVIGATION USING ENDOVASCULAR ENERGY MAPPING**

VORRICHTUNG UND VERFAHREN ZUR KATHETERNAVIGATION MIT ENDOVASKULÄRER ENERGIEKARTIERUNG

APPAREIL ET PROCÉDÉ DESTINÉS À ASSISTER LA NAVIGATION D'UN CATHÉTER EN UTILISANT LA CARTOGRAPHIE DE L'ÉNERGIE ENDOVASCULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.02.2011 US 201113019939**
**23.09.2010 US 344732 P**

(43) Date of publication of application:
**31.07.2013 Bulletin 2013/31**

(73) Proprietor: **C. R. Bard, Inc.**
**Franklin Lakes, NJ 07417 (US)**

(72) Inventor: **GRUNWALD, Sorin**
**Bucarest 21918 (RO)**

(74) Representative: **Hoffmann Eitle**
**Patent- und Rechtsanwälte PartmbB**
**Arabellastraße 30**
**81925 München (DE)**

(56) References cited:
| | |
|---|---|
| WO-A1-2009/100158 | US-A- 5 121 750 |
| US-A- 5 910 120 | US-A- 5 957 857 |
| US-A1- 2002 123 679 | US-A1- 2008 097 232 |
| US-A1- 2009 177 090 | US-A1- 2009 259 124 |
| US-A1- 2009 259 124 | US-A1- 2010 036 227 |
| US-B1- 6 368 285 | US-B2- 6 577 896 |

- **MAURO PITTIRUTI ET AL: "The electrocardiographic method for positioning the tip of central venous catheters", THE JOURNAL OF VASCULAR ACCESS, vol. 12, no. 4, 30 May 2011 (2011-05-30), pages 280-291, XP055309521, IT ISSN: 1129-7298, DOI: 10.5301/JVA.2011.8381**
- **GJENDEMSJO, A. ET AL.: 'Energy and Power' CONNEXIONS. 20 February 2004, XP008172078 Retrieved from the Internet: <URL:http://cnx.org/content/m11526/1.201> [retrieved on 2011-12-19]**

**Description**

## BACKGROUND

The electrical conduction system of the heart creates specific electrical signals, electrical energy distributions and behaviors thereof which are indicative of specific locations in the thoracic cavity and/or of specific heart functions or conditions. When measured endovascularly, i.e., from within blood vessels or from within the heart, certain parameters of the electrical activity of the heart can be used to identify specific locations in the cardiovascular system and/or functional conditions, normal or abnormal. Moreover, by locally and accurately identifying the location and the type of condition, therapy of such conditions can be optimized and the effect of the therapy monitored in real-time.

Two types of clinical applications are typically addressed. The first is related to guiding endovascular devices through the cardiovascular system, while the second is related to the non-invasive or the minimally invasive remote monitoring of the electrical activity of the heart.

The guidance, positioning, and placement confirmation of endovascular catheters are necessary in a number of clinical applications, such as, for example:

1. Central venous access, e.g., CVC, PICC, implantable ports;

2. Hemodialysis catheters;

3. Placement of pacemaker leads;

4. Hemodynamics monitoring catheters, e.g., Swan-Ganz and central pressure monitoring catheters; and

5. Guiding guidewires and catheters into the left heart.

The location of the catheter tip is very important to the patient safety, the duration and the success of the procedure. Today's golden standard for confirming the target location of the catheter tip is the chest X-ray. In addition, there are currently two types of real-time guiding products available on the market, which try to overcome the limitations of chest X-ray confirmation: electromagnetic and ECG-based. In hospitals where real-time guidance is used results have improved in terms of reducing the number of X-rays, the procedure time, and the cost of the procedure. Under real-time guidance first-time success rate has typically increased from 75%-80% to 90%-95%. In addition, in hospitals where ECG guidance is used, e.g., in Italy, Belgium, Germany, chest X-ray confirmation has been eliminated for more than 90% of the patients. Electromagnetic systems are used mostly in the United States while ECG-based systems are used mostly in Europe. Amongst other factors which determine the difference between the markets in the United States and Europe in terms of technology adoption: a) type of health care personnel allowed to perform procedures: nurses have more flexibility in the United States, b) type of devices placed: PICCs are placed more and more often in the United States, c) price sensitivity: the European market seems to be more price sensitive, and d) the current guiding devices are commercialized by specific manufacturers to work exclusively with their catheters: market penetration of the guiding systems reflects the market penetration of the catheter manufacturer.

It was also found that different opinions exist regarding where the target tip location should be: for example, lower third of the SVC or RA. Therefore guiding technologies should allow for discrimination of these locations. The chest X-ray, which is the current golden standard does not always allow for such discrimination requiring an accuracy of typically better than 2 cm. Also, because ECG-based systems make use of physiological information related to the heart activity, their ability to guide placement is accurate with respect to the anatomy. This is not the case with electromagnetic guiding systems which measure the distance between the catheter tip in the vasculature and an external reference placed typically on the patient's chest. Because of this aspect, ECG-based systems can be used to document the final result of the catheter placement potentially replacing the chest X-ray as the golden standard.

One of the most valuable diagnostic tools available, the ECG records the heart's electrical activity as waveforms. By interpreting these waveforms, one can identify rhythm disturbances, conduction abnormalities, and electrolyte imbalance. An ECG aids in diagnosing and monitoring such conditions as acute coronary syndromes and pericarditis. The heart's electrical activity produces currents that radiate through the surrounding tissue to the skin. When electrodes are attached to the skin, they sense these electrical currents and transmit them the electrocardiograph. Because the electrical currents from the heart radiate to the skin in many directions, electrodes are placed at different locations on the skin to obtain a total picture of the heart's electrical activity. The electrodes are then connected to an electrocardiograph device, or computer, and record information from different perspectives, which are called leads and planes. A lead provides a view of the heart's electrical activity between two points or poles. A plane is a cross section of the heart which provides a different view of the heart's electrical activity. Currently, the interpretation of an ECG waveform is based on identifying waveform component amplitudes, analyzing and then comparing the amplitudes with certain standards. Modifications of these amplitude components are indicative of certain conditions, e.g., the elevation of the ST segment or of certain locations in the heart, e.g., the amplitude of the P-wave. In today's practice ECG monitors are widely used to record ECG waveforms. More and more often applications are made available for automatic identification of the ECG amplitude components. In certain cases tools are available for decision making support and for automatic interpretation of ECG amplitude components with respect to underlying heart conditions.

Remote patient monitoring is a well established medical field. Still remote monitoring of heart conditions is not as widely accepted as it would be need and possible. One of the reasons is related to the relatively complicated way of acquiring signals related to the heart activity, in particular ECG signals. Another important limiting factor of the current remote monitoring technologies is the use of communications channels, like the telephone line, which are difficult to interface with at both the patient and the physician ends.

WO 2009/100158 A1 is directed to a method of locating the tip of a central venous catheter, which catheter includes at least one electrode which is used to detect a voltage and to generate a P-wave, for which then a reference deflection value is determined when the tip is within the proximal superior vena cava. The tip is then advanced and a new deflection value is determined. A ratio of the new and reference deflection values is used to determine the tip location.

## BRIEF SUMMARY

Briefly summarized, embodiments of the present invention are directed to systems and methods for obtaining and using endovascular electrograms (or electrocardiograms/ECGs) in a number of clinical applications and settings. For example, the devices can be used to guide endovascular devices in and around the heart, e.g., guiding central venous access devices in the superior vena cava, right atrium, and right ventricle. Such central venous access devices may include central venous catheters (CVC), peripherally inserted central catheters (PICC), implantable ports, hemodialysis catheters, tunneled catheters and others.

One or several skin electrodes are used to obtain skin surface ECG signals simultaneous with the acquisition of endovascular (intracavitary) electrogram (ECG) signals via the use of endovascular (intracavitary) electrodes. The simultaneous and synchronized skin surface and endovascular ECG signals are used in one of several ways to analyze and quantify the ECG signals as a function of the location of the endovascular electrode, e.g. as a function of the tip of a catheter.

In light of the above, in one embodiment the ease-of-use of the ECG-based catheter navigation and tip location is enhanced. In one aspect, for instance, skin ECG reference waveforms are simultaneously presented on a display with endovascular ECG waveforms measured at the tip of a catheter or other indwelling medical device. Such simultaneous acquisition and display of concurrent ECG signals allows for ready interpretation of the endovascular ECG waveform at the tip of the catheter. In another aspect, a skin ECG reference signal is used to synchronize information processing algorithms applied to the endovascular ECG signal, yielding results of enhanced reliability concerning changes of P-wave of the endovascular ECG signal in terms of shape and energy.

In greater detail, in one embodiment a skin ECG signal can be used as a reference and compared to an endovascular ECG signal in order to detect changes in the endovascular ECG relative to the skin ECG.

In another embodiment, analysis of the synchronized skin and endovascular ECG signals can be linked to one another and/or to the periodic electrical activity of the heart. For example, a skin ECG lead can be used to detect the R-peak of the QRS complex of a detected skin ECG waveform. Detection of the R-peak in the skin ECG waveform can be used to trigger analysis of the endovascular ECG signal in a simultaneously corresponding segments of the endovascular ECG waveform, e.g., in the segment corresponding to the P-wave. Such triggering is particularly useful in case of arrhythmia, wherein the skin ECG waveform does not typically demonstrate a consistent P-wave, while the endovascular ECG waveform indeed includes a detectable P-wave segment that changes as a function of the location in the vasculature.

In another embodiment, a skin ECG lead can be used to monitor the patient's heart activity at the same time an endovascular ECG lead is employed to guide a catheter or other suitable indwelling or endovascular devices through the vasculature. In another embodiment, R-peaks detected in the skin ECG waveform are used to trigger correlation computation and other types of signal processing on the endovascular ECG signal in order to allow for efficient noise reduction in the resultant endovascular ECG waveform.

In another aspect, a connector for establishing an operable connection between a catheter in the sterile field of the patient and an ECG cable outside of the sterile field is described, allowing for single operator utilization of the apparatus for catheter navigation and tip location introduced herein.

In another aspect, algorithms are introduced that allow for mapping certain ECG waveforms to corresponding locations in the vasculature. In one embodiment, the algorithm analyzes directional electrical energy present at the tip of a catheter or other endovascular device capable of detecting endovascular ECG signals. In another embodiment the algorithm can map the catheter tip to a certain location in the vasculature based on endovascular ECG signals so as to allow for catheter navigation.

In another aspect, a simplified graphical user interface is disclosed, depicting a moving graphical indicator over a heart icon so as to indicate a location of a catheter tip in the vasculature as determined by the endovascular ECG signal. The graphical indicator can include different colors and shapes, such as dots or arrows, for instance. The colors and shapes of the graphic indicator may change as a function of the tip location in the vasculature.

In another aspect, an ECG signal acquisition module is disclosed that is operably connectable, via a suitable interface, to a mobile phone or other portable electronic device. This enables control of the ECG signal acquisition module, including ECG signal analysis, by a user of the mobile phone. In another embodiment, the ECG signal acquisition module can be operably connected interfaced to other handheld or remote devices.

In another aspect, a user interface is included for use in connection with the mobile phone or other portable device to enable ECG signal-based guiding of endovascular devices by the mobile phone. In another embodiment, the user interface enables use of the mobile phone to support analysis and archival of ECG signals, catheter information, and results of a catheter placement procedure. In another embodiment, the user interface optimizes ECG signal acquisition for remote patient monitoring via the mobile phone or other handheld device.

In another aspect not according to the invention, a method is disclosed for locating an indwelling medical device within a vasculature of a patient. The method comprises identifying an endovascular ECG waveform complex from an endovascular ECG signal associated with the indwelling medical device, then calculating an absolute value of the energy of the endovascular ECG waveform complex over a predetermined segment thereof. A position of the medical device within the vasculature is then determined by observation of the absolute value of the energy of the predetermined segment of the endovascular ECG waveform complex.

The invention is defined by the appended claims.

## BRIEF DESCRIPTION OF THE DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:

FIG. 1A is a block diagram that depicts an apparatus according to an embodiment of the present invention.

FIG. 1B is a block diagram of an electronic module for acquisition and processing of endovascular electrocardiogram according to an embodiment of the present invention.

FIG. 2 depicts an adaptor for an endovascular device according to an embodiment of the present invention.

FIG. 3 depicts a catheter steering device according to an embodiment of the present invention.

FIGS. 4A, 4B, 4C, and 4D depict electrode configurations that provide optimal acquisition of endovascular electrocardiogram according to various embodiments of the present invention. FIG. 4A depicts a single lead configuration, FIG. 4B depicts a modified 3-lead configuration with monitoring and guiding capabilities, FIG. 4C depicts a telemetry configuration with a single grounded lead, and FIG. 4D depicts one use of ECG monitors for guiding endovascular devices.

FIG. 5 illustrates exemplary electrocardiogram signal amplitudes at different locations in the central venous system.

FIG. 6 illustrates exemplary electrocardiogram signal power spectra at different locations in the central venous system.

FIG. 7 illustrates exemplary electrocardiogram signal electrical energy distribution at different locations in the central venous system.

FIG. 8 depicts a graphical user interface according to an embodiment of the present invention.

FIG. 9 depicts a graphical user interface according to another embodiment of the present invention.

FIGS. 10A and 10B depict exemplary printouts for the information displayed by the graphical user interface, according to an embodiment of the present invention.

FIG. 11 is a block diagram for a computer-based method for positioning an endovascular device in or near the heart using electrocardiogram signals.

FIG. 12 illustrates another decision support algorithm for a computer-based method for positioning an endovascular device in or near the heart using electrocardiogram signals, according to one embodiment.

FIG. 13 illustrates the cardiac conduction system of the heart.

FIG. 14 illustrates electrical signal propagation in the conduction system of the heart.

FIG. 15 illustrates electrical activity in the cardiovascular system due to neuronal control system.

FIG. 16 illustrates a framework for analyzing the endovascular electrography signals, according to an embodiment of the present invention.

FIG. 17 illustrates several embodiments for electrogram waveform processing.

FIG. 18A shows ECG leads arranged to form an Einthoven triangle.

FIGS. 18B-18F show various views of a skin ECG waveform and an endovascular ECG waveform as depicted on a graphical user interface according to one embodiment.

FIGS. 19A and 19B show various views of a skin ECG waveform and an endovascular ECG waveform as depicted on a graphical user interface according to one embodiment.

FIGS. 20A and 20F show various views of a skin ECG waveform and an endovascular ECG waveform as depicted on a graphical user interface according to one embodiment.

FIGS. 21A and 21B show various views of a skin ECG waveform and an endovascular ECG waveform as depicted on a graphical user interface according to one embodiment.

FIGS. 22A-22D show various magnetic sterile connectors according to certain embodiments.

FIGS. 23A and 23B show various steerable sterile connectors according to certain embodiments.

FIGS. 24A-24F show various views of a skin ECG waveform and an endovascular ECG waveform together with a heart icon to indicate a position of an endovascular device as depicted on a graphical user interface according to one embodiment.

FIGS. 25A and 25B show various possible depictions for use in ECG signal-based guidance as displayed on a mobile phone according to one embodiment.

FIG. 26 shows a depiction of the zooming of multiple ECG waveforms as displayed on a mobile phone according to one embodiment.

FIGS. 27A and 27B show additional ECG waveform-related depictions as displayed on a mobile phone according to one embodiment.

FIG. 28 is a view of skin and endovascular ECG waveforms together with additional elements as depicted on a graphical user interface according to one embodiment.

FIG. 29 is a view of skin and endovascular ECG waveforms together with additional elements as depicted on a graphical user interface according to one embodiment.

FIG. 30 is a view of skin and endovascular ECG waveforms together with additional elements as depicted on a graphical user interface according to one embodiment.

FIG. 31 is a view of skin and endovascular ECG waveforms together with additional elements as depicted on a graphical user interface according to one embodiment.

FIG. 32 is a view of skin and endovascular ECG waveforms together with additional elements as depicted on a graphical user interface according to one embodiment.

FIG. 33 is a view of skin and endovascular ECG waveforms together with additional elements as depicted on a graphical user interface according to one embodiment.

FIG. 34 is a view of skin and endovascular ECG waveforms together with additional elements as depicted on a graphical user interface according to one embodiment.

FIG. 35 is a view of skin and endovascular ECG waveforms together with additional elements as depicted on a graphical user interface according to one embodiment.

FIG. 36 is a table showing various ECG waveform energy values and ratios according to one embodiment.

FIG. 37 is a graph showing energy vs. location for an endovascular ECG electrode and a skin ECG electrode.

FIG. 38 is a simplified view of a heart and proximate vasculature showing various possible locations for positioning a catheter or other medical device.

FIG. 39 is a view of a Doppler ultrasound signal, including various peaks and a region of interest, according to one embodiment.

## DETAILED DESCRIPTION OF SELECTED EMBODIMENTS

Reference will now be made to figures wherein like structures will be provided with like reference designations. It is understood that the drawings are diagrammatic and schematic representations of exemplary embodiments of the present invention, and are neither limiting nor necessarily drawn to scale.

For clarity it is to be understood that the word "proximal" refers to a direction relatively closer to a clinician using the device to be described herein, while the word "distal" refers to a direction relatively further from the clinician. For example, the end of a catheter placed within the body of a patient is considered a distal end of the catheter, while the catheter end remaining outside the body is a proximal end of the catheter. Also, the words "including," "has," and "having," as used herein, including the claims, shall have the same meaning as the word "comprising."

Embodiments of the present invention advantageously provide an inventive apparatus(es), computer-based data processing algorithms and methods for obtaining and using endovascular ECGs in a number of clinical applications and settings. For example, once device can be used to guide endovascular devices in and around the heart, e.g., guiding central venous access devices in the superior vena cava, right atrium, and right ventricle. Such central venous access devices may include central venous catheters (CVC), peripherally inserted central catheters (PICC), implantable ports, hemodialysis catheters, tunneled catheters and others. Other devices which may benefit from guidance with the inventive apparatus are temporary pacemaker leads placed through the central venous system. Catheters and guidewires used in left heart procedures may also benefit from the embodiments described herein by decreasing the amount of contrast and radiation required to guide these devices in position. In another example, the apparatus can be used for minimally invasive monitoring and assessing heart conditions based on its electrical activity, e.g., assessing preload in a heart cycle or monitoring ST segments and T-waves in congestive heart failure.

In one aspect, an apparatus is described consisting of sterile adaptors, an electronic module for signal acquisition, a computer module, software, and peripheral devices and connections. In one embodiment, the electronic module for signal acquisition can be dedicated to acquiring and processing endovascular electrical signals generated by the body (endovascular ECG), in another embodiment the electronic module can be dedicated to acquiring and processing endovascular ECGs as well as skin ECGs.

In one embodiment, the electronic module and the computer module can be separate modules, in another embodiment they can be integrated in the same module and enclosure, and yet in another embodiment they can communicate with each other via a wireless connection, such as Bluetooth. In one embodiment, the apparatus can contain an integrated printer, while in another embodiment the printer can be external and attached to the apparatus and the apparatus connected via network, e.g., wireless to other devices. In yet another embodiment the apparatus can be used for telemetry and for transmitting the endovascular electrograms to a remote location, e.g., via a telephone line, Internet, and/or wireless phone. Any combination of embodiments mentioned above is also possible.

In another aspect, various configurations allow the connection of endovascular devices, such as central venous access devices, to the electronic module for signal acquisition and processing. In one embodiment, the device consists of a connecting wire with two ends and special connectors at each end. At one end, the wire can be connected to a metal or nitinol guidewire or stylet as commonly available on the market. At the other end, the wire can be safely connected to the electronic module. In another embodiment, the device includes a coated guidewire, e.g., made of nitinol or stainless steel with uncoated distal and proximal ends and cm markings. In such an embodiment, the coated guidewire is inserted endovascularly, while the connecting wire is connected to the proximal end of the coated guidewire. In another embodiment, the device includes a catheter-syringe adaptor provided with an electrical connecting wire. At one end, the electrical connecting wire is in contact with the fluid, e.g., saline flowing within the catheter-syringe adapter. At the other end the connecting wire can be connected to the electronic module.

In another aspect, various electrode configurations allow for the optimal acquisition of endovascular ECGs. In one embodiment, a single lead is used to provide information about the tip location of an endovascular device within the vasculature. In another embodiment a modified three lead configuration is used to provide simultaneous 3-lead monitoring of the heart activity at the same time with providing tip location information. In another embodiment a modified single lead configuration plus ground is used for telemetry and transferring information from the tip of the catheter remotely.

In another aspect, algorithms are introduced for the analysis of the ECG waveforms and for supporting decision making based on these waveforms. These algorithms discriminate between different locations in the vasculature and assess body functions (systemic and at specific locations in the body), in particular heart functionality. In various embodiments, these algorithms use time domain analysis of waveforms: morphologic, for example shape; statistic, for example behavior.

In other embodiments, the algorithms use frequency domain analysis of waveforms: morphologic, for example shape; statistic, for example behavior. In further embodiments, signal energy analysis in time and frequency domains is also performed, morphologic and statistic. Fuzzy, statistical, and knowledge-based decision making are also contemplated by the present embodiments as decision support tools.

In another aspect, a user interface is provided that advantageously simplifies interpretation of data and workflow. In one embodiment the user interface includes simplified graphics showing the location in the vasculature and in the heart of the tip of the endovascular device in use without showing any of the ECG waveforms. In another embodiment, the user interface shows, in real-time, the change in location of the tip of the endovascular device in use.

In another aspect, several inventive methods are presented which use the apparatus described herein in clinical applications. In one embodiment, a computer-based method is provided that guides central venous catheters (CVC, PICCs, hemodialysis, implantable ports, and others) using stylets, guidewires and saline solution to the superior vena cava, inferior vena cava, the right atrium, and the right ventricle. This method is advantageously less sensitive to patients with arrhythmias than the prior art, and represents an alternative to chest X-ray confirmation of tip location of central venous catheters in most clinical cases. In another embodiment, a computer-based method is provided that guides coated guidewires in the right and left heart. In another embodiment, a computer-based method is provided that guides the placement of temporary pacemaker leads through the central venous system. In another embodiment, a method is provided that is minimally invasive and monitors preload using depolarization and heart rhythms. In another embodiment, a method is provided that is minimally invasive and monitors arrhythmias using P-wave analysis. In another embodiment, a method is provided that is minimally invasive and monitors heart failure using ST segment and T-wave analysis.

In another aspect, one or several skin electrodes are used to obtain skin surface ECG signals simultaneous with the acquisition of endovascular (intracavitary) electrogram (ECG) signals via the use of endovascular (intracavitary) electrodes. The simultaneous and synchronized skin surface and endovascular ECG signals are used in one of several ways to analyze and quantify the ECG signals as a function of the location of the endovascular electrode, e.g. as a function of the tip of a catheter.

In light of the above, in one embodiment the ease-of-use of the ECG-based catheter navigation and tip location is enhanced. In one aspect, for instance, skin ECG reference waveforms are simultaneously presented on a display with endovascular ECG waveforms measured at the tip of a catheter or other indwelling medical device. Such simultaneous acquisition and display of concurrent ECG signals allows for ready interpretation of the endovascular ECG waveform at the tip of the catheter. In another aspect, a skin ECG reference signal is used to synchronize information processing algorithms applied to the endovascular ECG signal, yielding results of enhanced reliability concerning changes of P-wave of the endovascular ECG signal in terms of shape and energy.

In another embodiment, analysis of the synchronized skin and endovascular ECG signals can be linked to one another and/or to the periodic electrical activity of the heart. For example, a skin ECG lead can be used to detect the R-peak of the QRS complex of a detected skin ECG waveform. Detection of the R-peak in the skin ECG waveform can be used to trigger analysis of the endovascular ECG signal in a simultaneously corresponding segments of the endovascular ECG waveform, e.g., in the segment corresponding to the P-wave. Such triggering is particularly useful in case of arrhythmia, wherein the skin ECG waveform does not typically demonstrate a consistent P-wave, while the endovascular ECG waveform indeed includes a detectable P-wave segment that changes as a function of the location in the vasculature.

In other embodiments, magnetic and steerable sterile connectors are disclosed, as well as aspects of display and control solutions to enable a mobile phone or other handheld device to control an ECG-based system including one or more of the above aspects.

In yet another embodiment, a method is disclosed according to claim 1.

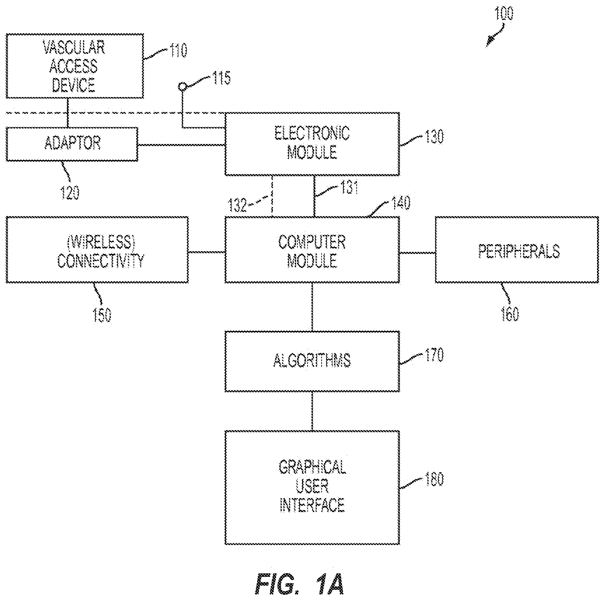

FIG. 1A is a block diagram that depicts an apparatus according to an embodiment of the present invention.

The apparatus 100 can be attached through an adaptor (120) to a large variety of commercially available and custom designed vascular access devices (110). Examples of such devices are: central venous catheters (CVC), peripherally inserted central catheters (PICC), implantable ports, tunneled catheters, hemodialysis catheters, guiding catheters for pacemaker leads, guidewires used for coronary and other vascular interventions, guiding catheters for coronary and other vascular interventions, stylets, syringe needles, and others. If the vascular access devices is a stylet, a guidewire, or a syringe needle, its material must be sufficiently electrically conductive, e.g., stainless steel or nitinol. In such a case the hook or the alligator clip adaptor according to one embodiment should be used If the vascular access device is a catheter, than saline should be used to establish a conductive path through one of the catheter's lumens. In such a case, the syringe-catheter adaptor according to one embodiment should be used.

The electronic module (130) receives electrical signals from the adaptor and from one or more other electrodes placed on the patient's skin (115). Alternatively, more than one adaptor can be used at the same time to connect to more than one endovascular device in order to provide different electrical signals to the electronic module. The use of skin electrodes is optional in certain device configurations. The electronic module processes the electrical signals and transmits them to a computer module (140) for further processing and other functions. In one embodiment the electronic module and the computer module can be packaged separately, in another embodiment they can be integrated in the same package. In one embodiment the connection between the electronic module and the computer module can be hardwired, in another embodiment it can be wireless, e.g., using Bluetooth. In one embodiment the computer module (140) can be a notebook or a netbook, in another embodiment the computer module can be a PDA or a mobile phone, e.g., an iPhone or an iPad.

The computer module processes the signals from the electronic module applying algorithms (170) as described by current embodiments. The computer module can also be connected to peripherals (160), e.g., a printer or a label printer and storage devices and provides connectivity including wireless connectivity (150) to other computers or to the internet. The storage device can be used to store a database of knowledge and information regarding the application at hand. The connectivity interface can be used to update this database remotely with newest relevant knowledge and information, e.g., new clinical cases, new findings regarding the relationship between electrograms and heart conditions. The computer module supports a graphical user interface (180) optimized for the purpose of the clinical application at hand.

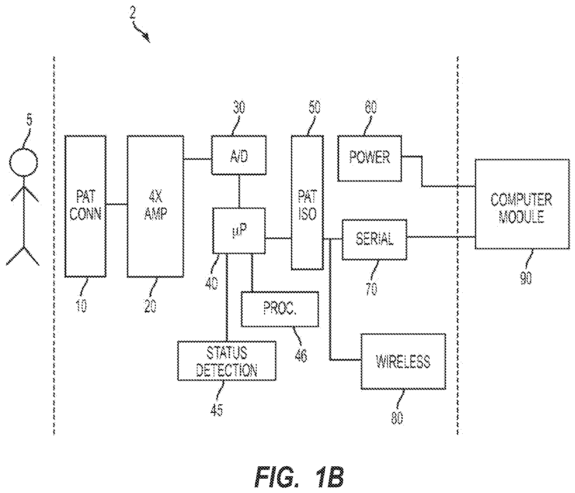

FIG. 1B is a block diagram of an electronic module (2) for acquisition and processing of endovascular electrocardiogram according to an embodiment of the present invention.

The patient connector interface (10) allows for connecting electrical leads to the patient (5). Any combination of skin electrodes and/or electrical connections to endovascular devices using the adaptors discussed above can be used. In one embodiment, the amplifier (20) is a four stage amplifier with variable gain, which can amplify electrical signals coming through the patient cable, for example, typical of electrocardiographic values. The analog-to-digital converter (30) converts the signals in digital format readable by the micro-processor (40). Any number and configurations of microprocessors, microcontrollers, and digital signal processors can be used to implement the micro-processing function (40).

In one embodiment, a microcontroller is responsible for controlling the serial communication with a computer module (90) via the serial interface (70) or via the wireless interface (80) and a digital signal processor (DSP) is responsible for implementing one or several of the inventive algorithms described herein. Alternatively, a single processor can be used for both communication and processing.

The micro-processor (40) also receives commands from the computer module (90) and controls different elements of the electronic module, e.g., the amplifier (20) accordingly. The patient isolation block (50) decouples electrically the power (60) and the serial communication channel (70) from the patient interface (10) in order to ensure patient protection to electrical shock. In one embodiment the isolation block (50) can consists of a transformer and/or couplers, e.g. optical couplers.

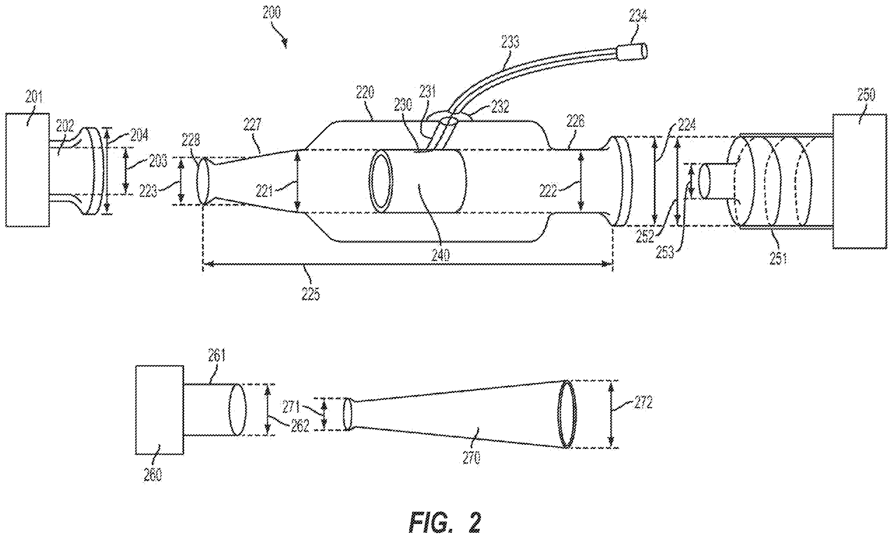

FIG. 2 depicts an adaptor (200) for an endovascular device according to an embodiment of the present invention. Vascular access devices like catheters, syringes, syringe needles, stopcocks, infusion pumps and others connect to each other through standard connections. For example, in FIG. 2 such a standard connection between two devices is illustrated on device (201) by the luer (202) with inner diameter (203), and on device (250) by threaded port (251) with inner diameter (252) and fluid opening diameter (253). The threaded port (251) and the luer (202) allow for connecting the two devices (201, 250) by threading, attaching, coupling, etc., the port (251) into the luer (202).

The adaptor (200) has a body (220) with two ends (226, 227), and is made, for example, of strong biocompatible plastic material with some degree of elasticity. End (227) has a shape of a cone. In one embodiment, end (227) has an elastic sealing portion (228) such that end (227) can easily fit in the luer (202) of device (201) to seal the connection for fluid flow. The other end (226) is in the shape of a standard luer, such as, for example, luer (202) of device (201). The threaded port (251) of the device (250) can be connected to end (226) of the adaptor (200). The cone piece (227) also allows a connection to a device that does not have a luer. The stand alone cone piece (270) allows a connection between two devices with different accessible diameters. The end (227) of adaptor (200) fits inside the diameter (272) of the cone piece (270). The end (271) of the cone piece (270) fits in a simple catheter end portion (261) of a typical device (260). For example, device (260) can be a catheter for an implantable port.

In one embodiment, device (201) is a syringe needle, and device (250) is a syringe. Fluid, e.g., a conductive electrolyte, flows through adaptor (200) through a central inner bore or lumen (222) having a certain diameter, and provides a fluid path between the devices (250, 201). A conductive metal ring (240) is attached to a portion of the substantially cylindrical surface of lumen (222) and, preferably, induces very little perturbations to the fluid flow. For example, the metal ring (240) may be disposed within a recessed portion of the substantially cylindrical surface of the lumen (222). One end (230) of a conductive wire (233) is electrically coupled to the metal ring (240); in one embodiment, the end (230) is soldered to metal ring (240). In another embodiment, the end (230) is captured between the surface of the lumen (222) and the metal ring (240), and the end (230) and the metal ring (240) maintain good electrical contact through mechanical pressure. The wire (233) may be bare or insulated. In a preferred embodiment, the metal ring (240) is fixedly attached to the surface of lumen (222) using, for example, adhesive, an interference fit, a press fit, etc., while in other embodiments, the metal ring (240) may be removably attached to the surface of lumen (222), free-floating, etc.

The wire (233) passes through a channel (231), which extends from the lumen (222) to an opening in the outer surface of the body (220). Epoxy (232), or other suitable material, may be used to seal the opening of the channel (231), as well as to provide a strain relief for the wire (233). The metal ring (240) may be advantageously disposed adjacent to the channel (231) to provide additional sealing. Thus, the metal ring (240), the wire (233), the channel (231) and the epoxy (232) provide a sealed, electrical connection to the fluid flowing through the adaptor (200). A connector (234) may provide a standard electrical connection to the electrography system; a non-terminated wire may also be used. In one embodiment, the wire (233) terminates at the opening of the channel (231) and the connector (234) is attached directly to the body (222), while in another embodiment, the wire (233) extends through the opening of the channel (231) and the connecter (234) is attached to the free end of the wire (233).

In one embodiment, the substantially cylindrical surface of lumen (222) is tapered along the longitudinal direction. This taper may extend along the entire length of lumen (222), or restricted to a certain portion thereof. For example, the surface of lumen (222) may be cone-shaped and have a larger diameter at the proximal end, or, alternatively, the larger diameter may be located at the distal end.

In one example, device (201) is a syringe needle that is inserted into a lumen of a catheter for an implantable port, and device (250) is a syringe. The syringe is filled with saline, which is then injected into the catheter through the adaptor (200). Thus, the adaptor (200) becomes filled with saline solution, and, because the conductive metal ring (240) is in contact with saline and the conductive wire (233), an electrical connection is established between the catheter lumen and the wire (233). In this way, the electrical signal at the tip of the catheter may be measured through the saline solution. Other electrically conductive solutions may also be used to obtain the endovascular electrogram using the adaptor (200). In another embodiment, the adaptor (200) may be used with infusion pumps, as well as other types of power injections. In an alternative embodiment, the adaptor (200) does not include the metal ring (240), and the electrically conductive ending (230) is in direct contact with the electrolyte.

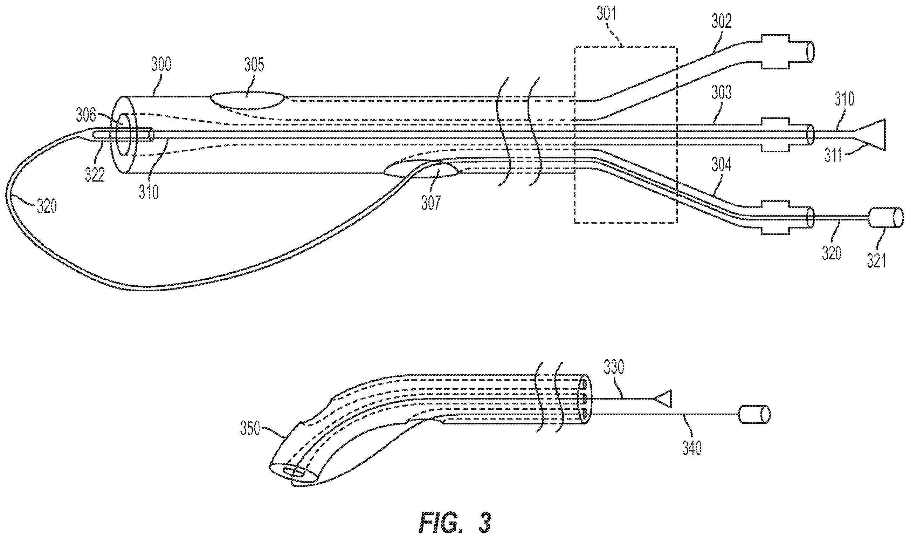

FIG. 3 illustrates a catheter steering device according to an embodiment of the present invention. In this embodiment, the catheter (300) is a triple lumen catheter and the distal end of each of the lumens is spaced with respect to each other. The catheter steering device can be used with any catheter having two or more lumens with spaced distal lumen openings. The open end of one lumen (306) of catheter (300) is at the very distal end of the catheter, another end or opening of a lumen (305) is spaced back from the distal end and the end or opening of the third lumen (307) is spaced back compared to the second end (305). The distance between the open end (306) and the end (307) is typically from one to several centimeters.

Several types of catheters have multiple lumens with spaced ends, and the inventive steering device can accommodate such catheters. For example, in the case of a peripherally inserted central catheter, the typical length of a catheter is 50 to 60 centimeters and the spacing between the distal lumen ends (305, 306, and 307) is from one to several centimeters. A hemodialysis catheter with two lumens can typically be 20 to 40 centimeters in length, with one to several centimeters spacing between the distal ends of the two lumens. A multi-lumen central venous catheter (CVC) can typically be 15 to 25 cm in length with spacing between distal ends or openings of the lumens being from several millimeters to several centimeters.

At the proximal end, the catheter has a catheter hub (301) which splits the three lumens and connects each of them with a luer (302, 303, 304). The inventive catheter steering device includes a stylet (310) with a handle (311) at the proximal end to allow for pushing, pulling, and removal after use, and a steering member (320) which connects to the distal end of the stylet (322) and which can be fed back into a distal lumen opening of one of the other lumens, such as, for example, lumen (307). The steering member (320) returns to the proximal end of the catheter through the catheter lumen and exits at the proximal end through the luer corresponding to the respective lumen (304). So disposed, the steering device is in the installed position. In one embodiment, the member (320) has a handle (321) which can be used to pull the member through the lumen. In another embodiment, the handle (321) is detachable from the member (320).

The member (320) may be polyurethane, silicone, PTFE, or other similar materials. In different embodiments, the member (320) may be any kind of biocompatible thread, e.g., surgical thread. In another embodiment, the member (320) is stainless steel wire. In one embodiment, the stylet is provided pre-inserted into one of the catheter lumens, typically the central lumen with the most distal opening (306) with the member 320 attached at the distal end of the stylet (322) and pre-inserted into the lumen (304) through the lumen opening (307). In order to steer the catheter, the user pulls the member 320 out of the catheter while preventing the stylet 310 to be pulled into the catheter. Thus, the catheter tip can be bent in a desired direction. This situation is illustrated by the bent catheter tip (350), the member (340) which was pulled back and the member (330) which is its initial position with respect to the catheter. If the stylet (310) or the steering member (320), or both are made of any electrically conductive material, then each or both of them can be used to measure electrical signals or endovascular electrograms at the distal tip of the catheter by connecting their proximal ends to the endovascular electrography system. In one embodiment, the steering member (320) can be tied to the stylet (310) through the opening (307) of the catheter lumen.

In another embodiment, the stylet (310) and the steering member (320) are manufactured as a single component to form an extended steering member that is looped back through the opening (305) of a different catheter lumen. By pulling one of the two ends of the extended steering member coming out through luers (304) and (302), the same effect is achieved and the catheter tip can be bent in a desired direction. In another embodiment, in the case of a double lumen catheter, the stylet (310) can be inserted in one lumen and the steering member (320) can be inserted in the other lumen, such that the effect of bending the catheter tip can be achieved by pulling the proximal ends. In a further embodiment, the steering member (320) can be inserted in the lumen (302) and through the opening (305).

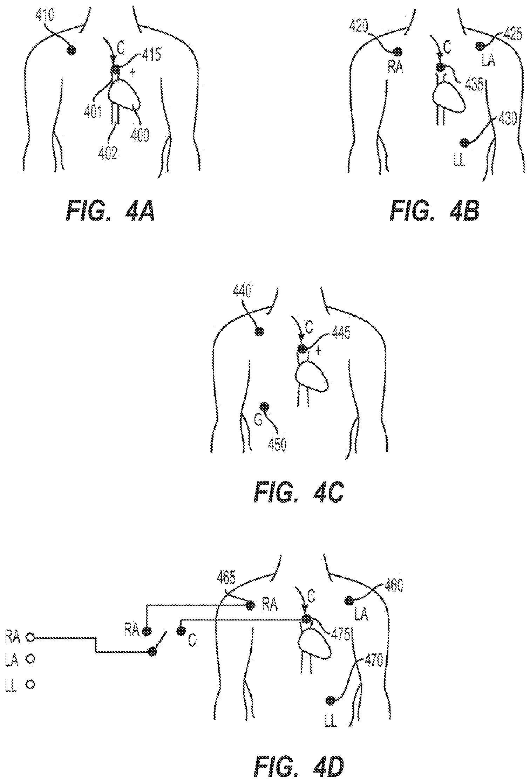

FIGS. 4A, 4B, 4C, and 4D depict electrode configurations that provide optimal acquisition of endovascular electrocardiogram according to various embodiments of the present invention.

FIG. 4A depicts a single lead configuration with a reference electrode (410), for example attached to the patient's skin over the right arm and with the other electrode attached through an adaptor to an endovascular device (415). The reference electrode attached to the skin over the right arm is presented in this configuration for illustration purposes only. Other locations of the reference electrode are possible depending on the type of ECG required. The reference electrode over the right arm together with the tip of the endovascular device used with the adaptor can be similar to lead II of a standard ECG. In this case the ECGs obtained from the superior vena cava (401) and inferior vena cava (402) can be optimized. The reference electrode can be attached to the skin in any other location in order to simulate other leads of the standard ECG. The reference electrode can be also connected to adaptors attached to other endovascular devices in order to obtain more local information from within the patient's heart (400).

FIG. 4B depicts a modified 3-lead configuration, with monitoring and guiding capabilities, with 4 electrodes. Three (3) electrodes correspond to the standard ECG electrodes: right arm (RA, 420), left arm (LA, 425), and left leg (LL, 430) used as reference. The fourth electrode is attached through an adapter to the endovascular device (C, 435). In this configuration, the electronic module and the algorithm perform two functions simultaneously: the three standard electrodes (RA, LL, and LL) perform a monitoring function of the heart, while the C electrode (435) allow for recording the ECG at the tip of device.

FIG. 4C depicts a telemetry configuration with a single grounded lead, including the configuration illustrated in FIG. 4A and a ground reference (450). This configuration can be used to transmit ECGs remotely through a telemetry system configuration.

FIG. 4D depicts one use of ECG monitors for guiding endovascular devices. A standard ECG monitor is used having standard inputs RA (465), LA (460), and LL (470). LA (460) is connected to the left arm and LL (470) to the left leg of the patient. The RA input (465) is connected to a switch which can be used be the clinician to switch the RA input (465) between the RA electrode and the catheter (C) electrode 475. Thus either monitoring or guiding of catheter placement can be achieved alternatively.

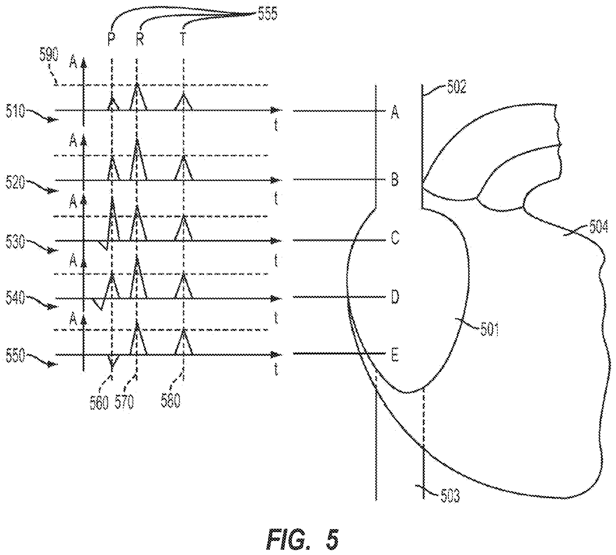

FIG. 5 illustrates exemplary electrocardiogram signal amplitudes at different locations in the central venous system.

The heart (504), right atrium (501), superior vena cava (SVC) (502), and the inferior vena cava (IVC) (503) are illustrated. Location A is in the upper SVC, location B is in the lower third of the SVC, location C is at the caval-atrial junction, location D is in the right atrium, and location E is in the upper inferior vena cava.

Graph 510 illustrates an ECG waveform as a function of time at recorded at location A. The absolute amplitude of the waveforms is recorded on an amplitude scale (590). In the case of an endovascular ECG, the standard elements of the electrocardiogram are illustrated: the P-wave (560), the R-wave (570), and the T-wave (580). The amplitudes and shape at location A recorded with a lead configuration as in FIG. 4D are similar to an electrocardiogram recoded at the skin level with the same electrode configuration.

Graph 520 illustrates an endovascular ECG depicted at location B. The amplitude at this location is higher than the one at location A but the overall shapes of the waveform are similar at location A and B.

Graph 530 illustrates an endovascular ECG depicted at location C. At location C at the caval-atrial junction, the amplitude of the waveform is yet higher than the one at location B and the P-wave has dramatically changed becoming higher than the R-wave. This waveform is an indication of the proximity of the sino-atrial node.

Graph 540 illustrates an endovascular ECG depicted at location D. At location D in the right atrium, the amplitudes are similar to location C but the P-wave changes polarity becoming bi-polar. This is an indication that the measurement of the ECG occurs beyond the sino-atrial node.

Graph 550 illustrates an endovascular ECG depicted at location E. At location E in the inferior vena cava, the waveform is similar to the one at location A in terms of amplitude except the P-wave has reverse polarity. The differences in the ECG waveforms at different locations are used by the algorithms introduced herein to discriminate between the corresponding locations and to assess heart and blood vessel functionality.

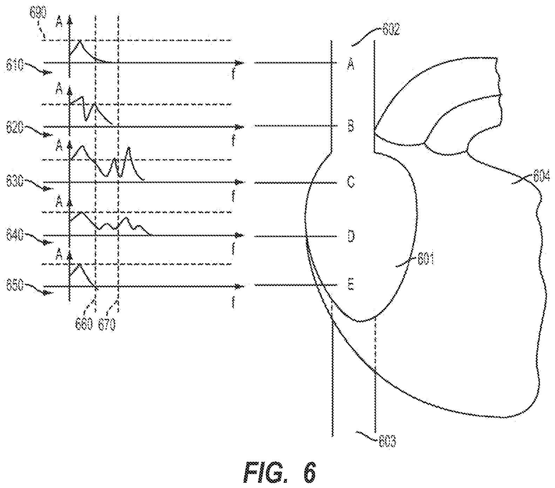

FIG. 6 illustrates exemplary electrocardiogram signal power spectra at different locations in the central venous system, using a spectral scale (690).

The heart (604), right atrium (601), superior vena cava (SVC) (602), and the inferior vena cava (IVC) (603) are illustrated. Graph 610 illustrates an endovascular ECG spectrum depicted at location A. At location A, the spectrum (610) has the appearance of a single central frequency or single band (660) and with a frequency distribution spectral power and energy similar to those at skin level.

Graph 620 illustrates an endovascular ECG spectrum depicted at location B. At location B the frequency distribution has two major bands and a higher energy and spectral power than the one at location A.

Graph 630 illustrates an endovascular ECG spectrum at location C. At location C, there are multiple (3-4) major frequencies or principal spectral components distributed over a wider range of frequencies (670). This spectral distribution is indicative of the energy distribution around the sino-atrial node. The spectral power and signal energy have increased compared to location B.

Graph 640 illustrates an endovascular ECG spectrum depicted at location D. At location D the spectrum is wider and more broadband indicative of the electrical activity of the right atrium.

Graph 650 illustrates an endovascular ECG spectrum depicted at location E. The frequency spectrum at location E is similar to the one at location A. The differences in the spectral waveforms at different locations are used by the algorithms introduced herein to discriminate between the corresponding locations and to assess heart and blood vessel functionality.

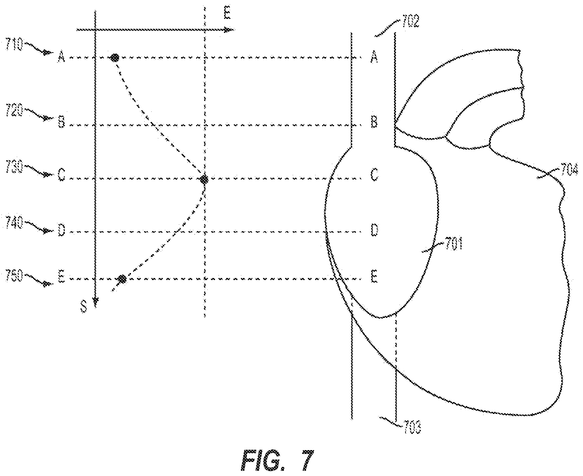

FIG. 7 illustrates exemplary electrocardiogram signal electrical energy distribution at different locations in the central venous system. The heart (704), right atrium (701), superior vena cava (SVC) (702), and the inferior vena cava (IVC) (703) are illustrated. Graphs (710, 720, 730, 740, 750) depict the energy distribution at different locations (A, B, C, D and E, respectively) and the changes in time are used by the algorithms introduced herein to discriminate between the corresponding locations and to assess heart and blood vessel functionality.

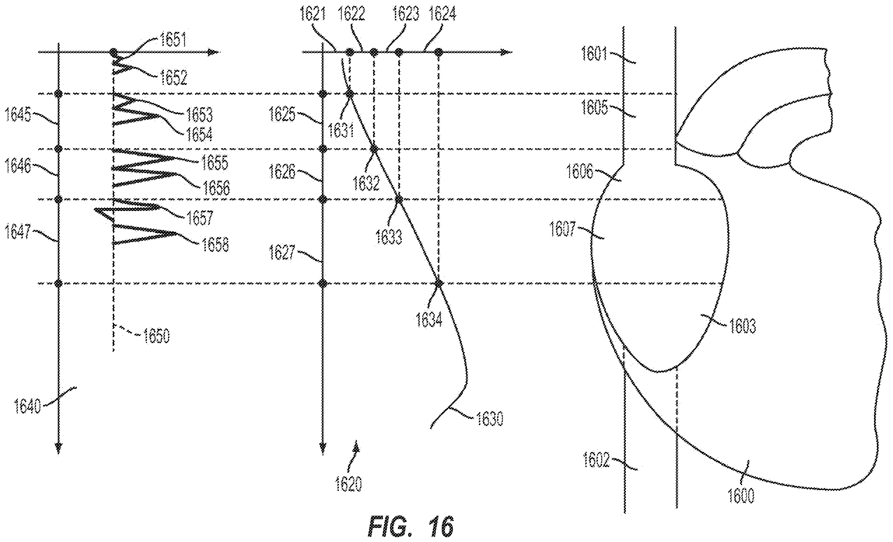

Considering FIG. 16 for a moment, a framework for analyzing the endovascular electrography signals according to an embodiment of the present invention is illustrated. The heart is represented by (1600), the superior vena cava by (1601), the inferior vena cava by (1602) and the right atrium by (1603). In this embodiment, there are three regions of interest for placing central venous access devices: the lower third of the superior vena cava or SVC (1605), the caval-atrial junction or CAJ (1606), and the upper right atrium or RA (1607).

The graph (1620) illustrates the electrical energy profile as a function of location in the heart and the graph (1640) illustrates the different electrography waveforms which can be obtained at different locations in the heart. The curve (1630) illustrates the increase of electrical energy detected in each of the regions at the tip of an endovascular catheter advancing from the superior vena cava into the heart. In one embodiment, the energy curve is calculated in time domain, while in another embodiment, the energy curve is calculated in the frequency domain using the frequency spectrum. In one embodiment, the energy is calculated for the actual signal levels, while in another embodiment, the baseline value or other mean values are first subtracted from the signal values before energy calculations. The signal energy or power is calculated in time domain by summing up the squared amplitude values before and/or after baseline subtraction over a determined period of time, e.g., a heartbeat. In the frequency domain, the signal energy or power is calculated by summing up the squared values of the frequency components. In one embodiment, the curve is calculated using the entire electrogram, while in other embodiments, only certain segments of the electrogram are used for the energy calculations, e.g., only the segment corresponding to a "P-wave" of an electrocardiogram. Such a "P-wave" segment is representative of the electrical activity of the sino-atrial node.

Different levels of energy characterize the different locations along the catheter path from the SVC to the heart. These regions can be differentiated in terms of their electrical energy level by using thresholds. Threshold (1631) of energy level defines the beginning of the lower third of the superior vena cava. The energy levels (1621) define the regions in the vasculature of low energy which are distant or further away from the sino-atrial node. The energy levels (1622) between thresholds (1631) and (1632) define the region labeled as the lower third of the superior vena cava (1625 and 1605). The energy levels (1623) between thresholds (1632) and (1633) define the region labeled as the caval-atrial junction (1626 and 1606). The energy levels (1624) between thresholds (1633) and (1634) define the region labeled right atrium (1627 and 1607).

Similarly, the shape and size of the electrogram in graph (1640) relative to a baseline (1650) can be correlated to a location in the heart. Thresholds (1631), (1632), (1633), and (1634) are determined specifically for the type of energy considered for calculations, e.g. the entire electrogram, the P-wave, and/or the S-T segment. Before the lower third of the SVC and corresponding to a relatively low level of energy (1621), the P-wave (1651) and the R-wave (1652) are similar in size and shape with a standard electrocardiogram lead II recorded at the skin level if the right arm standard ECG lead is connected to the catheter and measuring the electrogram signal at the tip of the catheter. In the lower third of the SVC (1605 and 1645), the energy level of the electrogram increases, the electrogram amplitudes increase and the P-wave (1653) increases amplitude and energy relative to the R-wave (1654) to where the P-wave amplitude and energy between half and three quarters of the amplitude and energy of the R-wave. At the caval-atrial junction (1606 and 1646), the energy level of the electrogram increases further, the electrogram amplitudes continue to increase and the P-wave (1655) increases amplitude and energy relative to the R-wave (1656) to where the P-wave amplitude and energy are larger or equal to the amplitude and energy of the R-wave. In the right atrium (1607 and 1647), the energy level of the electrogram increases further, the electrogram amplitudes increase, the P-wave (1657) becomes bipolar and its amplitude and energy relative to the R-wave (1658) start decreasing. These behaviors are quantified, analyzed, and used in order to provide location information regarding the tip of the catheter.

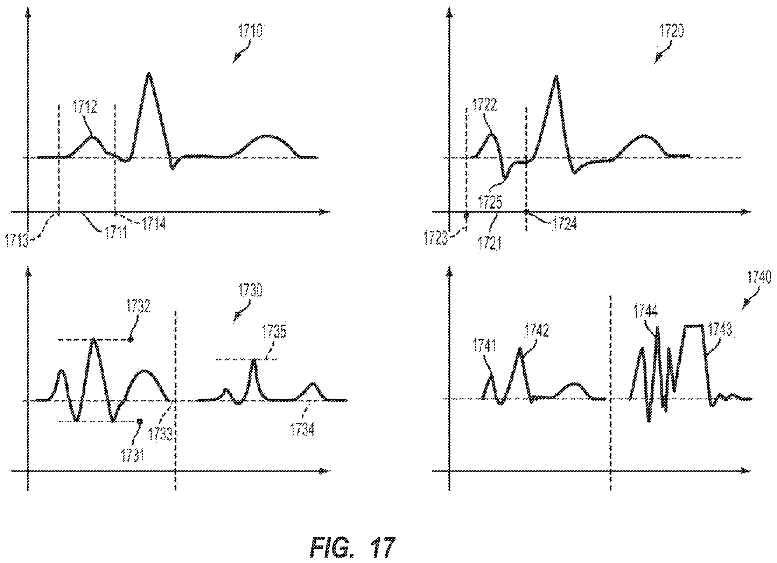

Considering FIG. 17 for a moment, several electrogram waveform processing embodiments are illustrated. Graphs (1710) and (1720) illustrate a P-wave analysis embodiment. Since the P-wave corresponds to electrical activity of the heart generated by the sino-atrial node, the changes of the P-wave are most relevant with respect to determining the proximity of the sino-atrial node in an endovascular approach. Therefore, in order to assess proximity of the sino-atrial node and location in the vasculature, signal analysis methods in time and frequency domains, as well as signal energy criteria can be applied only to the P-wave segment of an electrogram. In graph (1710), the segment designated for the P-wave analysis (1711) starts at moment (1713) and ends at moment (1714). During the period of time between the starting moment and the ending moment of the P-wave segment, the highest amplitude detected corresponds to the P-wave peak (1712). The starting moment (1713) of the P-wave segment analysis can be determined in a number of ways. In one embodiment, the heart beat is calculated and the R-peak is detected as the maximum amplitude of the heart beat. Going back from each R-peak a certain percentage of the heart beat, for example between 20% and 30%, determines the moment when the analysis of the P-wave starts (1713). Going back 2% to 5% of the heart beat from each R-peak determines the end of the segment designated for the P-wave analysis (1714). Similarly, in graph (1720), the designated segment for the P-wave analysis (1721) starts at moment (1723) in the heart cycle and ends at moment (1724). The P-wave in this case is bipolar with a positive maximum amplitude (1722) and a negative maximum amplitude (1725) when compared to the baseline (amplitude equals zero). For the P-waveform defined between the starting point (1713 on graph 1710 and 1723 on graph 1720) and the end point (1714 on graph 1710 and 1724 on graph 1720) time-domain and frequency-domain algorithms are applied according to embodiments of the present invention.

Graph (1730) illustrates the advantages of baseline subtraction prior to signal energy computation. If the signal energy is calculated in time domain as the sum of the squared signal amplitudes over a heartbeat, then the amplitude variations between levels (1731 and 1732) around baseline (1733) may lead to a lower energy level than the signal with amplitude variations between levels (1734 and 1735) whereby the level (1734) is the baseline. The baseline value (1733) is subtracted from the amplitude values (1731 to 1732) and the baseline value (1734) is subtracted from the amplitude values (1734 to 1735). After subtracting the baseline, the sum of squared amplitude values is calculated. Thus, this sum is proportional to the energy of signal variation around the baseline and therefore it is more appropriate to characterize changes in the signal values/behavior.

Graph (1740) shows a typical electrogram waveform with a P-wave (1741) and an R-wave (1742) and a distorted signal with the P-wave covered by high frequency noise (1744) and the R-wave saturated to a maximum value (1743). In the presence of these kinds of artifacts (1744 and 1743) it is very difficult and sometimes impossible to recover the original signal (1741 and 1742). Therefore, according to embodiments of the present invention, an algorithm is used to detect the presence of artifacts and reduce the amount of artifacts as much as possible. If, after reducing the artifacts, the signal cannot be recovered, then the signal is discarded for the computation of signal energy. The presence of artifacts can be detected in time domain by a high value of the derivative and of its integral, a jump in signal energy, a jump in the value of the baseline or in different averages calculated from the signal. In frequency domain, the artifacts can be detected as a jump in the value of the DC component (frequency zero of the spectrum), as the sudden appearance of high frequency components, and in a jump of the spectral power/energy. In the frequency domain, selective filtering can be applied and all components removed, which are not "typical" for the average behavior of the signal. After selective filtering, the signal is reconstructed in the time domain using an inverse Fourier transform in order to allow for verification of the success of the selective filtering.

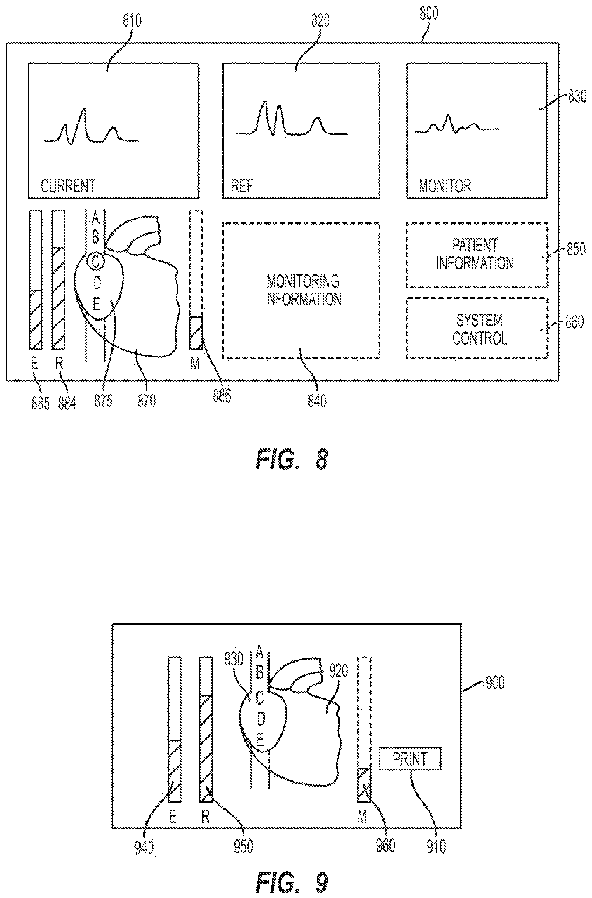

FIG. 8 depicts a graphical user interface according to an embodiment of the present invention.

Window (810) presents the ECG waveform in real-time as it is acquired by the electronic module using the attached electrode configuration. Window (820) is a reference window and shows a frozen waveform used to compare with the current window. In one embodiment, the reference waveform in window (820) can be obtained through the electrodes connected to the electronic module at a reference location of the catheter and/or using a reference configuration of the skin electrodes. For example, such a reference waveform can be the ECG recorded using an adaptor according to an embodiment of the present invention connected to an endovascular device placed at the caval-atrial junction. In a different embodiment, the reference waveform in window 820 can be a typical waveform at a certain location in the vasculature or of a certain heart condition as it is recorded in a database of waveforms and as it is stored in the storage medium of the computer system. If the electrode configuration allows for simultaneous heart monitoring and recording of electrograms using an endovascular device, window (830) shows one of the standard ECG leads for heart monitoring, while window (810) shows the ECG at the tip of the endovascular devices when connected to an adaptor, such as the ones discussed above.

The icon (870) is a representation of the heart, and the locations A through E (875) illustrate different locations in the heart and vascular system which can be discriminated by analyzing endovascular ECGs in accordance with the methods disclosed herein. As a location in the vasculature is identified by the algorithms, the corresponding place and letter on the icon (875) becomes highlighted or in some other way is made visible to the user. The bars (884), (885), and (886) show signal energy levels. The "E" bar (885) presents the amount of electrical energy computed from the ECG frequency spectrum at the current location of the tip of the endovascular device. The "R" bar (884) presents the amount of electrical energy computed from the ECG frequency spectrum at a reference location. The "M" bar (886) presents amount of electrical energy computed from the ECG frequency spectrum using the monitoring ECG signal from the skin electrodes. The window (840) depicts monitoring information, e.g., heart rate. Patient information (name, date of procedure and others) are shown in window (850). Window (860) contains system control elements like buttons and status information, e.g., scale, scroll speed, system parameters and system diagnostics.

FIG. 9 depicts a graphical user interface according to another embodiment of the present invention.

The icon (920) is a representation of the heart and the locations A through E (930) illustrate different locations in the heart and vascular system which can be discriminated by analyzing endovascular ECGs. As a location in the vasculature is identified by the algorithms, the corresponding place and letter on the icon (930) becomes highlighted or in some other way is made visible to the user. The bars (940), (950), and (960) show signal energy levels. The "E" bar (940) depicts the amount of electrical energy computed from the ECG frequency spectrum at the current location of the tip of the endovascular device. The "R" bar (950) shows the amount of electrical energy computed from the ECG frequency spectrum at a reference location. The "M" bar (960) shows amount of electrical energy computed from the ECG frequency spectrum using the monitoring ECG signal coming from the skin electrodes. The button "Print" (960) allows the user to print the information documenting the case on a printer, for example on a label printer for quick attachment to the patient's chart.

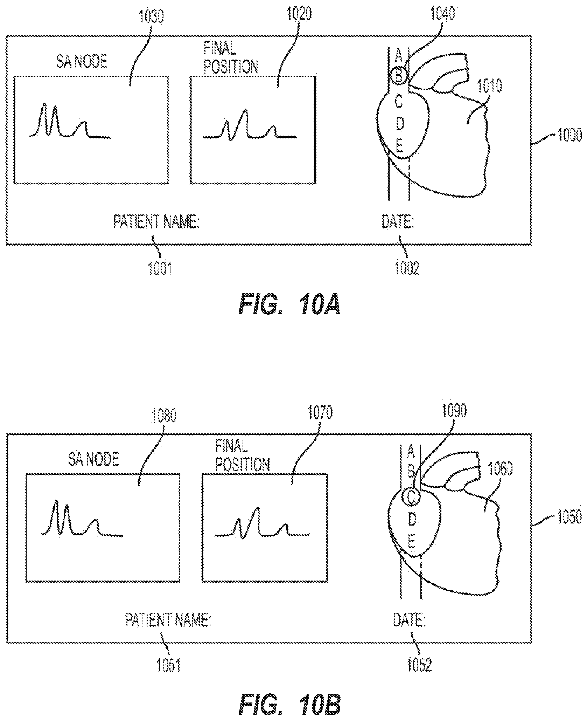

FIGS. 10A and 10B depict exemplary printouts for the information displayed by the graphical user interface, according to an embodiment of the present invention.

FIG. 10A illustrates a printout (1000) for the case of a catheter tip placement procedure in the lower third of the SVC. The field 1010 depicts the heart icon whereby the letter "B" corresponding to the lower third of the superior vena cava (SVC) is highlighted (1040). Field 1030 depicts the reference ECG waveform recorded at the tip of the catheter at the caval-atrial junction in the proximity of the sino-atrial node. Field 1020 depicts the ECG waveform at the tip of the catheter in the position in which it was placed at the end of the procedure. For FIG. 10A, this location is in the lower third of the SVC and the ECG waveform corresponds to this location. The patient name (1001) and the date of procedure (1002) are also printed.

FIG. 10B depicts a similar printout (1050) except that the final position at the end of the procedure is at the caval-atrial junction at location C (1090) on the heart icon (1060). The "SA Node" field depicts the reference ECG waveform (1080), and the "Final Position" field (1070) shows that the catheter was placed with the tip at the sino-atrial node: the ECG waveform in final location is similar or even identical with the one in the reference location at the sino-atrial node (SA Node). It is known that the proximity of the SA Node indicates a location at the caval-atrial junction. These locations are sometimes considered identical by some clinicians.

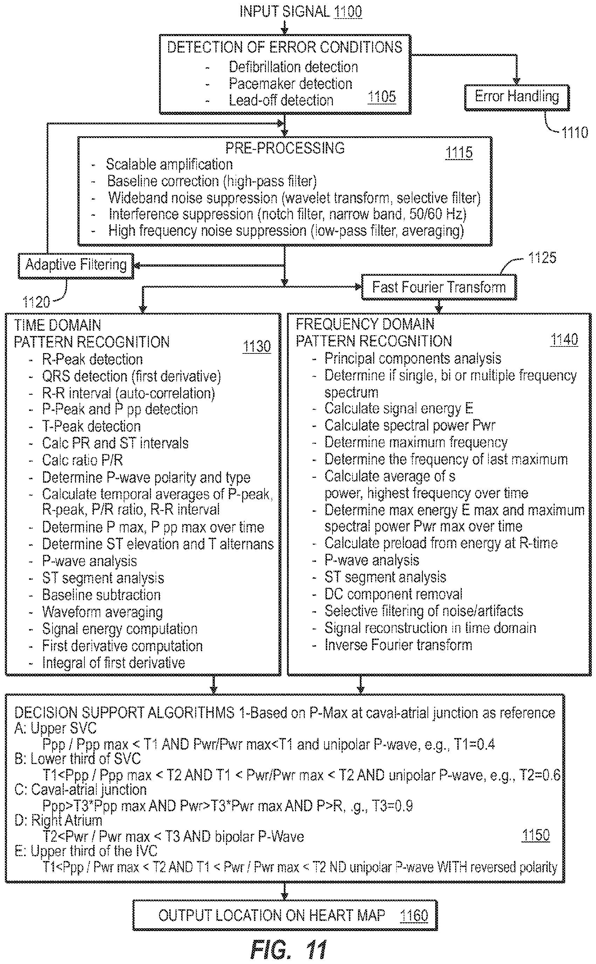

FIG. 11 is a block diagram for a computer-based method (1100) for positioning an endovascular device in or near the heart using electrocardiogram signals.

The algorithms are applied to the input signal (1102) (ECG) acquired by the adaptor to the endovascular devices and, optionally, through skin electrodes as well. The Error Detection Block (1105) detects at least three types of error conditions/exceptions, such as, for example, when a defibrillator has been applied to the patient, when a pacemaker is firing excitation pulses and/or when a lead/electrode is off. These errors/exceptions may be handled differently, and the user may be informed about the presence of an exception and the way of handling the exception (1110).

The Pre-Processing block (1115) may amplify the signal, reduce noise, eliminate artifacts, etc. In one embodiment, rescaling the signal to the display range occurs under user control and is not automatic, as with most currently available ECG monitors. Thus, changes in the amplitude of the ECGs are easily noticed. A high-pass filter corrects the baseline and reduces such artifacts as respiratory artifact. Wideband noise suppression may be achieved using a selective filter, e.g., a wavelet transform. Electromagnetic interference with other equipment and the power grid may be suppressed by a notch filter (narrow band filter) centered at 60 Hz or 50 Hz to accommodate domestic or international power supplies. High frequency noise may be suppressed with a low-pass filter, which, in one embodiment, is implemented with variable length averaging, such as, for example, a running window corresponding to a heart cycle, an averaging of the ECG over several consecutive heart cycles, etc. The Adaptive Filtering block (1120) optimizes the filter coefficients by minimizing an error signal.

The Time-Domain Pattern Recognition block (1130) identifies elements of the ECG waveform, their relationship(s) and their behavior(s) in time. An important aspect of the time-domain pattern recognition algorithm in block 1130, as well as of the Frequency Domain Pattern Recognition block 1140, is data history. The ECGs are analyzed in real time for certain elements, and, for other elements, a data buffer with an appropriate buffer length is maintained in the memory of the electronic and/or computer modules in order to allow for historic data analysis and prediction based on this analysis. In one embodiment, the data history buffer is several seconds long allowing for the ECG signal corresponding to several heartbeats to be saved in the buffer. A double buffering technique allows the waveform in one buffer to be processed while the second buffer continues to store signals. Thus no signal data are lost while the waveform in one buffer is processed. After data processing on one buffer is completed, the results are sent to the Decision Support Algorithms (1150) and the two buffers switch roles. The length of the buffer accommodates the time duration of data processing in order to ensure that no data are lost. A similar double buffering technique is also applied to the data subject to Frequency Domain Pattern Recognition block (1140).

In the case of an endovascular ECG, elements of interest may include, but are not limited to, one or more of the following:

1. The P, Q, R, S, T, and U waves, their peaks, amplitudes and duration;

2. The duration of the P-R, S-T, and T-P segments/intervals;

3. The elevation of the S-T segment;

4. The variances of the P-P and R-R intervals;

5. The variance of the S-T and of the R-T intervals, etc.;

6. The peak-to-peak values of the P-wave and of the QRS complex;

7. The ratio of the P-wave and R-wave amplitudes and the ratio of the P-wave and QRS complex peak-to-peak amplitudes;

8. The polarity of the P-wave: single positive, single negative, or bipolarity;

9. The derivative of the P-wave, QRS-complex, and T-wave;

10. Temporal average of the R-R interval and the heart beat;

11. Maximum value of the P-wave amplitude/peak and of the P-wave peak-to-peak amplitude over a certain period of time;

12. Maximum value of the R-wave amplitude/peak and of the ORS complex peak-to-peak amplitude over a certain period of time.

In the time domain, additional computations include:

13. Baseline subtraction, for example for removing of respiratory artifacts and in order to allow for the analysis of changes with respect to the baseline;

14. Waveform averaging for noise reduction;

15. Signal energy computation in time domain as the sum of the squares of signal amplitudes (before and after baseline removal);

16. First derivative computations for estimation of signal changes and removal of high frequency artifacts;

17. Integral (sum) of the first derivative values;

In the frequency domain, additional computations include:

18. DC and quasi-DC component removal (equivalent to baseline subtraction and removal of respiratory artifact);

19. Selective filtering, i.e., the removal of certain frequencies associated with artifacts and noise, e.g., high frequency noise, muscle artifacts, changes in signal due to catheter and electrode handling, etc.;

20. Inverse Fourier transform for reconstructing the signal into the time domain.

Several techniques may be used to derive the information listed above from the ECG waveforms, including, but not limited to, one or more of the following:

1. "Peak detection;"

2. Computation of first derivatives;

3. Running averages along the signal in one heartbeat and along multiple heartbeats;

4. Adaptive thresholding;

5. Auto-correlation.

The Fast Fourier Transform in block (1125) performs a Fast Fourier Transform on a number of ECG samples stored in a buffer of a certain length, e.g., 256, 512, 1024, 2048 or more data samples. The Fourier Transform transforms the waveform from the time domain into the frequency domain.

The Frequency-Domain Pattern Recognition block (1140) illustrates various aspects of pattern recognition performed on the ECGs in the frequency domain, including, but not limited to, one or more of the following:

1. Principal components analysis, i.e., determination of the most significant elements of the frequency spectrum (similarly to determining the morphological elements of the electrograms, e.g., certain waves and segments in time domain);

2. Data compression in order to reduce the amount of computation based on the principal components;

3. Determination of the number and morphology of the principal components, in particular determination if the spectrum has only one, two or multiple main frequencies (frequency bands);

4. Calculation of the spectral power and of the signal energy from the frequency spectrum;

5. Running average along the frequency dimension over a single spectrum in order to reduce wideband noise;

6. Running average along several spectra in order to filter out artifacts;

7. Determination of additional morphological elements of the spectrum, e.g., the maximum frequency, the energy contained in the maximum frequency, the frequency histogram, i.e., what frequencies contain how much energy, the frequency of the highest significant maximum energy peak, etc.;

8. Calculation of behavior and averages over time of the principal components and other parameters determined from the spectral distribution, e.g., determining the maximum value of the signal energy and of the spectral power over a certain period of time;

9. Determine/estimate certain heart conditions based on the spectral analysis. This determination/estimation is also performed in more detailed in the decision support blocks 1150 and 1250.

Several decision support algorithms use the information provided by the time domain pattern recognition and frequency-domain pattern recognition algorithms. In one embodiment, block (1150) supports placing an endovascular device in either the lower third of the SVC or at the caval-atrial junction.

In particular, block 1150 is based on the concept of first reaching the caval-atrial junction during catheter placement. At the caval-atrial junction or near the sino-atrial node the P-wave and other electrical parameters reach a maximum value. At the caval-atrial junction the P-wave is unipolar. After reaching the sino-atrial node at the caval-atrial junction, i.e., the maximum value of the P-peak amplitude and spectral power, the catheter is pulled back several centimeters until the P-wave decreases to half the amplitude reached at the caval-atrial junction. At the location where the P-wave has decreased to half the amplitude as the caval-atrial junction, the catheter is considered to be in the lower third of the superior vena cava. The P-wave peak amplitude or peak-to-peak amplitude, as well as the spectral power, is used to map the location in the vasculature to the ECG waveform.

More particularly, after receiving an endovascular ECG signal associated with an endovascular device, the signal is processed, over a plurality of predetermined time periods, to calculate a P-wave amplitude and a spectral power for each predetermined time period. A maximum P-wave amplitude is then determined from the plurality of P-wave amplitudes, as well as an associated maximum spectral power from the plurality of spectral powers. The location at which these maximum values are determined is associated with a predetermined location in or near the heart, such as the cava-atrial junction. The location of the endovascular device is then calculated, for each predetermined time period, based on a ratio of the P-wave amplitude to the maximum P-wave amplitude and a ratio of the spectral power to the maximum spectral power, and the location of the endovascular device is then displayed to the user. Additionally, the polarity of the P-wave and the R-wave amplitude may also be used to determine the location of the endovascular device.

A single criterion or a combination of such criteria can be used to support decision making. In one embodiment, T1, T2, and T3 may be empirically established thresholds which are different for each patient, and the algorithm can use an adaptive loop to adjust the thresholds based on the current measurements. In another embodiment, these thresholds are predetermined.

In other embodiments, the ratio between the P-peak/P amplitude or the P-wave peak-to-peak amplitude to the R-peak/R amplitude or to the QRS complex peak-to-peak amplitude can also be used to establish location relative to the sino-atrial node. In one embodiment the P-peak/amplitude must be approximately half of the R-peak/amplitude and the P-wave must be unipolar for the location to correspond to the lower third of the SVC. In another embodiment, the P-wave peak-to-peak must be half of the QRS peak-to-peak amplitude and the P-wave must be unipolar for the location to correspond to the lower third of the SVC.

As discussed above, the results of the decision support algorithms block 1150 may be presented to the user, for example, by high lightening the appropriate location on the heart icon corresponding to the type of ECG identified by the system (1160).

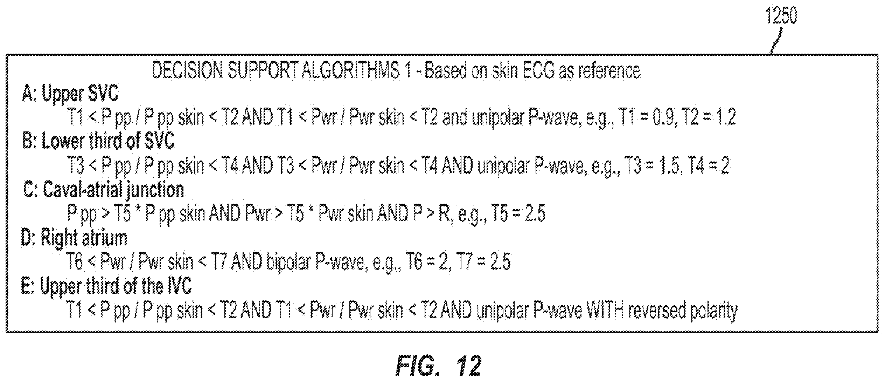

The decision support algorithm block 1250, depicted in FIG. 12, is based on comparing the P-wave, R-wave and P-wave spectral power at the current locations with the values of these parameters determined from the skin electrocardiograms in an equivalent lead, e.g., lead II. Thresholds T1 through T6 are empirical values subject to adaptive adjustments relative to each patient. Each of the criteria or a combination of criteria shown in FIG. 12 can be used.

Other decision algorithms can also be used, in particular related to the level of electrical energy as calculated from the ECG spectrum. In the case of placing endovascular devices, one criterion may be that, at the location corresponding to the lower third of the SVC, the average electrical energy calculated from the endovascular ECG is twice as high as the average electrical energy calculated from the endovascular ECG at skin level or from a skin ECG in a corresponding lead, e.g., lead II.

Method for Placement of Central Venous Catheters

A method of placing a central venous catheter (CVC) is presented below.

1. Estimate or measure the required length of the vascular access device (CVC) for the given patient.

2. If using saline and adaptor (200), go to step 11; if not, proceed as follows. Insert a guidewire into the CVC and flush align the guidewire tip and the catheter tip. Measure the length of the guidewire outside the CVC. This measurement is necessary in order to be able to realign the tip of the catheter and of the guidewire after inserting the guidewire in the vasculature. After taking the measurement, for example with sterile measuring tape or with surgical thread, remove the guidewire from the CVC.

3. Gain vascular access and insert the guidewire for the estimated required length.

4. Insert the CVC over the wire such as to leave outside the CVC the length of the guidewire measured at step 1.

Thus the CVC inserted over the wire and the guidewire tips are flush-aligned.

5. Connect a sterile electrical adaptor to the guidewire per the instructions for use.

6. Connect the other end of the sterile electrical adapter to the ECG cable of the electrography system.

7. Check that the display of the electrography system indicates desired position of the catheter tip per the instructions for use of the electrography system: in the lower third of the SVC, at the caval atrial junction or in the right atrium. Typically, the location of the tip of the catheter will be identifiable through the specific shape of the P-wave and of the P-wave relative to the R-wave of the electrogram and/or by the energy levels and thresholds.

8. Adjust the position of the guidewire and CVC by pulling and/or pushing them together as not to change the flush alignment until the ECG waveform on the screen indicates that the desired position has been reached. Correlate the actual inserted length with the estimated length.

9. After the position has been reached, disconnect the electrical adaptor and remove the guidewire.

10. Secure the CVC in location.

11. Continue here if saline and adaptor (200) are used.

12. Gain vascular access and introduce the CVC over the guidewire as currently specified by the existing protocols.

13. Remove the guidewire

14. Attach the sterile adaptor (200) to the CVC.

15. Attach the electrical connection (234) of the adaptor (200) to the ECG cable of the electrography system.

16. Fill a syringe with saline and connect it to the other end of the adaptor (200). Flush the catheter lumen with saline as to create a conductive saline column all way through the catheter tip.

17. Check that the ECG waveform shown on the display of the electrography system indicates desired position of the catheter tip per the instructions for use of the electrography system: in the lower third of the SVC, at the caval atrial junction or in the right atrium. Typically, the location of the tip of the catheter will be identifiable through the specific shape of the P-wave and of the P-wave relative to the R-wave of the electrogram and/or by energy levels and thresholds.

18. Adjust the position of the CVC by pulling and/or pushing until the ECG waveform on the screen indicates that the desired position has been reached. Correlate the actual length with the estimated length.

19. After the desired position has been reached remove the syringe and the adaptor (200).

20. Secure the catheter.

Method for Placement of Implantable Ports

A method of placing the catheter piece of an implantable port is similar to the method for placing a CVC. The adaptor (200) should be connected to the catheter of the implantable port, and the syringe with saline must be connected to the other end of the universal adaptor. A different electrical adaptor should be connected to a syringe needle placed in the catheter of the implantable port. After reaching the desire position, the catheter should be connected to the implantable port.

Method for Placement of Peripherally Inserted Central Catheters Open and Closed Ended

Both open-ended and closed-ended peripherally inserted central catheters (PICC) can be placed as described herein, and the method of PICC placement is similar to the one of placing CVCs. The inventive steering mechanism described herein can be used to bend the tip of the PICC in case the catheter fails to advance in the desired direction.

Method for Placement of Hemodialysis Catheters

A method for placing hemodialysis catheters is similar to the method introduced herein for placing CVCs. The inventive steering mechanism described herein can be used to bend the tip of the hemodialysis catheter in case the catheter fails to advance in the desired direction. Two different guidewires with adaptors (220) can be used for each of the lumens of the hemodialysis catheter as to guide placement of one lumen into the right atrium and of the other lumen at the caval atrial junction using the electrography system. Each of the lumens of the hemodialysis catheter can be placed independently in sequence or at the same time by connecting the adaptors (220) of each of the lumens with different electrodes of the ECG cable of the electrograph system.

Method for Placing Central Venous Access Devices in Patients with Arrhythmias

Traditionally, patients with arrhythmias have been excluded from procedures of guiding central venous lines placement using the endovascular ECG method because of the lack of visible changes in the shape of the P-wave. The energy criteria for the P-wave analysis described herein can be used to guide the placement of central venous access devices in patients with arrhythmias. In arrhythmia patients, the electrical signals generated by the sino-atrial node have a certain degree of randomness, such that they are not synchronized in order to produce a consistent P-wave. Nevertheless, as previous studies have shown, the electrical activity of the sino-atrial node exists and generates electrical energy of intensities typical to the proximity of the sino-atrial node. In one embodiment, the algorithm uses the energy as measured from the endovascular electrogram in order to map certain location in the vasculature. As such, this algorithm can be used to guide placement in patients with arrhythmias when only the electrical energy is indicative of location but not the shape of the P-wave.

Method for Monitoring Tip Location and Certain Aspects of the Electrical Activity of the Heart

Certain aspects of the electrical activity of the heart can be monitored continuously or intermittently using the devices introduced herein. Either an electrical adaptor or adaptor (200) connected to the electrography system can be used for monitoring. The electrical adaptor can be connected to any stylet or other conductive member introduced in any venous access device or in any arterial device. Adapter (200) can also be connected to any venous or arterial line as long as the infusion of a conductive solution, e.g., saline is possible. Adapter (200) can also be used when electrically conductive fluids are inserted in the body using an infusion pump. Monitoring the tip location and/or certain aspects of the electrical activity of the heart can be performed in a number of clinical situations.

1. Adaptor (200) can be attached to a number of central venous devices post insertion, e.g., at bedside and/or in home care situations: PICCs, CVC, hemodialysis catheters. By connecting the adapter to such a catheter and to an electrography system according to an embodiment of the present invention and by injecting saline into the catheter, the location of the tip of the catheter can be confirmed and/or certain electrically activity of the heart can be monitored during the time the adapter is connected by using methods similar to those introduced above in embodiments of the present invention.

2. Adaptor (200) can be connected to an arterial line between the arterial line and the other devices connected to the arterial line. The blood present in the arterial line and in the universal adaptor ensures the electrical connection between the blood and the electrography system. Thus the electrical activity of the heart can be continuously monitored. This is particularly important in the case of monitoring the preload changes which translate in changes of the electrical energy of the heart during the S-T segment of the ECG waveform.

3. Monitoring of the tip location and of the electrical activity of the heart can also be achieved by using the electrography system and connecting the adaptor (200) between a central venous line and a pressure measuring system while performing central venous pressure measurements.

4. In the case of an implanted port, a needle can be inserted into the port chamber and the catheter can be flushed with saline using a syringe filled with saline. An electrical adaptor can be attached to the needle and to the electrography system. The detected electrogram signal will contain information from the skin level where the needle is in contact with the skin and from the tip of the catheter through the injected saline column. Since the impedance of the path to the catheter tip is lower than the one to the skin, the detected signal contains both components, i.e., at the skin level and at the tip of the catheter. By subtracting the skin level signal, the signal at the tip of the catheter can be estimated and thus the tip position and certain electrical activity of the heart according to the algorithms described in embodiments of the present invention.

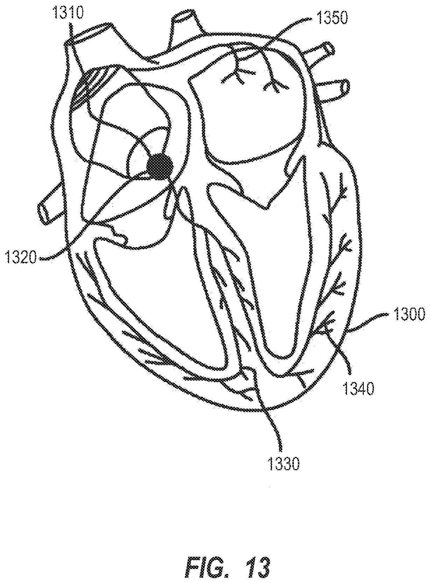

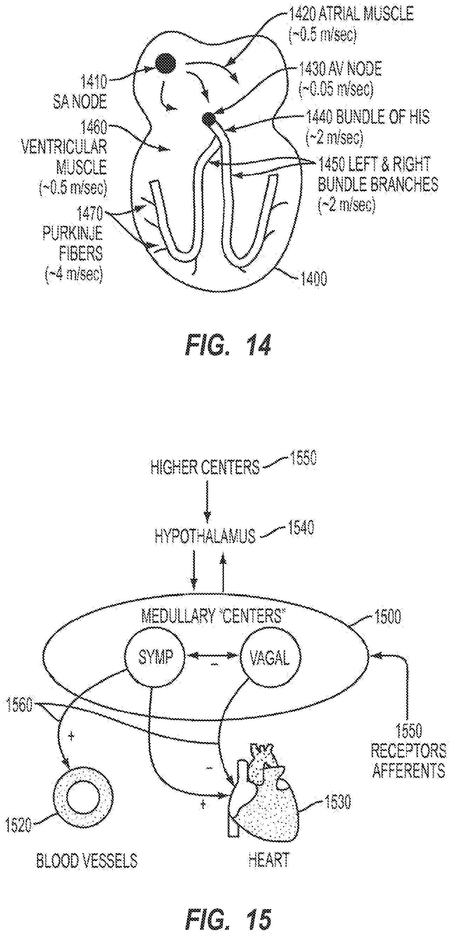

FIG. 13 illustrates the cardiac conduction system of the heart, while FIG. 14 illustrates electrical signal propagation in the conduction system of the heart.

These figures illustrate the conductive mechanism of the heart, which explains why the electrical energy distribution within the heart as measured is indicative of specific locations within the heart. Accordingly, local electrical signals, behaviors and energy concentrations can be measured and locations within the heart and blood vessel can be determined more accurately; local heart conditions can also be described more accurately.

The conduction system of the heart begins with the heart's dominant pacemaker, the sino-atrial node (1310). The intrinsic rate of the SA node is 60 to 100 beats/minute. When an impulse leaves the SA node, it travels through the atria along the Bachmann's bundle (1350) and the inter-nodal pathways, on its way to the atro-ventricular (AV) node (1320) and ventricles. After the impulse passes through the AV node, it travels to the ventricles, first down to the bundle of His (1330) then along the bundle branches and finally down to the Purkinje fibers (1340). Pacemaker cells in the junctional tissue and Purkinje fibers on the ventricles normally remain dormant because they receive impulses from the SA node. They initiate an impulse only they do not receive one from the SA node. The intrinsic rate of the AV junction is 40 to 60 beats/minute, the intrinsic rate of the ventricles 20 to 40 beats/minute. The different propagation speeds of the electrical impulses are shown in FIG. 14. From the SA node (1410) the impulses propagate through the atrial muscle (1420) and through the ventricular muscle (1460) at app. 0.5 ms, through the bundle branches (1440) and (1450) at app. 2 m/sec, through the Purkinje fibers (1470) at app 4 m/s and through the AV node (1430) at app. 0.05 m/s.

The electrical signals and the electrical energy distribution are advantageously used to identify the proximity of the sino-atrial node and right atrial electrical activity even in the cases of arrhythmia, i.e., in the absence of a coherent P-wave measured by standard skin electrocardiogram. While in some cases of arrhythmia random electrical signal generated in the right atrium is not coherent enough to propagate through the body to the skin, the electrical energy is still present in the right atrium and can be detected by local endovascular measurements as a non-coherent P-wave, i.e., as significant electrical activity in the P-segment of the ECG waveform. Energy measurements are also less sensitive to some local abnormalities in impulse conduction: altered automaticity (arrhythmias), retrograde conduction of impulses, reentry abnormalities.

The electrical signals and the electrical energy distribution are also advantageously used to quantify heart functionality, e.g., preload which is related to the depolarization and extension of the heart muscle.

The electrical signals and the electrical energy distribution are also advantageously used to guide guidewires and guiding catheters through the aorta into the left heart. This method is useful in simplifying the access to the left atrium and to the coronary arteries and in reducing the amount of contrast and radiation needed to guide endovascular devices to those locations. In a different application, the inventive apparatus can also be used to guide catheters, e.g. Swan-Ganz through the right ventricle into the pulmonary artery. Other endovascular devices can be guided and be used to measure endovascular electrical activity in other locations of the cardiovascular system which are identifiable by the cardiograms measured with the new apparatus introduced in embodiments of the present invention.

FIG. 15 illustrates electrical activity in the cardiovascular system due to neuronal control system. Several paths of conduction are related to the mechanism of control of heart (1530) and blood vessel (1520) activity: receptors (1510), e.g., pressure receptors transmit information related to the state of the blood vessels and to the state of the heart to the nervous system through the Medullary centers (1500). The hypothalamus (1540) and the higher centers (1550) are involved in processing and reacting to the information received from the sensors/receptors. In turn they send impulses (1560) back to blood vessels and the heart. By measuring electrical activity related to the control system, information regarding heart conditions can be obtained which could not have been obtained previously.

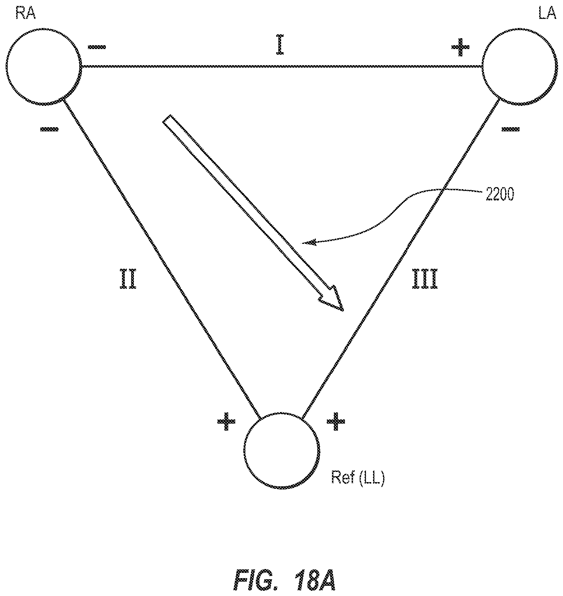

FIG. 18A illustrates the Einthoven ECG triangle and the naming convention for the ECG leads as they are used herein in connection with various embodiments. In order to obtain ECG signals from the patient, typically, one electrode is placed on the right arm (RA), one on the left arm (LA) and one is used as reference on the left leg (LL). The direction in which the P-wave changes most is shown by the arrow (2200). Therefore, when using endovascular ECG for catheter navigation and tip location, the electrode corresponding to the right arm (RA) is operably connected to the proximal end of the vascular access device (110) (FIG. 1A), such as a catheter, in one embodiment. In this way, an ECG waveform detected with respect to the distal end of the catheter, e.g., via an electrode disposed on the catheter, can be considered as detected by Lead II of the Einthoven triangle. Thus, when the catheter is advanced through the vasculature, Lead II will show most significant changes of the P-wave and therefore is best suited to detect the proximity of the sino-atrial node. The sino-atrial node is located at the caval-atrial junction and is responsible for generating the P-wave (indicative of the right-atrial electrical activity). The waveform corresponding to Lead III in the Einthoven triangle remains relatively unchanged as the catheter navigates through the vasculature in one embodiment if the RA electrode is operably connected to the catheter. Therefore, Lead III is used in an embodiment of the present invention as a reference lead which serves multiple purposes, as described herein. In one embodiment, the apparatus introduced herein displays simultaneously ECG signal-based waveforms for Lead II, also referred to herein as the endovascular ECG lead, (for catheter navigation and tip positioning) and for Lead III, also referred to herein as the skin ECG lead, (as a reference waveform).

Reference is again made to FIG. 5, which illustrates the mapping of different endovascular ECG waveforms to the corresponding locations in the vasculature and in the heart, according to one embodiment. In detail, Location A corresponds to the upper superior vena cava (SVC), Location B corresponds to the lower 1/3 of the SVC, Location C corresponds to the caval-atrial junction, Location D corresponds to the right atrium, and Location E corresponds to the lower atrium and/or to the inferior vena cava.

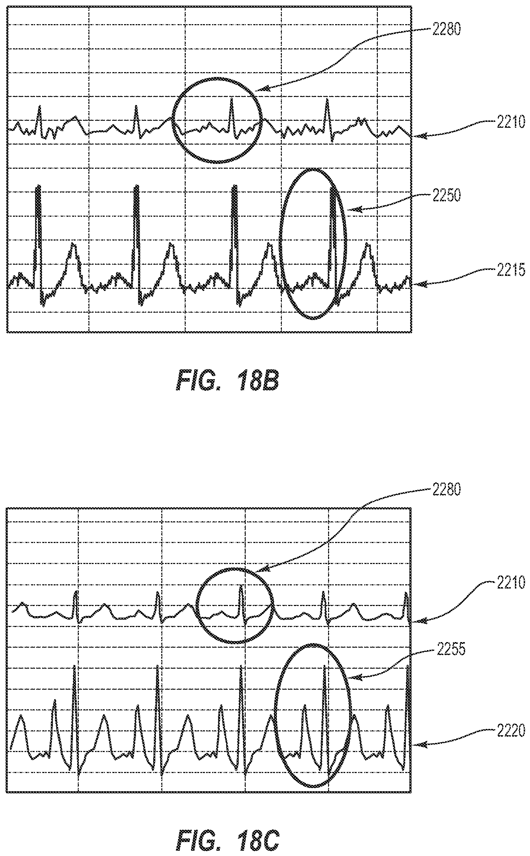

FIG. 18B illustrates the endovascular (Lead II) ECG waveform (2215) obtained with a device disclosed herein, such as an ECG sensor-containing catheter, as measured at Location A in FIG. 5. The skin ECG waveform (2210) represents a skin reference ECG lead equivalent with Lead III. A reference P-R complex is illustrated by (2280). The typical P-R complex at Location A is illustrated by (2250). While the P-wave changes dramatically on Lead II according to movement of the catheter and its ECG sensor within the vasculature, as seen in the P-R complex (2250) for instance, the P-wave remains substantially constant on Lead III used as a reference (2280).

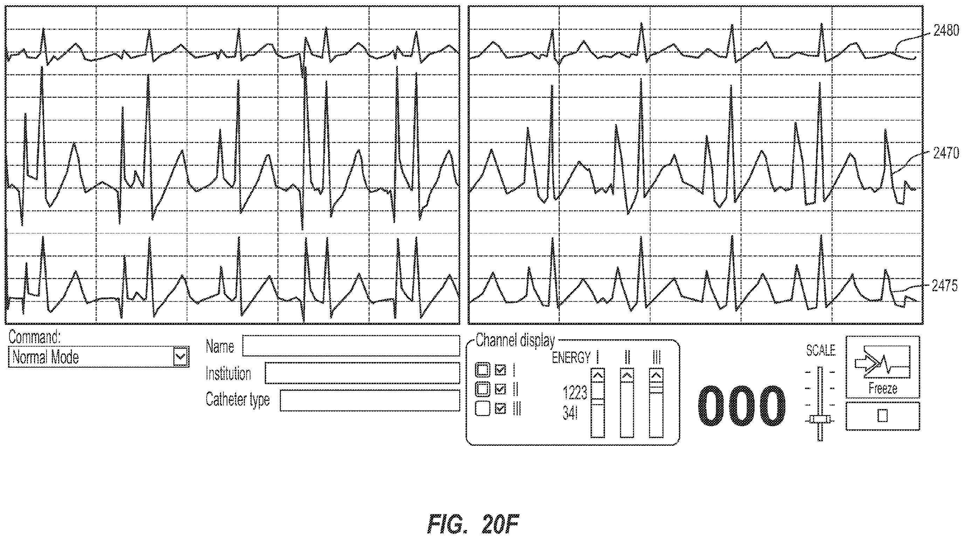

In one embodiment, waveforms from two ECG leads (e.g., Lead II and III in FIG. 18B) are simultaneously depicted on a display of an apparatus, such as a catheter placement system for example, as illustrated in FIGS. 18B-18F. In another embodiment, three leads (Lead I, II, and III of FIG. 18A) may be displayed at the same time as shown in FIG. 20F.

By using the method, apparatus, and ECG electrode configuration introduced herein, it is possible in one embodiment to monitor the patient condition, e.g., the patient's heart rate using the skin reference lead (Lead III) while at the same time guiding the catheter placement using endovascular Lead II.

FIG. 18C illustrates the endovascular ECG waveform (2220) obtained with the device disclosed herein as measured at Location B in FIG. 5. The skin ECG waveform (2210) represents a skin reference lead equivalent with Lead III. A reference P-R complex is illustrated by (2280). The typical P-R complex at Location B is illustrated by (2255). As before, while the P-wave changes dramatically in the P-R complex (2250) on Lead II corresponding to the tip of the catheter, the P-wave remains quite constant on Lead III used as a reference (2280).

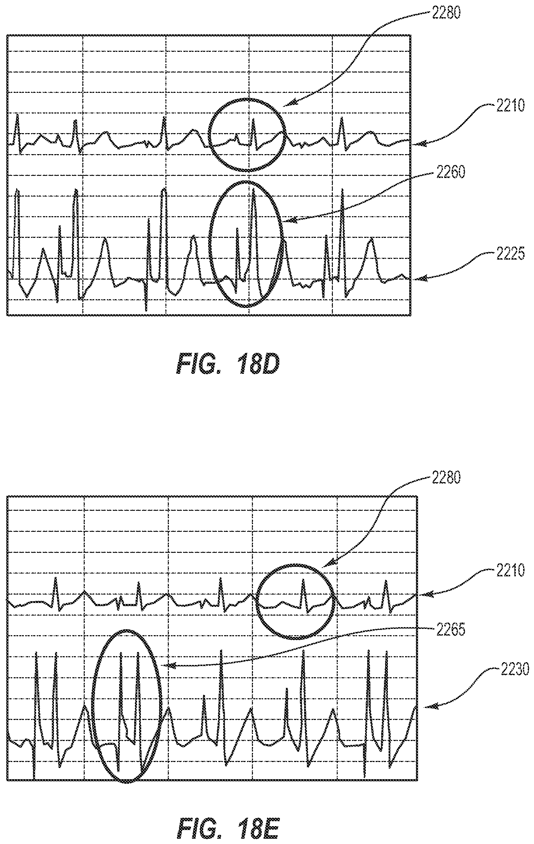

FIG. 18D illustrates the endovascular ECG waveform (2225) obtained with the device disclosed in an embodiment of the present invention at Location C in FIG. 5. The ECG waveform (2210) represents a skin reference lead equivalent with Lead III. A reference P-R complex is illustrated by (2280). The typical P-R complex at Location C is illustrated by (2260). While the P-wave changes dramatically in the P-R complex (2260) on Lead II corresponding to the tip of the catheter, the P-wave remains quite constant on Lead III used as a reference (2280).

FIG. 18E illustrates the endovascular ECG waveform (2230) obtained with the device disclosed in an embodiment of the present invention at Location D in FIG. 5. The ECG waveform (2210) represents a skin reference lead equivalent with Lead III. A reference P-R complex is illustrated by (2280). The typical P-R complex at Location D is illustrated by (2265). While the P-wave changes dramatically in the P-R complex (2265) on Lead II corresponding to the tip of the catheter (265), the P-wave remains quite constant on Lead III used as a reference (2280).

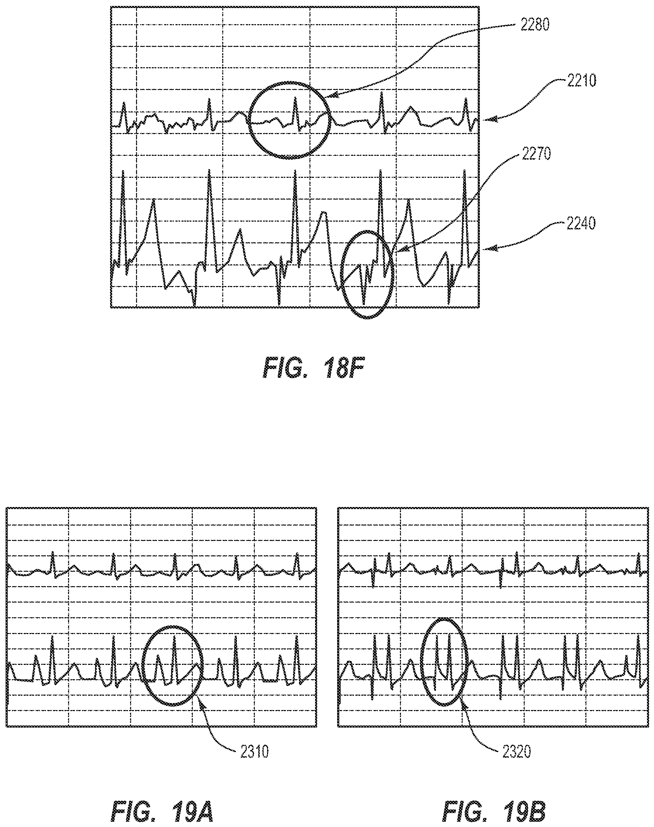

FIG. 18F illustrates the endovascular ECG waveform (2240) obtained with the device disclosed in an embodiment of the present invention at Location E in FIG. 5. The ECG waveform (2210) represents a skin reference lead equivalent with Lead III. A reference P-R complex is illustrated by (2280). The typical P-R complex at Location E is illustrated by (2270). While the P-wave changes dramatically in the P-R complex (2270) on Lead II corresponding to the tip of the catheter, the P-wave remains quite constant on Lead III used as a reference (2280).

FIG. 19A illustrates the ability of the apparatus introduced herein, e.g., a catheter placement system, to show several display windows at the same time on the screen thereof. One, two, or more display windows can be included. Each of the display windows (3310 and 3320) can display one to three ECG waveforms (leads I, II, and III) in any combination, in real-time acquisition, in playback, or frozen modes. In one embodiment, one display window (3310) is used to show real-time ECG waveforms (catheter guiding, or endovascular, lead II and skin reference lead III) and another display window (320) is used to show frozen ECG waveforms (catheter guiding lead II and skin reference lead III). Thus, the user can compare the changes in the catheter guiding lead and in particular in the P-R complex at two different catheter tip locations: at the tip location frozen in display window (2320) and at the current (real-time) tip location displayed in window (2310).

The above multi-window comparison enables the use of the following catheter placement method, according to one embodiment: first advance the catheter in the atrium until the P-wave reaches its maximum amplitude as seen in window (2320) (FIG. 19B) and then pull back the catheter to a location where the P-wave is half the size of its maximum amplitude. Such a location where the amplitude of the P-wave is half the size of its maximum amplitude is indicative of the lower third of the superior vena cava (Location B in FIG. 5).

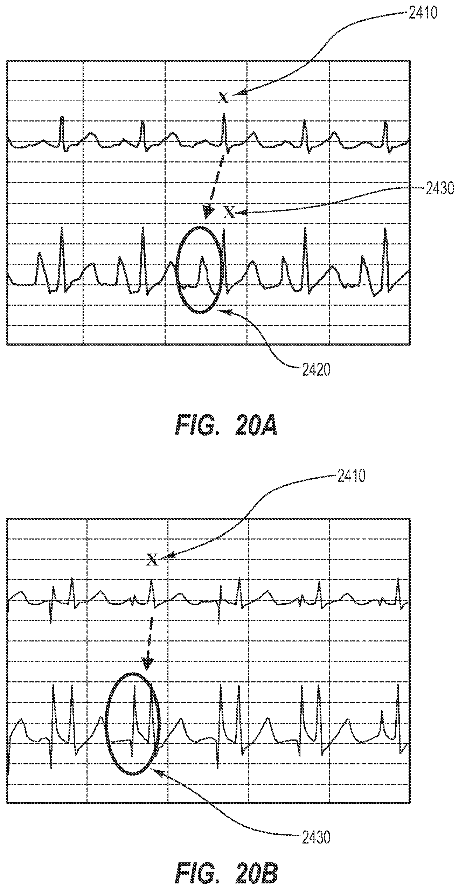

FIG. 20A illustrates how the skin reference lead can be used to analyze the P-wave segment of the catheter guiding lead (Lead II), according to one embodiment. The P-wave segment, in which is found the P-wave itself, is characterized by the fact that it immediately precedes the QRS complex of the same heart beat. The P-wave segment of a heart beat also follows the T-wave of the previous beat. In order to detect the P-wave segment, an algorithm can be applied including detection of the R-peak of the QRS complex. The algorithm in one embodiment includes the following steps:

Detect R-peak.

Compute R-R interval.

Assume that a certain percentage of the R-R interval prior to the R-peak is the interval in which the P-wave occurs. This interval where the P-wave occurs is defined as the P-wave segment.

Detect the P-peak in the P-wave segment, its amplitude and polarity.

Apply processing, analysis and decision making algorithms as illustrated in FIGS. 11 and 12.

In one embodiment, in order to apply the algorithm described above, the R-peak and the R-R interval can be detected on endovascular Lead II, i.e., on the same ECG lead which is used for guidance. In another embodiment, the R-peak and the R-R interval can be detected using Lead III (the skin reference lead). In particular, the R-peak detection on Lead III (2410) in FIG. 20A can be used to trigger the analysis of any segment of the ECG waveform on Lead II including the analysis of the P-wave segment (2420) in FIG. 20A. It is also possible, if the signal quality of Lead II allows, to use the R-peak (2430) detected on lead II itself to trigger processing of the Lead II waveform. In other embodiments, other leads can be used to implement triggering on a different lead than the one used for catheter navigation and tip positioning. For example, Lead I can optionally be used for catheter navigation and tip positioning. The apparatus according to one embodiment allows also the use of Lead I for catheter navigation and tip positioning, though Lead II is suitable in many clinical settings. Note that in one embodiment the above triggering can occur for peaks on a waveform detected by the same lead. Also, a peak detected on Lead II can be used to trigger analysis of Lead I, in one embodiment. Thus, these and other variations are contemplated.

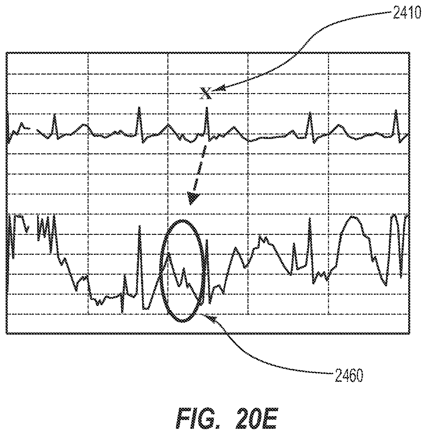

Triggering analysis on one ECG lead that is different than the ECG lead used for catheter navigation and positioning as introduced herein is useful in many practical situations regardless of which ECG lead is used for triggering the analysis and which ECG lead is used for catheter navigation and positioning. As will be seen in FIGS. 20B-20E and especially in FIG. 20E, triggering on a stable, noiseless lead, e.g., Lead III improves the ability to process different segments of other leads, e.g., endovascular Lead II used for catheter navigation and positioning in cases where the Lead II ECG signal includes a greater than desired amount of signal noise. Noisy Lead II ECG signals appear quite frequently in practical settings because of the manual handling of the Lead II connection by the user. Other situations can benefit from the trigger concept introduced herein, as will be seen below.

FIG. 20B illustrates how the R-peak detected on reference skin Lead III (2410) and the corresponding R-R interval trigger the analysis of the PQRS segment (2430) on the navigation Lead II. As described herein, the P-wave segment and the QRS complex of the ECG Lead II can be analyzed separately or in relationship to each other in order to predict the location of the catheter tip in the vasculature. In the case shown in FIG. 20B, the P-wave has a large positive amplitude which is equal to the R-amplitude and is also bipolar (has a negative first segment). In such a case, the detection of the R-peak on the Lead II itself is very difficult if not impossible through the use of algorithms. Triggering the ECG waveform analysis of Lead II (2430) based on detection of the R-peak detected on the reference Lead III (2410), as introduced herein, allows for detection and processing of the changes of the P-wave segment characteristics of the catheter tip location. Such algorithmic ECG waveform analysis of Lead II would otherwise be difficult in the case shown in FIG. 20B because of the difficulty of clearly detecting the R-peak on this lead.

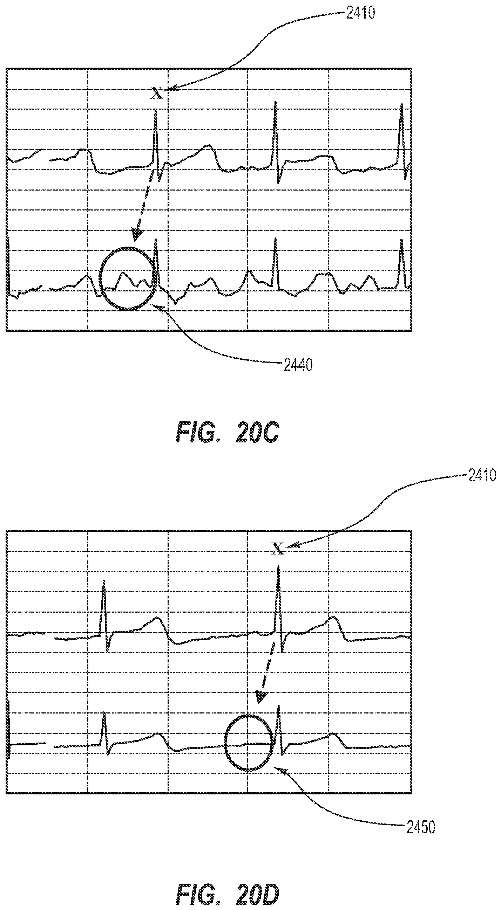

FIG. 20C illustrates how triggering on the R-peak of one lead, for example the R-peak of Lead III (2410) can be used to trigger the analysis of the P-wave segment on catheter navigation Lead II (2440) in the case of a patient with arrhythmias. Typically, the P-wave segment is not present in the skin ECG lead in patients with arrhythmias, as seen in FIGS. 20C and 20D. However, the catheter navigation and tip positioning lead, e.g., Lead II, can detect a relatively higher level of electrical activity in the P-wave segment as the catheter approaches the sino-atrial node and the caval-atrial junction. The level of electrical activity (energy) in the P-wave segment increases further as the catheter tip passes the sino-atrial node and enters the right atrium. Since the highest level of this increased electrical activity on the P-wave segment of navigation Lead II cannot be predicted, e.g., the P-wave amplitude could be higher than that of the R-wave on the Lead II, triggering the analysis of the said P-wave segment on the R-peak of a skin ECG lead provides a suitable solution to P-wave detection and subsequent catheter tip location and positioning.

FIG. 20D illustrates the lack of P-wave in the case of an arrhythmia patient on both ECG leads II and III. In FIG. 20D, lead II is connected to a skin electrode on the right arm of the patient and lead III to a skin electrode on the left arm of the patient. The R-peak on lead III (2410) is depicted in this figure and the corresponding segment showing the absence of a discernible P-wave on lead II is shown as (2450).

FIG. 20E illustrates the situation in which the catheter navigation lead, e.g., Lead II, is noisy or unstable and the detection of the R-peak and of the corresponding P-wave is thus difficult. In this case, as before, detecting the R-peak (2410) on a stable reference lead, e.g., the skin Lead III, preserves the ability via the above-described triggering, to find and analyze the P-wave segment (2460) on the noisier catheter navigation lead.

FIG. 20F illustrates another embodiment, wherein two leads (in this example Leads I and II- see FIG. 18A) are used to detect simultaneous, corresponding ECG waveforms (2470) and (2475) and triangulate, together with an additional, simultaneous ECG waveform (2480) of the reference lead (Lead III), the location of the catheter tip. In particular, a substantially accurate location of the catheter tip can be determined by looking at Leads I and II at the same time and use their correlation (or lack thereof) to reduce noise and more accurately determine the changes of the P-segment, of the QRS segment, and the relative changes between the P-wave and the QRS complex.

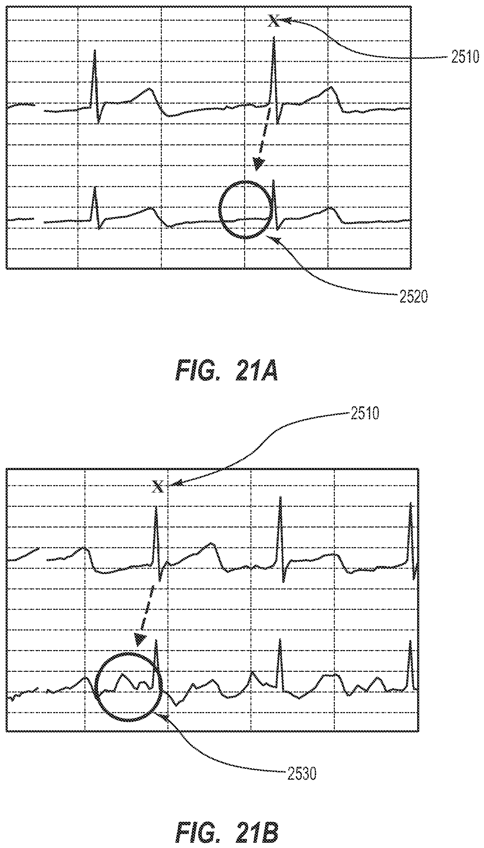

FIGS. 21A and 21B illustrate details regarding an algorithm to use the P-wave segment and/or its relationship with the QRS complex for catheter navigation and tip location in the case of arrhythmia, according to one embodiment.

Specifically, FIG. 21A illustrates the ECG waveforms for two skin ECG leads (using skin electrodes). In FIG. 21A, Lead III with its corresponding R-peak (2510) is detected using the skin left arm electrode and Lead II showing the lack of the P-wave (2520) is detected using the skin right arm electrode, both being compared with the skin left leg electrode, in one embodiment. Previously, patients showing these typical arrhythmia ECG waveforms were not considered as candidates for using the ECG-based method for catheter navigation and tip location. It was believed that, because the P-wave is not present at the skin level, the ECG method cannot be used to determine catheter tip location at the caval-atrial junction. FIG. 21A thus illustrates a situation where the R-peak of the skin reference lead (2510) can be used to compute the characteristics and the energy of the P-segment (P-wave) on the navigation lead at locations where the P-wave is not present.

In greater detail, FIG. 21B illustrates ECG waveforms as obtained with the apparatus described in connection with FIGS. 20A-20E and show that, with the apparatus and method described herein, even arrhythmia patients can be treated using ECG-based catheter navigation and tip location. Due to the processing algorithms described in FIGS. 11 and 12, the ECG signal obtained from the tip of the catheter on Lead II is more accurate and less noisy when compared to prior art. Thus, changes in the P-wave segment (2530) become visible when the catheter tip is in the proximity of the sino-atrial node. They correspond, as justified by physiology, to a random electrical activity of the right atrium. This random electrical activity and its changes can be detected with the apparatus introduced herein as illustrated by the P-wave segment (2530). This random electrical activity typically cancels out once reaching the skin and the Lead III and thus is difficult or impossible to detect by prior art ECG methods.

Sometimes the above random electrical activity of the right atrium is also very weak and an apparatus such as the one introduced herein is needed to detect it even at the tip of the catheter. By observing and/or analyzing the changes of the P-wave segment on the catheter navigation lead, the tip location of the catheter can be mapped, for example, to locations in the superior vena cava (weak, low energy or no P-wave), to locations at the caval atrial junction and to locations in the right atrium. FIG. 21B illustrates how the R-peak on the reference lead (e.g., skin Lead III) can trigger the analysis of the corresponding P-wave (P-segment) on the navigation lead (e.g., endovascular Lead II) at locations where a P-wave segment (2530) is present.

In addition to those described in FIGS. 11 and 12, it is appreciated that other decision algorithms can be used, such as those related to the level of electrical energy as calculated from the electrogram spectrum, in placing a catheter or other endovascular devices. For instance, one criterion specifies that at the location corresponding to the lower third of the SVC, the average electrical energy calculated from the endovascular electrogram is twice as high as the average electrical energy calculated from the endovascular electrogram at skin level, e.g., from a skin electrocardiogram in a corresponding lead, e.g., Lead III.

In addition to the algorithms disclosed above in connection with FIGS. 11 and 12, the concept of directional energy and of decision making based thereon are introduced herein. As seen, for example, in FIGS. 18B at (2250) and 18C at (2255), the P-wave is uni-polar, i.e., has a single polarity, the polarity being positive. In comparison, FIGS. 18D at (2260) and 18E at (2265) illustrate a bi-polar P-wave, i.e., a P-wave which has both a negative and a positive component. FIG. 18F illustrates a P-wave segment at (2270) with a uni-polar P-wave segment but of polarity reversed compared to that of the P-wave segment shown in FIGS. 18B and 18C.

The above change of polarity in the P-wave segment is due to the location of the catheter tip relative to the sino-atrial node and of the locations of the skin electrodes according to the Einthoven triangle (FIG. 18A). In the cases illustrated herein, as the catheter navigates from the superior vena cava through the caval-atrial junction, through the right atrium and into the inferior vena cava, the polarity of the P-wave segment changes correspondingly.

According to one embodiment and in light of the above, catheter tip location can be determined by the following: a positive energy value and a negative energy value are determined for a detected P-wave by the apparatus described herein, such as a catheter placement system. The positive P-wave energy value is determined according to the energy computation algorithms described herein, but only for positive values of the P-wave (i.e., values above the ECG baseline). Correspondingly, the negative P-wave energy value is determined according to the energy computation algorithms described herein, but only for negative values of the P-wave (i.e., values below the ECG baseline). These energy values (positive and negative) determined according to the present embodiment are also referred to herein as "directional energy" values because they are related to the direction and location of the catheter tip at which point the P-wave is being detected via an appropriate sensor in operable connection with a corresponding ECG lead, such as the endovascular Lead II discussed above.

The P-wave directional energy described above can be used to guide navigation of a catheter and for locating a tip thereof, according to one embodiment. Particularly, in one embodiment a standard Einthoven electrode configuration, with the right arm electrode detecting endovascular ECG signals at the catheter tip (as described above in connection with FIGS. 20A-20E) is considered. Note that other electrode configurations are also possible. If the P-wave energy is substantially entirely positive, the catheter tip is considered to be located above the sinoatrial node, for example in the superior vena cava. If the P-wave includes positive energy and a relatively small amount of negative energy, but the positive energy is smaller relative to the R-wave energy, such as is seen at (2260) in FIG. 18D, the catheter tip may be located at the caval atrial junction. If the P-wave segment includes a large amount of negative energy relative to its positive energy, and the positive energy is comparable to that of the R-wave energy, such as is seen in at (2265) in FIG. 18E, the catheter tip may be in the right atrium. If the P-wave includes substantially entirely negative energy, as seen at (2270) in FIG. 18F, the catheter tip is approaching the inferior vena cava or is in the inferior vena cava. In this way, the directional energy introduced herein is used by the present method described herein for catheter navigation and tip location.

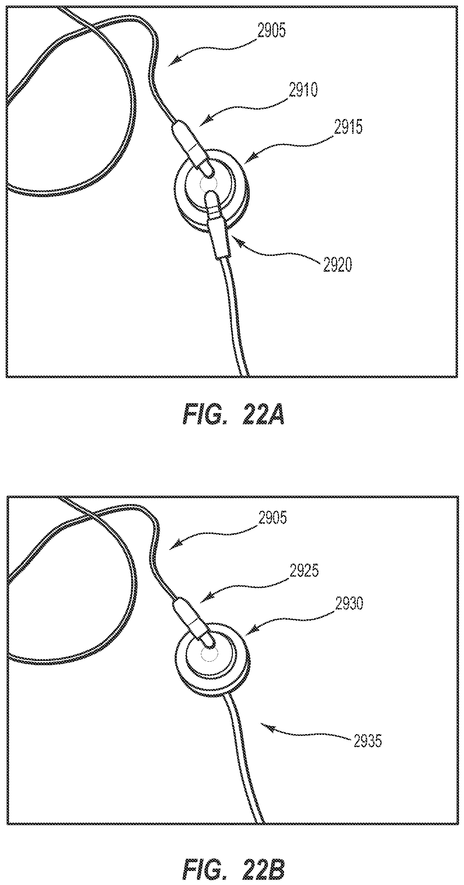

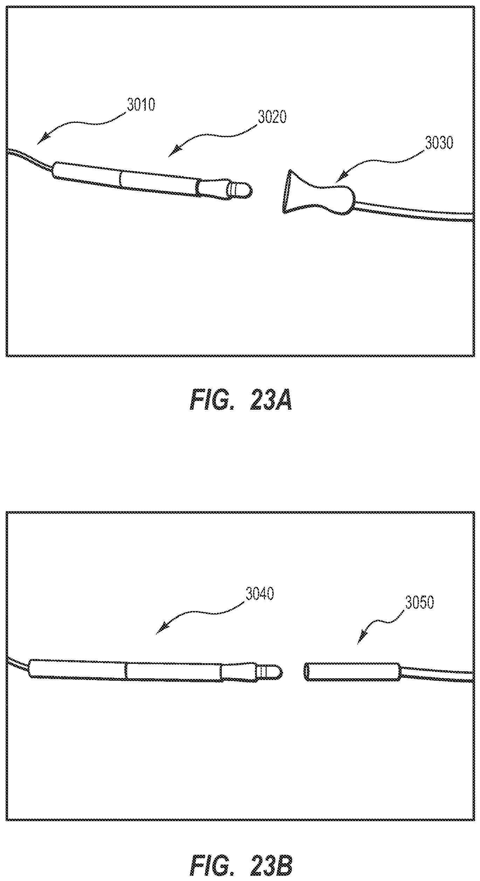

FIGS. 22A-22D and 23A-23B illustrate various details regarding a connector according to example embodiments, which allow for the use of the apparatus and method described herein by a single operator in the sterile field.

In particular, FIG. 22A shows a connecting object (2915) including magnetic attraction properties and a surface including electrically conductive properties. The connecting object (2915) electrically connects to two connectors (2910) and (2920). The connector (2910) is connected to one end of a sterile device / adaptor (2905). The other end of the sterile device (2905) can be connected to a sterile guidewire or stylet or to a sterile saline adaptor as described further above. The connector (2920) can be attached to or be itself one end of an EGC cable connected to the apparatus illustrated herein in FIG. 1A.

The surface of the connecting object (2915) can be implemented in several ways. In one embodiment, a magnet is built into an enclosure with an electrical conductive surface. The magnet attracts the electrical connectors (2910) and (2920) to the metallic surface and locks them on the surface, thus establishing electrical contact between connector (2910) and the electrically conductive surface of the connecting object (2915) and another electrical contact between the electrically conductive surface of the connecting object (2915) and the other electrical contact (2920).

The connecting object 2915 illustrates one type of connector that can be used with the methods described herein by a single operator in the sterile field. Accordingly, in one embodiment, the object (2915) is placed prior to the commencement of a catheter placement procedure in the non-sterile field such that it can be reached by the single sterile operator during the procedure. Then, the as-yet non-sterile operator connects one end of the non-sterile connector (2920) to an ECG cable and "drops" the connector end shown in FIG. 22A onto the surface of the connecting object 2915. Because of the magnet incorporated in the object (2915), the connector (2920) is attracted to the electrically conductive surface of the connecting object and adheres to the surface thereof. The end of the ECG cable to which the connector (2920) is attached or included can be an ECG lead itself, thus simplifying the workflow.

During the procedure, the single operator is sterile. The operators opens the sterile package in which the connector (adaptor) (2910, 2905) is packaged, holds the sterile connector end (2915) with a sterile gloved hand and drops the sterile connector onto the electrically conductive surface of the connecting object (2915). Similarly to connector (2920), the connector (2910) is magnetically attracted to the connecting object (915) by the built-in magnet, which secures the connector (2910) on the electrically conductive surface of the connecting object. Using this method, an electrical connection can be established between a sterile electrical connector (2910) and a non-sterile connector (2920) without compromising the sterile field. Again, this method can be used by a single operator and enables the single sterile operator use of the apparatus described herein.

FIG. 22B illustrates another embodiment of the connector, wherein the connecting object (2930) is directly connected to a wire or is an integral part of an ECG cable (2935). This embodiment simplifies the method described above in connection with FIG. 22A, as only the sterile connector (2925) connected to the sterile adaptor (2905) must be dropped on to the electrically conductive surface (2930) of the connecting object (2930) during the sterile procedure.

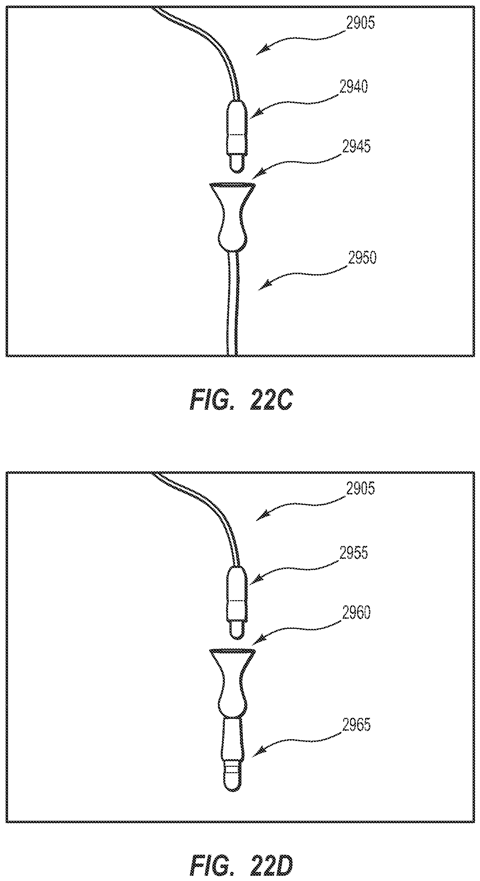

FIG. 22C illustrates another embodiment of the connecting object, wherein a connector (2940) of the sterile adaptor (2905) is similar to the adapter (2905) and connector (2910) described above in connection with FIG. 22A. During a catheter placement procedure, the sterile operator drops the sterile connector (2940) into a connecting object, or mating piece (2945). The mating piece (2945) includes a cup that receives therein the connector (2940). A magnet is built into the cup, which attracts the connector (2940) into the cup to secure it therein. At the same time, the cup ensures electrical connectivity. The mating piece (2945) can be an integral part of an ECG cable (2950) (e.g., one end of an ECG lead), one end of a wire for connection to an ECG cable, or some other suitable configuration. The method for using the mating piece 2945 is similar to that described in connection with FIG. 22B, with a difference being that the cup (2945) has the ability to suck in the connector (2940) for a relatively secure male/female-type connection. As is the case with the embodiments described in connection with FIGS. 22A, 22B, and 22D, the shapes and materials used for the connecting objects can vary while still ensuring proper electrical contact for the component interconnected therewith.

FIG. 22D illustrates a connecting object configuration similar to that described in connection with FIG. 22C, except that a cup (2960), which contains a magnet for operably connecting with a connector (2955), includes on an opposite end a connector (2965) to which an ECG cable clip can be attached. As such, during a placement procedure a non-sterile operator can connects the connector (2965) to a commercially available ECG cable by using the clip provided with the ECG cable. Later, during the sterile procedure, the sterile operator drops the sterile connector (2955) into the cup (2960), similar to the method described in connection with FIG. 22C.

FIG. 23A illustrates details of a steerable sterile adaptor (3010) according to one embodiment, including a reinforced sterile connector piece (3020) of rigid plastic, for instance. Instead of dropping the sterile connector piece (3020) into a mating piece (3030) as in FIGS. 22C and 22D, the sterile operator can use the rigid connector piece (3020) of the sterile adaptor (3010) to steer, e.g., push, rotate, etc., it into the mating piece. In one embodiment, the mating piece (3030) includes a built-in magnet to attract the connector piece 3020. In another embodiment, the mating piece (3030) includes no magnet, but is of an appropriate size and shape for the connector piece (3020) to fit therein so as to establish suitable electrical contact therebetween.

FIG. 23B illustrates a steerable connector piece (3040) according to one embodiment, which can be pushed into and operably connect with a simple mating piece (3050) without the need of a magnet. In addition to what has been shown and described, other shapes are possible for the connector (3040) and its mating piece (3050), e.g. rails or screws.

It is appreciated that any suitable combination of the connector embodiments discussed above can be used. For example, the steerable connector of FIG. 23B can include a mating piece like that shown in FIG. 22D.

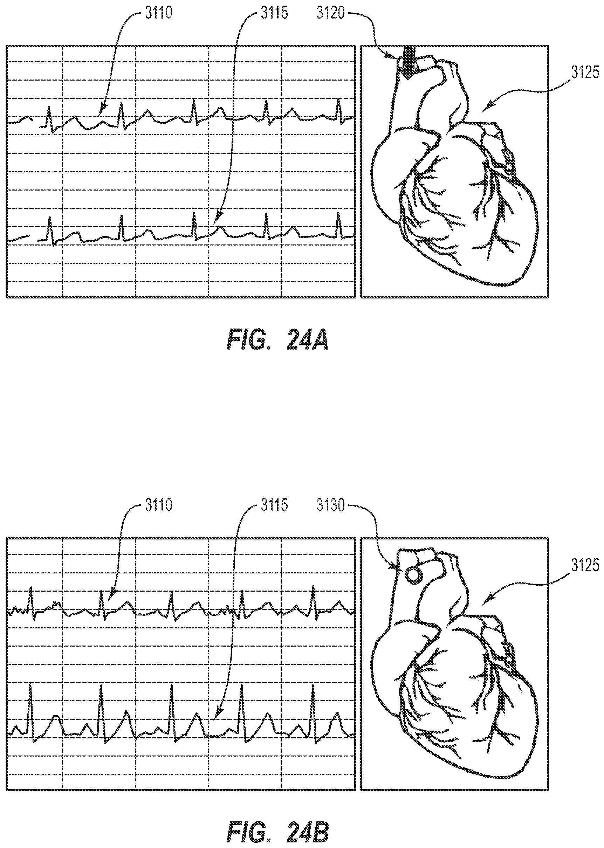

FIGS. 24A-24F illustrate various details of catheter navigation according to one embodiment. As shown, each of these figures includes two display windows: a first window showing ECG waveforms, and a second window showing a representation, or icon, of a heart and an additional location icon indicating the point of measurement of the ECG signal to which the ECG waveforms in the first window correspond. The mapping between ECG waveforms and the location icon is performed in one embodiment using the algorithms and methods described above. The two display windows can be used independently or together. In one embodiment, the two display windows are shown simultaneously on the graphical user interface (FIG. 1A) in order to allow the operator to correlate the observed ECG waveform(s) with catheter tip location. In another embodiment, only the heart and location icon window is shown in order to simplify the user interface.

The location icon can include any one or more of several possible configurations, including an arrow to show advancement in a certain direction, a dot, a cross, a star, etc., to show an identifiable location. Each of these icons can include different colors to emphasize location relevance. In another embodiment, different sounds can be associated to each of the identifiable tip locations. Sounds and icons identifying tip locations can be used together or independently to help the user navigate the catheter and locate the tip within the patient vasculature.

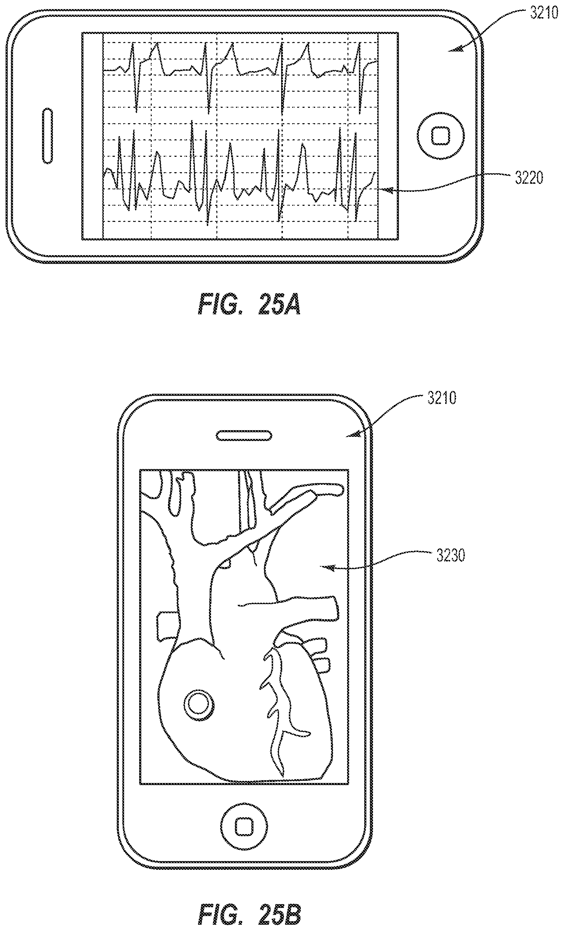

In one embodiment a simplified user interface is employed, wherein only the heart icon and the corresponding location icon(s) are displayed. In this instance, the ECG waveforms and the computation behind the location mapping are not visible to the user. Thus, the apparatus described herein can be employed for navigation and tip location without requiring user interpretation of the ECG waveforms. The simplified user interface with only the heart and catheter tip location icons can be used as shown in the embodiment illustrated in FIG. 25B, for instance.

In greater detail, FIG. 24A illustrates ECG waveforms corresponding to catheter tip locations outside the thoracic cavity in the upper body: a skin reference ECG Lead III (3110) and an endovascular catheter navigation ECG Lead II (3115). In the icon display window, a heart icon (3125) is displayed and a location icon (3120) shows that the catheter is moving towards the thoracic cavity. In another embodiment, the arrow-shaped location icon (3120) can be replaced with a cross, a dot, or any other suitable icon showing location above and outside the superior vena cava.

The arrow-shaped location icon (3120) is displayed by the apparatus according to one embodiment only if the algorithms detect changes in the navigation ECG Lead II that support the fact that the catheter tip is moving towards the heart, e.g., a steady increase in the electrical energy and a P-wave with positive directional energy, indicating that the tip is approaching the sino-atrial node. If the algorithms do not detect a steady increase in the electrical energy of the endovascular ECG signal as the catheter advances through the vasculature, only a dot, star, cross or other suitable location icon is displayed at a location above and outside the superior vena cava. Sounds associated with each of these locations and situations can be played in addition to or instead of the graphical icons.

FIG. 24B illustrates the ECG waveforms corresponding to the reference lead (3110) and catheter navigation lead (3115) at a location corresponding to the upper superior vena cava. The icon display window shows the heart icon (3125) and a dot-shaped location icon (3130) indicating the upper superior vena cava on the heart icon. This location is determined by the apparatus, as described further above, based on the ECG waveforms (3110) and (3115). As in FIG. 24A, any suitable icon shape and color can be used, and/or a sound or tune can be played when the tip of the catheter reaches the location indicated by the detected ECG waveforms.

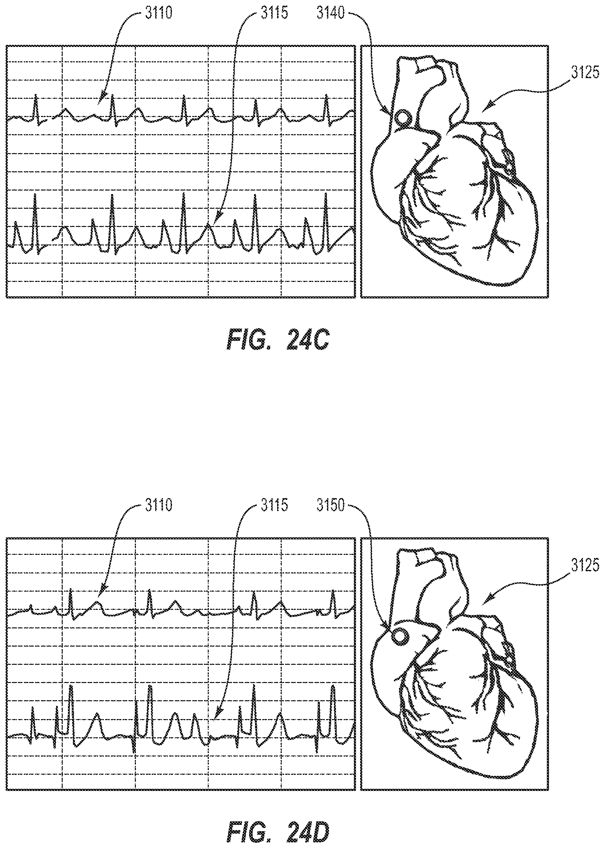

FIG. 24C illustrates the ECG waveforms corresponding to the reference lead (3110) and catheter navigation lead (3115) at a location corresponding to the lower third of the superior vena cava. The icon display window shows the heart icon (3125) and a dot-shaped location icon (3140) indicating the lower third of superior vena cava on the heart icon. This location is computed by the apparatus, as described further above, based on the ECG waveforms (3110) and (3115). As in FIG. 24A, any suitable icon shape and color can be used and/or a sound or tune can be played when the tip of the catheter reaches the location as indicated by the detected ECG waveforms.

FIG. 24D illustrates the ECG waveforms corresponding to the reference lead (3110) and catheter navigation lead (3115) at a location corresponding to the caval-atrial junction. The icon display window shows the heart icon (3125) and a dot-shaped location icon (3150) indicating the caval-atrial junction on the heart icon. This location is computed by the apparatus, as described further above, based on the ECG waveforms (3110) and (3115). As in FIG. 24A, any suitable icon shape and color can be used and/or a sound or tune can be played when the tip of the catheter reaches this location as indicated by the detected ECG waveforms.

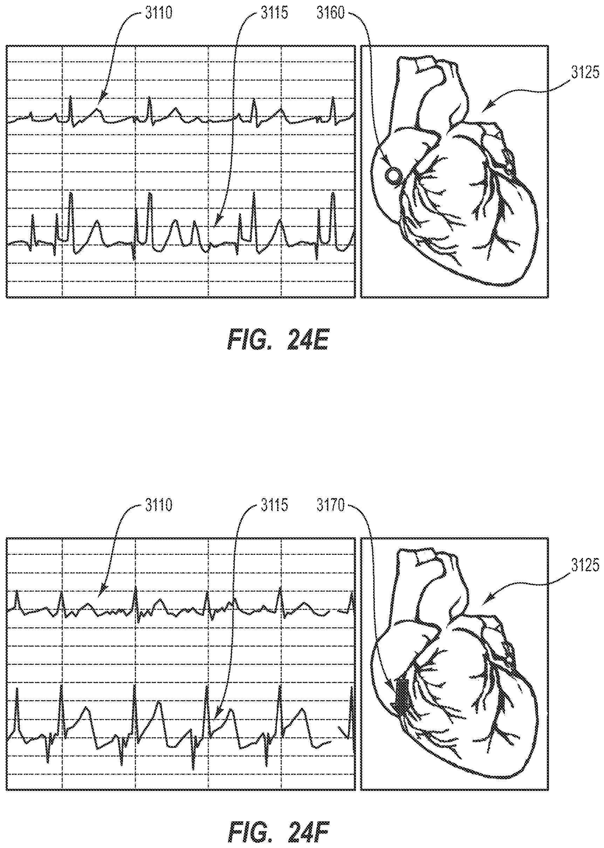

FIG. 24E illustrates the ECG waveforms corresponding to the reference lead (3110) and catheter navigation lead (3115) at a location corresponding to the right atrium. The icon display window shows the heart icon (3125) and a dot-shaped location icon (3160) indicating the right atrium on the heart icon. This location is computed by the apparatus, as described further above, based on the ECG waveforms (3110) and (3115). As in FIG. 24A, any suitable icon shape and color can be used and/or a sound or tune can be played when the tip of the catheter reaches this location as indicated by the detected ECG waveforms.

FIG. 24F illustrates ECG waveforms corresponding to catheter tip locations outside the thoracic cavity in the lower body: the skin reference ECG Lead III (3110) and the endovascular catheter navigation ECG Lead II (3115). In the icon display window, the heart icon is displayed (3125) and an arrow-shaped location icon (3170) showing that the catheter is moving away from the thoracic cavity, such as toward the inferior vena cava. In another embodiment, the arrow-shaped location icon (3170) can be replaced with a cross, a dot, or any other suitable icon showing location below the right atrium.

The arrow-shaped location icon (3170) is displayed by the apparatus in one embodiment only if the algorithms detect changes in the navigation ECG Lead II that support the fact that the catheter tip is moving away from the heart, e.g., a steady decrease in the electrical energy and a P-wave with negative directional energy, indicating that the tip is moving away from the sino-atrial node. If the algorithms do not detect a steady decrease in the electrical energy of the endovascular ECG signal as the catheter advances through the vasculature but detect a negative P-wave, only a dot, star, cross or any other location icon is displayed at a location below and outside the right atrium. The sounds associated with each of these locations and situations can be played in addition to or instead the graphical icons.

FIG. 25A illustrates a display window on a graphical user interface of a mobile phone (3210), tablet PC, or other suitable handheld or portable device. In particular, the user interface of the mobile phone is shown displaying waveforms (3220) of two ECG leads: the reference lead and the catheter navigation lead. The mobile phone or other suitable device is in held in one embodiment in a horizontal position as to allow for a longer time (more heart cycles) of the ECG waveform to be displayed. If the display device is in a real-time display mode, the display switches automatically to showing the ECG waveforms each time the device is turned horizontally. In another embodiment only one ECG lead is displayed at a time. In yet another embodiment, three or more leads can be displayed simultaneously. As described in an embodiment of the present invention, in another embodiment the screen of the display device can be split in real-time to depict a (current location) display window and a frozen (reference location) window to allow for easier assessment of ECG waveform changes. Two-way interaction between the apparatus shown in FIG. 1A and the mobile phone (3210) to enable the functionality shown and described in connection with FIGS. 25A-27B can be achieved in one embodiment via the wireless connectivity component 150, shown in FIG. 1A. It is appreciated that the mobile phone (3210) is equipped with corresponding wireless connectivity so as to enable communication therebetween, as appreciated by one skilled in the art.

FIG. 25B illustrates a simplified user interface (3230) displayed on the screen of a mobile phone (3210) or other suitable handheld/portable device based on the navigation interface described in FIGS. 24A-24F. When in real-time display mode and if the display device is positioned vertically, the device automatically displays the simplified user interface shown in FIG. 25B. When in real-time display mode, the device automatically switches back and forth between displaying ECG waveforms when the device is held horizontally, illustrated in FIG. 25A, and displaying the navigation user interface, illustrated in FIG. 25B when the device is held vertically.

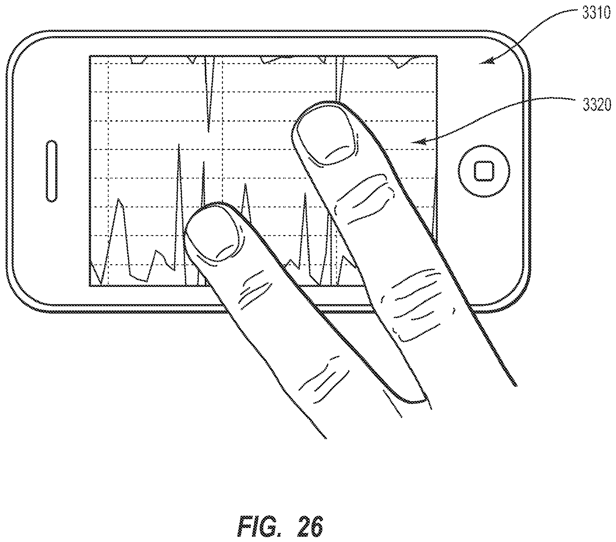

FIG. 26 illustrates, in one embodiment, the zoom and scroll functions on the touch screen of a mobile phone (3310), tablet PC or similar device. The user can use two fingers (3320) to zoom in and out the ECG waveforms for better visualization. Scrolling through the ECG waveform recording can be also achieved using the fingers and the touch screen.

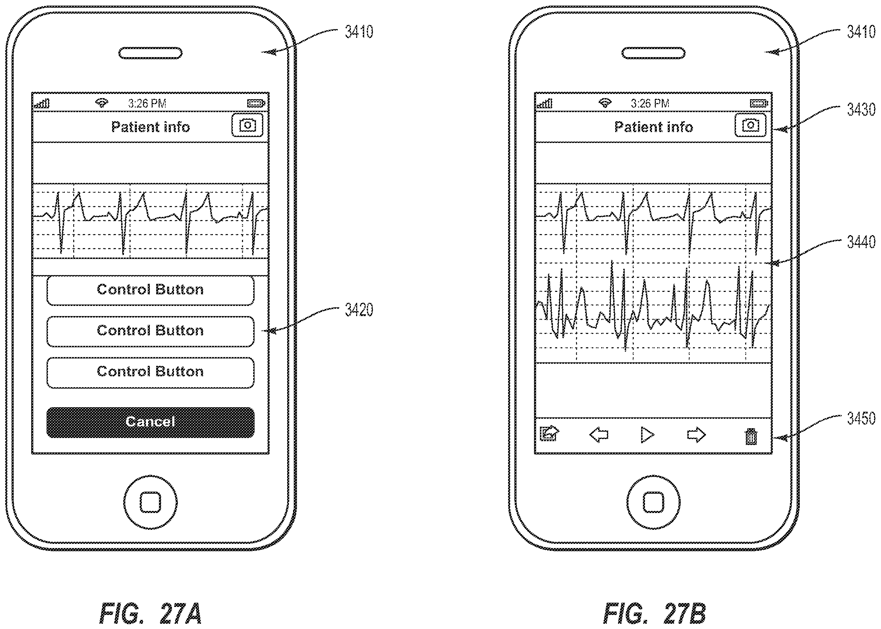

FIG. 27A illustrates the ability, in one embodiment to use a mobile phone (3410), tablet PC, or other suitable handheld/portable device to communicate the ECG waveforms and/or the simplified user interface and patient data to another computer or device. The communication interface is illustrated by (3420). Such transfer can be performed by the mobile phone (3410) via a Wi-Fi, cell phone, or other suitable network.

FIG. 27B illustrates the graphical user interface of a mobile phone (3410), tablet PC, or other suitable handheld/portable device, which allows for the display of patient data (3430), ECG waveforms (3440) or a simplified heart icon depending on the device orientation, and an interface (3450) browsing, control, and disposition of one or more patient records, including deleting, copying to memory, etc.

It is appreciated that the apparatus, algorithms, and methods described herein can be practiced in connection with a variety of environments and using a variety of components and systems. One example of an ECG monitoring system with which embodiments of the present invention can be practiced can be found in U.S. Patent Application Publication No. 2010/0036227, filed September 10, 2009, and entitled "Apparatus and Display Methods Relating to Intravascular Placement of a Catheter." Another example of an ECG monitoring system can be found in U.S. Patent Application Publication No. 2009/0259124, filed April 21, 2009, and entitled "Method of Locating the Tip of a Central Venous Catheter."

Non-limiting examples of ECG sensor stylets that can be used in connection with embodiments of the present invention can be found in U.S. Patent Application Publication No. 2010/0222664, filed August 21, 2009, and entitled "Catheter Assembly Including ECG Sensor and Magnetic Assemblies," and U.S. Patent Application No. 2011/0015533, filed September 29, 2010, and entitled "Stylets for use with Apparatus for Intravascular Placement of a Catheter".

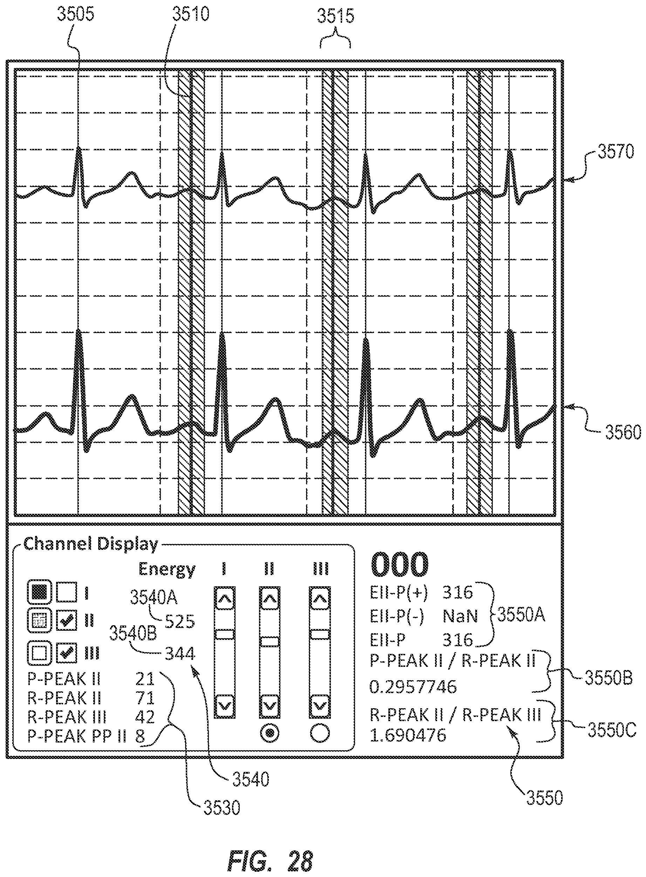

Reference is now generally made to FIGS. 28-39 in noting that additional methods can be employed to assist with the navigation and placement of a vascular access device, such as a catheter, within the vasculature of a patient using ECG signals, according to present embodiments, which are described below. As will be described, these embodiments disclose the automatic detection of regions and parameters of interest in the ECG signals, methods for analyzing the ECG signals to assist with determining in real time the intravascular location of the vascular access device, and an associated user interface.

Thus, in one aspect, an easy-to-use graphical interface is presented which is based on the automatic real-time detection of various parameters and regions of interest in signals related to endovascular ECG energy levels. For example, in the case of using endovascular ECG measurements for endovascular electrical energy computations, elements of the ECG signal can be automatically detected in real time, including the R-peak, the P-wave segment, the P-peak portions of an ECG waveform. These elements are highlighted in one embodiment using various graphical markers in order to allow an user to easily identify them, assess their characteristics, and follow their changes in real time.

In another aspect, the calculation of non-directional energy in an ECG signal complex is disclosed. In one embodiment, non-directional energy is calculated from an ECG signal corresponding to at least one heart beat of the patient, i.e., between two successive R-peaks bounding an ECG waveform complex. In one embodiment, the non-directional energy over this interval is calculated as: $E_{RR} = \text{Sum}_{RR} A^2 / \# \text{ of Samples}_{RR}$. In this context, the non-directional energy is calculated using all portions of the complex irrespective of the polarity of the portion, i.e., negative portions where the energy value is below the ECG signal baseline and positive portions where the energy value is above the ECG signal baseline.

The calculated non-directional energy values of one or more complexes of the ECG signal can be used to determine the location within the vasculature of the catheter or other vascular access device. Such a method can be readily implemented in an algorithm and automated for use by a suitable ECG monitoring and/or ECG-based tracking system. Further, the method can take into account changes in the electrical activity over the entirety of the complex, thus providing a reliable, "time-integrated" energy value calculation. In addition, the method eliminates the need for identifying a sub-segment of the ECG complex, such as the P-wave or the R-wave, and also can be used in situations where the P-wave is not easily identifiable, such as patients with arrhythmia.

In another aspect, the calculation of directional energy in an ECG signal complex is disclosed. In particular, the positive directional energy includes the sum of the squared amplitudes of portions of an ECG waveform complex where the energy value is above the ECG signal baseline. Similarly, the negative directional energy includes the sum of the squared amplitudes of portions of the ECG waveform complex where the energy value is below the ECG signal baseline. This can be applied to the P-wave segment of the ECG waveform complex such that the positive and negative directional energies and their respective ratio serve as an additional indicator of the location within the vasculature of a portion of a vascular access device, such as catheter tip relative to the sino-atrial node (SAN) of the patient's heart from where ECG signals emanate.

Specifically in one embodiment, substantially positive directional energy values indicate a catheter tip position superior to the SAN, while substantially negative directional energy values indicate a catheter tip position inferior to the SAN. If substantial portions of both positive and negative directional energy values are present, this indicates that the catheter tip is proximate to the right atrium. Note that the information provided by the directional energy of the P-wave segment of the ECG waveform complex as discussed above can also be provided by analysis of the amplitude of the P-wave, in one embodiment. Use of the ratio of positive and negative directional energy (or amplitude) of the P-wave segment can also be employed to clarify catheter tip position in situations where other tip location modalities may indicate that the tip can be positioned at either of two possible locations, such as the SVC or the IVC, for instance. Thus the sign (positive or negative) of the P-wave segment net directional energy value can be used to distinguish between SVC and IVC, wherein the net energy is positive for SVC placement and negative for IVC placement. Note that an additional method for distinguishing between tip placement in the IVC vs. the SVC includes the inserted catheter length, wherein the locations in the SVC and IVC where the non-directional energy (discussed further above) has a similar value are typically about 10 cm apart.

Note that practice of the system and methods to be described below enables a user to focus only on the features of the ECG signal waveform that are relevant to the location in the vasculature of the tip of the vascular access device. Also, the user can readily identify and follow the highlighted ECG signal features as they change over time.

FIG. 28 illustrates various elements of a graphical user interface for use with an apparatus, such as the apparatus (100) shown in FIG. 1A, for facilitating guidance of an indwelling catheter or other suitable vascular access device through a vasculature of a patient so as to position a tip thereof in a desired location therein, according to one embodiment. Note that as used herein, "indwelling" includes a position wherein at least a portion of the medical device is disposed within the body of the patient.

As shown, the graphical user interface (GUI) includes various highlighted signal features of interest for two waveforms: both an endovascular ECG signal (3560) displayed on the interface and a skin ECG signal (3570). The graphical user interface further includes various energy values calculated from the endovascular and skin signal waveforms of the ECG signals (3560) and (3570), as will be described. Note that the endovascular and skin ECG signals (3560) and (3570) respectively represent waveforms as detected by an electrode disposed proximate a tip of a vascular access device, such as a catheter, disposed at a point within the patient's vasculature outside the thoracic cavity region, and a skin reference electrode on the patient's skin.

In greater detail, the ECG signal (3570) represents a skin ECG control lead, typically associated with lead III, as described further above. The ECG signal (3560) represents an endovascular ECG signal, typically associated with lead II, measured at the tip of a catheter or other suitable vascular access device disposed in the vasculature outside the thoracic cavity region. An R-peak indicator (3505) highlights an R-peak of both ECG signals (3560) and (3570) for each waveform complex and is automatically computed by the apparatus 100 (FIG. 1A) and displayed in real-time to enable a user to readily identify the R-peak of each ECG signal. Note that the ECG signals (3560) and (3570) as depicted in the GUI are time-synchronized and each include a plurality of successive waveform complexes, each complex including a P, Q, R, & S-wave element generally corresponding to a single heartbeat, though other sampling directions can also be employed. Also note that the R-peak indicator (3505) (as with the other indicators described herein) marks the R-peak of each complex, though only select waveforms may be marked, if desired.

A P-peak indicator (3510) highlights a P-peak of both ECG signals (3560) and (3570) for each waveform complex therein and is automatically computed by the apparatus (100) and displayed in real-time to enable a user to readily identify the P-peak of each waveform complex. Also, a P-wave segment indicator (3515) highlights a P-wave of both ECG signal (3560) and (3570) for each waveform complex therein and is automatically computed by the apparatus (100) and displayed in real-time to indicate the P-wave portion of each complex of the displayed ECG signal waveforms. Note that the ECG signals as depicted by signal waveforms (3560) and (3570) on the GUI are continually updated as detected by the skin and endovascular electrodes during operation of the apparatus (100). As such, the graphical user interface is continually refreshed during operation of the apparatus (100) so as to depict the ECG signals in real time.

In one embodiment, the highlighted P-wave segment indicator (3515) is determined as follows. The waveforms corresponding to the ECG signals (3560) and 3570 are captured and stored by a processor or other suitable component of the apparatus (100) for at least two seconds, in one embodiment. This is sufficient to capture ECG signals relating to at least one heartbeat of the patient such that at least two consecutive R-peaks bounding a single ECG waveform complex is stored. The points in time of each of the two consecutive R-peaks are marked and the number of samples over which the ECG waveform complex was detected between the two consecutive R-peaks is calculated.

With respect to the presently captured waveform complex extending between the consecutive R-peaks, the P-wave segment is considered by the apparatus (100) in one embodiment to start a predetermined time after the first of the two consecutive R-peaks. Similarly, the P-wave segment is considered to stop a predetermined time before the second of the two consecutive R-peaks. In another embodiment, the start and stop points of the P-wave segment in the present waveform complex are calculated as a percentage of the length of the complex extending between the R-peaks (the R-R interval). For example, the P-wave segment in one embodiment can start at 70% of the R-R interval after the first R-peak and end at 5% of the R-R interval before the second R-peak. This information can be natively included in or programmed into the processor, such as the computer module 140 or other computing portion, of the apparatus (100) so as to be used thereby during P-wave segment detection. Note that other methods of computation of the P-wave segment are possible, based for instance on the assumption that the P-wave segment represents a certain percentage of the R-R interval starting and ending at some predictable points in the R-R cycle. These and other suitable methods for identifying desired components of the waveform are therefore contemplated, as appreciated by one skilled in the art.

Once the P-wave segment has been detected, it can be highlighted on the GUI and appear as the P-wave segment indicator (3515). The detection of the P-wave segment as described above, as well as detection of the R-peak indicator (3505) and P-peak indicator (3510), is continuously repeated for each new waveform complex detected by the apparatus. As such, the GUI in the present embodiment depicts a series of indicators across the screen as the waveform complexes are continuously captured and analyzed to identify the aforementioned complex components.

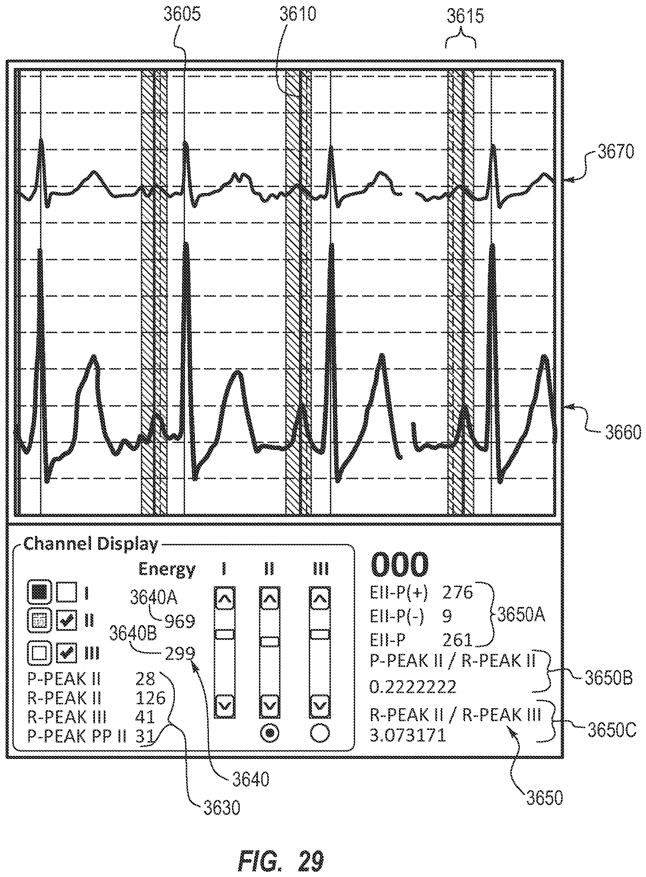

The above-described method for identifying and highlighting the P-wave segment can be applied to other ECG signals, including those depicted in FIGS. 29-35 herein, which are indicative of detection of ECG signals by the endovascular electrode of an indwelling catheter at different locations within the patient vasculature.

FIG. 28 further shows a field (3530) that displays various peak amplitude values for the respective R-peaks and P-peaks of the waveforms of the ECG signal (3560), captured by the reference skin electrode lead III, and the ECG signal (3570), captured by the endovascular catheter electrode lead II. The values included in the field (3530) and the other fields depicted in the GUI are calculated in the present embodiment by the computer module (140) of the apparatus (100) (FIG. 1A).

FIG. 28 also shows a field (3540) that displays the total energy levels per heartbeat, i.e., its corresponding waveform complex, detected on the ECG signal (3560) by lead II (shown at (3540A)) and on the ECG signal (3570) by lead III (shown at (3540B)). In the present embodiment, the total energy value, $E_{RR}$, for a waveform complex of either the endovascular ECG signal (3560) (shown at (3540A)) or the skin reference ECG signal (3570) (shown at (3540B)) is calculated using the following equation:

$$E_{RR} = \text{Sum}_{RR}\, A^2 \,/\, \text{\# of Samples}_{RR}, \qquad\qquad (1)$$

wherein the squares of the amplitudes of the complex at each discretely sampled portion of the complex between the two R-peaks are summed together, then divided by the number of sampled portions of the complex. Thus, in one example the apparatus (100) samples the ECG signal 100 times between successive R-peaks in order to ascertain the amplitude of the waveform complex at each of those sample points. Equation (1) would therefore include a summation of the square of the amplitudes of the waveform complex at each of the 100 sample points and would be divided by 100. This in turn yields a non-directional total energy value for the sampled waveform complex, wherein "non-directional" indicates that the total energy value is calculated by the apparatus 100 using all portions of the waveform complex irrespective of the polarity of the portion, i.e., negative portions where the energy value is below the ECG signal baseline and positive portions where the energy value is above the ECG signal baseline.

As mentioned further above, the calculated non-directional total energy values (3540A) of the endovascular ECG signal (3560) and (3540B) of the skin ECG signal (3570) for selected waveform complexes between consecutive R-peaks can be monitored by a user as the endovascular electrode at the tip of the catheter is advanced through the vasculature in order to assist the user in determining when the catheter tip has been placed in a desired intravascular position. Again, the method described above in connection with equation (1) can be readily implemented in an algorithm and automated for use by the computer module (140) of the apparatus (100), or other suitable ECG monitoring and/or ECG-based tracking system. Further, the method can take into account changes in the electrical activity over the entirety of the complex regardless of whether the complex contains positive and/or negative polarity portions, thus providing a reliable, "time-integrated" energy value calculation.

FIG. 28 further shows a field (3550) that displays various energy values and ratios related to the ECG signals (3560) and (3570). In particular, a P-wave field (3550A) is shown, including a positive directional energy value P(+) and a negative directional energy value P(-) and a resultant directional energy value (i.e., the difference of P(+) and P(-)) for a selected waveform complex, e.g., the current monitored heartbeat, of the endovascular lead II ECG signal (3560).

As described further above, the positive directional energy value P(+) includes the sum of the squared amplitudes of portions of the P-wave segment (identified by the P-wave segment indicator (3515)) of the current waveform complex, where the energy value is above the ECG signal baseline. Similarly, the negative directional energy value P(-) includes the sum of the squared amplitudes of portions of the P-wave segment (identified by the P-wave segment indicator (3515)) of the current waveform complex, where the energy value is below the ECG signal baseline.

Thus, the information contained in field (3550A) can serve as an additional indicator to the user of the location within the vasculature of the tip of the catheter, wherein in one embodiment, substantially positive directional energy values indicate a catheter tip position superior to the SAN, while substantially negative directional energy values indicate a catheter tip position inferior to the SAN. If substantial portions of both positive and negative directional energy values are present, this indicates that the catheter tip is proximate to the right atrium. Note that the information provided by the directional energy of the P-wave segment of the ECG waveform complex as discussed above can also be provided by analysis of the amplitude of the P-wave, in one embodiment.

Use of the ratio of positive to negative directional energy of the P-wave segment can also be employed to clarify catheter tip position in situations where other tip location methods, such as reference to the total energy values indicated in the total waveform complex energy field (3540), may indicate that the tip is positioned at either one of two possible locations, such as the SVC or the IVC, for instance. Thus the sign (positive or negative) of the net directional energy value of the P-wave segment as indicated in field (3550A) can be used to distinguish between SVC and IVC, wherein the net energy is positive for SVC placement and negative for IVC placement.

Note that an additional method for distinguishing between tip placement in the IVC vs. the SVC includes the inserted catheter length, wherein the locations in the SVC and IVC where the non-directional energy (discussed further above) has a similar value are typically about 10 cm apart.

Field (3550) further includes field (3550B), which depicts the ratio of the P-peak amplitude and the R-peak of the current endovascular waveform complex, and field (3550C), which depicts the ratio of the R-peak of the current endovascular waveform complex to the R-peak of the current skin waveform complex. These ratios, like the other energy and amplitude values depicted on the GUI, vary in time as the catheter tip including the endovascular electrode is advanced through the vasculature. Also, like the other energy and amplitude values depicted on the GUI, these ratios can be used by the user in determining proper catheter tip placement.

FIG. 29 illustrates various elements of the graphical user interface (GUI) for use with an apparatus, such as the apparatus (100) shown in FIG. 1A, for facilitating guidance of the catheter or other suitable vascular access device through the patient's vasculature, according to one embodiment. Note that many aspects of the GUI and the information depicted therein are similar to those aspects depicted in FIG. 28; as such, similar numbering conventions are used in FIG. 29 and subsequent figures, and only selected differences will be discussed herein.

As before, the GUI includes various highlighted signal features of interest for the two displayed waveforms, i.e., an endovascular ECG signal (3660) and a skin ECG signal (3670). The highlighted signal features include an R-peak indicator (3605), a P-peak indicator (3610), and a P-wave segment indicator (3615). The GUI of FIG. 29 further includes various energy values calculated from the endovascular and skin signal waveforms of the ECG signals (3660) and (3670), including the fields (3630), (3640), and (3650). The calculations to yield the information depicted in these fields are performed in a manner similar to that described above in connection with FIG. 28.

The ECG signals (3660) and (3670) and the energy and ratio data included in the fields (3630), (3640), and (3650) of FIG. 29 depict one example of waveforms and data detected and calculated when the endovascular electrode associated with the tip of the catheter is positioned in the upper SVC of the vasculature. As such, the information depicted on the GUI such as is seen in FIG. 29 can assist the user to determine when the catheter tip is located in the upper SVC of the patient vasculature.

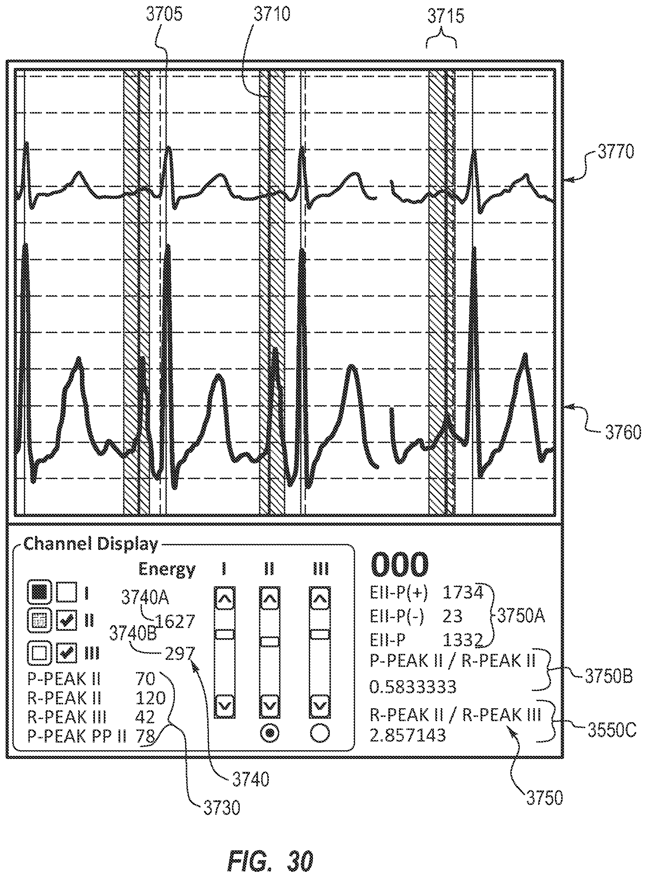

FIG. 30 illustrates various elements of the graphical user interface (GUI) for use with an apparatus, such as the apparatus (100) shown in FIG. 1A, for facilitating guidance of the catheter or other suitable vascular access device through the patient's vasculature, according to one embodiment. Note that many aspects of the GUI and the information depicted therein are similar to those aspects depicted in FIG. 28; as such, similar numbering conventions are used in FIG. 30, and only selected differences will be discussed herein.

As before, the GUI includes various highlighted signal features of interest for the two displayed waveforms, i.e., an endovascular ECG signal (3760) and a skin ECG signal (3770). The highlighted signal features include an R-peak indicator(3705), a P-peak indicator (3710), and a P-wave segment indicator (3715). The GUI of FIG. 30 further includes various energy values calculated from the endovascular and skin signal waveforms of the ECG signals (3760) and (3770), including the fields (3730), (3740), and (3750). The calculations to yield the information depicted in these fields are performed in a manner similar to that described above in connection with FIG. 28.

The ECG signals (3760) and (3770) and the energy and ratio data included in the fields (3730), (3740), and (3750) of FIG. 30 depict one example of waveforms and data detected and calculated when the endovascular electrode associated with the tip of the catheter is positioned in the lower $1/3^{rd}$ of the SVC of the vasculature. As such, the information depicted on the GUI such as is seen in FIG. 30 can assist the user to determine when the catheter tip is located in the lower $1/3^{rd}$ of the SVC of the patient vasculature.

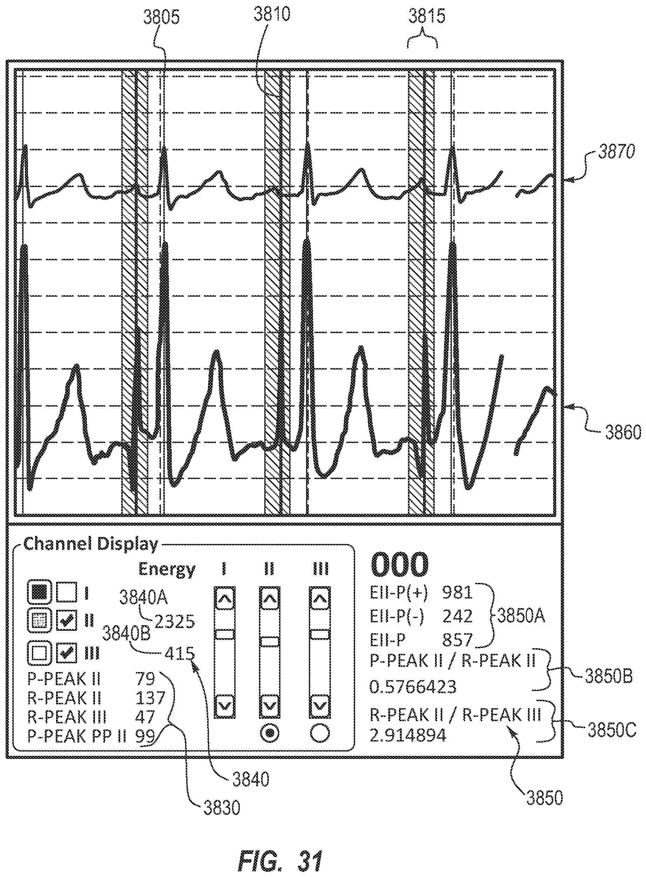

FIG. 31 illustrates various elements of the graphical user interface (GUI) for use with an apparatus, such as the apparatus (100) shown in FIG. 1A, for facilitating guidance of the catheter or other suitable vascular access device through the patient's vasculature, according to one embodiment. Note that many aspects of the GUI and the information depicted therein are similar to those aspects depicted in FIG. 28; as such, similar numbering conventions are used in FIG. 31, and only selected differences will be discussed herein.

As before, the GUI includes various highlighted signal features of interest for the two displayed waveforms, i.e., an endovascular ECG signal (3860) and a skin ECG signal (3870). The highlighted signal features include an R-peak indicator (3805), a P-peak indicator (3810), and a P-wave segment indicator (3815). The GUI of FIG. 31 further includes various energy values calculated from the endovascular and skin signal waveforms of the ECG signals (3860) and (3870), including the fields (3830), (3840), and (3850). The calculations to yield the information depicted in these fields are performed in a manner similar to that described above in connection with FIG. 28.

The ECG signals (3860) and (3870) and the energy and ratio data included in the fields (3830), (3840), and (3850) of FIG. 31 depict one example of waveforms and data detected and calculated when the endovascular electrode associated with the tip of the catheter is positioned in the CAJ of the vasculature. As such, the information depicted on the GUI such as is seen in FIG. 31 can assist the user to determine when the catheter tip is located in the CAJ of the patient vasculature.

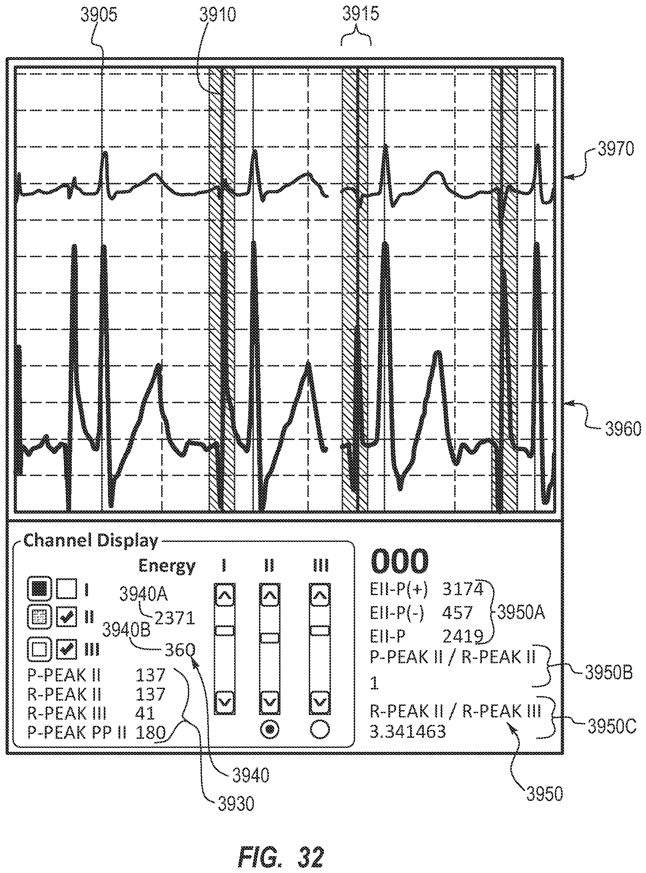

FIG. 32 illustrates various elements of the graphical user interface (GUI) for use with an apparatus, such as the apparatus (100) shown in FIG. 1A, for facilitating guidance of the catheter or other suitable vascular access device through the patient's vasculature, according to one embodiment. Note that many aspects of the GUI and the information depicted therein are similar to those aspects depicted in FIG. 28; as such, similar numbering conventions are used in FIG. 32, and only selected differences will be discussed herein.

As before, the GUI includes various highlighted signal features of interest for the two displayed waveforms, i.e., an endovascular ECG signal (3960) and a skin ECG signal (3970). The highlighted signal features include an R-peak indicator (3905), a P-peak indicator (3910), and a P-wave segment indicator (3915). The GUI of FIG. 32 further includes various energy values calculated from the endovascular and skin signal waveforms of the ECG signals (3960) and (3970), including the fields (3930), (3940), and (3950). The calculations to yield the information depicted in these fields are performed in a manner similar to that described above in connection with FIG. 28.

The ECG signals (3960) and (3970) and the energy and ratio data included in the fields (3930), (3940), and (3950) of FIG. 32 depict one example of waveforms and data detected and calculated when the endovascular electrode associated with the tip of the catheter is positioned in the RA of the vasculature. As such, the information depicted on the GUI such as is seen in FIG. 32 can assist the user to determine when the catheter tip is located in the RA of the patient vasculature.

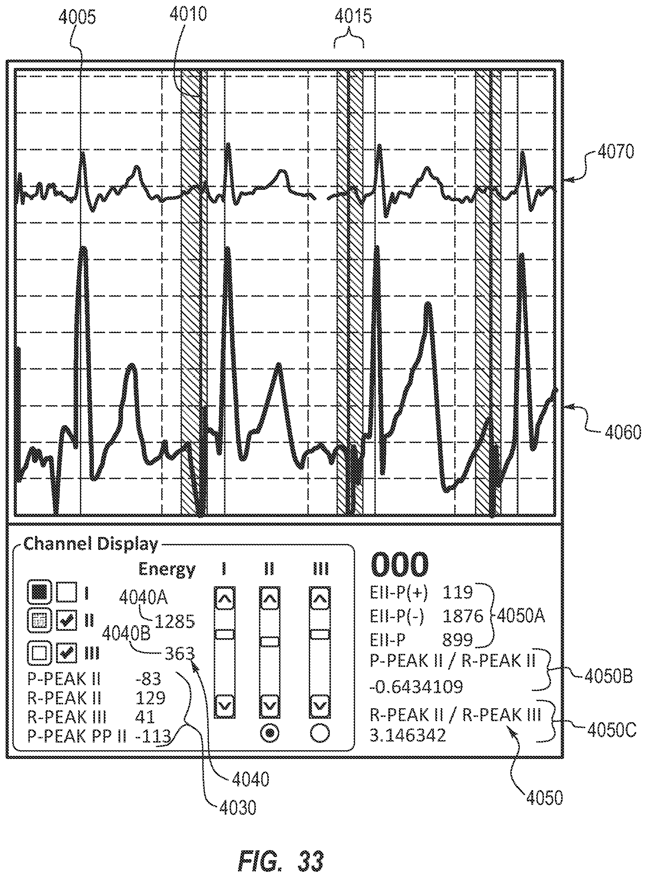

FIG. 33 illustrates various elements of the graphical user interface (GUI) for use with an apparatus, such as the apparatus (100) shown in FIG. 1A, for facilitating guidance of the catheter or other suitable vascular access device through the patient's vasculature, according to one embodiment. Note that many aspects of the GUI and the information depicted therein are similar to those aspects depicted in FIG. 28; as such, similar numbering conventions are used in FIG. 33, and only selected differences will be discussed herein.

As before, the GUI includes various highlighted signal features of interest for the two displayed waveforms, i.e., an endovascular ECG signal (4060) and a skin ECG signal (4070). The highlighted signal features include an R-peak indicator (4005), a P-peak indicator (4010), and a P-wave segment indicator (4015). The GUI of FIG. 33 further includes various energy values calculated from the endovascular and skin signal waveforms of the ECG signals (4060) and (4070), including the fields (4030), (4040), and (4050). The calculations to yield the information depicted in these fields are performed in a manner similar to that described above in connection with FIG. 28.

The ECG signals (4060) and (4070) and the energy and ratio data included in the fields (4030), (4040), and (4050) of FIG. 33 depict one example of waveforms and data detected and calculated when the endovascular electrode associated with the tip of the catheter is positioned in the IVC of the vasculature. As such, the information depicted on the GUI such as is seen in FIG. 33 can assist the user to determine when the catheter tip is located in the IVC of the patient vasculature.

Note that the negative directional energy value P(-) for the endovascular electrode as shown at field (4050A) is greater relative to the positive directional energy value P(+), indicative of the P-wave (highlighted by the P-wave segment indicator (4015)) including a large negative component below the ECG signal baseline for the P-wave of the endovascular ECG signal (4060), a fact that is borne out upon observation of the P-wave thereof. This fact can be employed in one embodiment to discern between catheter tip placement in the lower 1/3 of the SVC or the IVC, where the total energy values as depicted in the fields (3740A) and (3740B) (for the lower 1/3 SVC tip placement scenario shown in FIG. 30) and in the fields (4040A) and (4040B) (for the IVC tip placement scenario shown in FIG. 33) are often similar and do not alone always provide a clear indication of the catheter tip location. In addition to reference to the relatively greater negative directional energy value P(-) with respect to P(+), the user can observe that the total energy of the complex decreased as the IVC is approached by the endovascular electrode of the catheter tip, wherein an increase in total energy is observed as the SVC is approached.

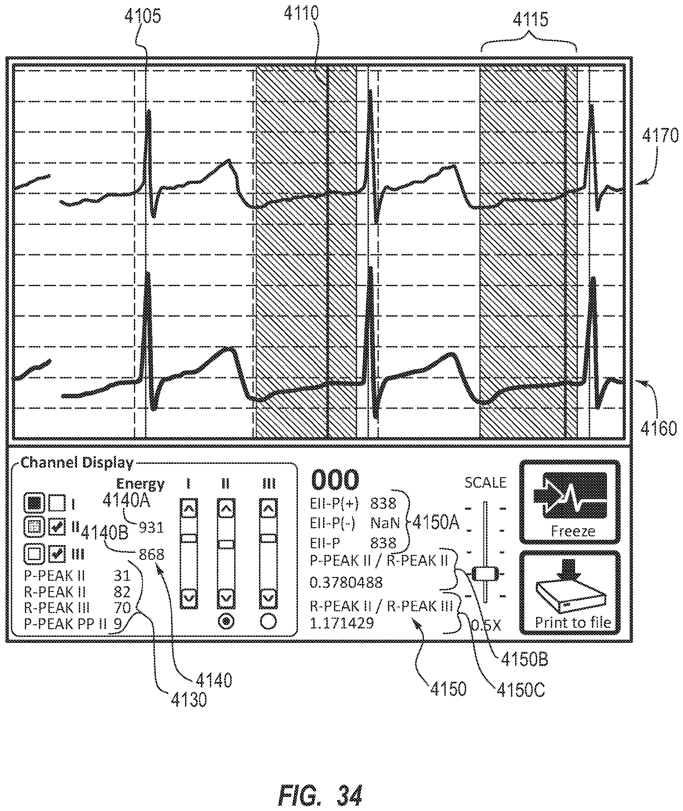

FIG. 34 illustrates various elements of the graphical user interface (GUI) for use with an apparatus, such as the apparatus (100) shown in FIG. 1A, for facilitating guidance of the catheter or other suitable vascular access device through the patient's vasculature, according to one embodiment. Note that many aspects of the GUI and the information depicted therein are similar to those aspects depicted in FIG. 28; as such, similar numbering conventions are used in FIG. 34, and only selected differences will be discussed herein.

As before, the GUI includes various highlighted signal features of interest for the two displayed waveforms, i.e., an endovascular ECG signal (4160) and a skin ECG signal (4170). The highlighted signal features include an R-peak indicator (4105), a P-peak indicator (4110), and a P-wave segment indicator (4115). The GUI of FIG. 34 further includes various energy values calculated from the endovascular and skin signal waveforms of the ECG signals (4160) and (4170), including the fields (4130), (4140), and (4150). The calculations to yield the information depicted in these fields are performed in a manner similar to that described above in connection with FIG. 28.

The ECG signals (4160) and (4170) and the energy and ratio data included in the fields (4130), (4140), and (4150) of FIG. 34 depict one example of waveforms and data detected and calculated for a patient with arrhythmia. As such, the information depicted on the GUI such as is seen in FIG. 34 can assist the user in placing the catheter tip as desired even in patients encountering arrhythmia.

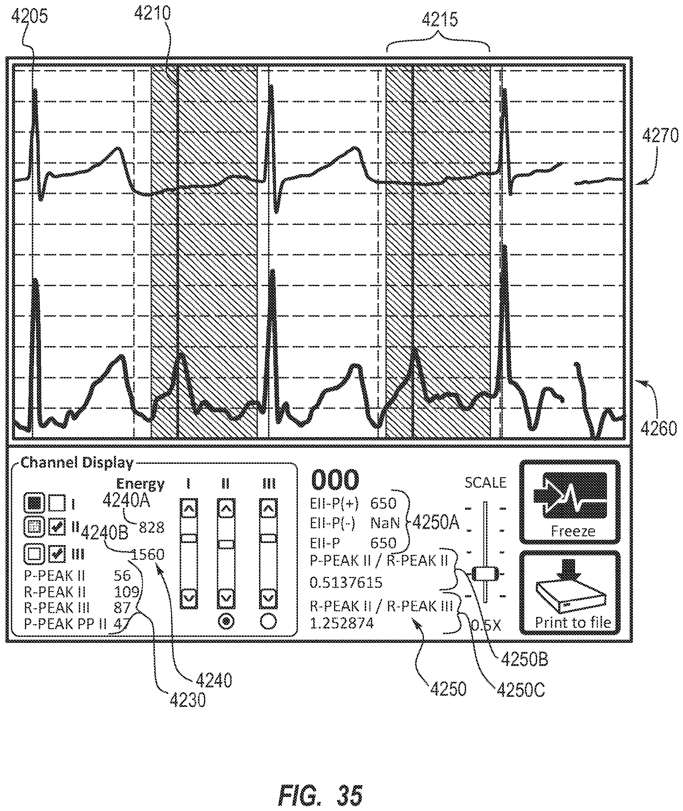

FIG. 35 illustrates various elements of the graphical user interface (GUI) for use with an apparatus, such as the apparatus (100) shown in FIG. 1A, for facilitating guidance of the catheter or other suitable vascular access device through the patient's vasculature, according to one embodiment. Note that many aspects of the GUI and the information depicted therein are similar to those aspects depicted in FIG. 28; as such, similar numbering conventions are used in FIG. 35, and only selected differences will be discussed herein.

As before, the GUI includes various highlighted signal features of interest for the two displayed waveforms, i.e., an endovascular ECG signal (4260) and a skin ECG signal (4270). The highlighted signal features include an R-peak indicator (4205), a P-peak indicator (4210), and a P-wave segment indicator (4215). The GUI of FIG. 35 further includes various energy values calculated from the endovascular and skin signal waveforms of the ECG signals (4260) and (4270), including the fields (4230), (4240), and (4250). The calculations to yield the information depicted in these fields are performed in a manner similar to that described above in connection with FIG. 28.

The ECG signals (4260) and (4270) and the energy and ratio data included in the fields (4230), (4240), and (4250) of FIG. 35 depict one example of waveforms and data detected and calculated for a patient with arrhythmia when the endovascular electrode associated with the tip of the catheter is positioned in the CAJ of the vasculature. As such, the information depicted on the GUI such as is seen in FIG. 35 can assist the user to determine when the catheter tip is located in the CAJ of the patient vasculature, even in patients encountering arrhythmia.

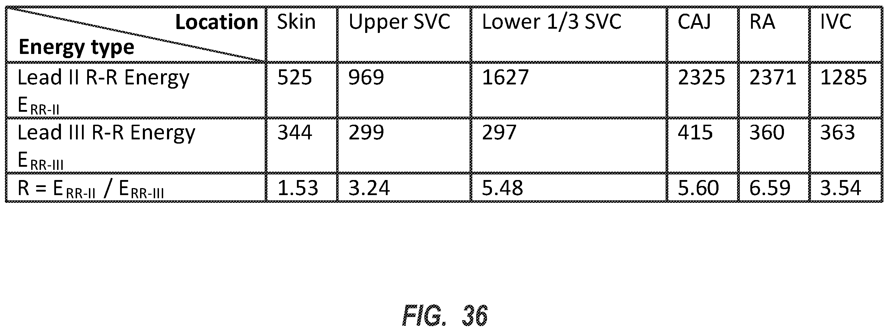

FIG. 36 shows a table including various energy values and ratios calculated for selected possible locations of the indwelling catheter within the patient vasculature. In particular, the table shows the total energy values of a waveform complex of the skin ECG signal (such as the ECG signal (3570) shown in FIG. 28) and of the endovascular ECG signal (such as the ECG signal (3560) shown in FIG. 28). The total energy values shown in the first two rows of the table correspond to the total energy values shown in the fields (3540A)...(4240A) and (3540B)...(4240B) of FIGS. 28-35, respectively.

The bottom row of the table shown in FIG. 36 shows the ratio of the total energy values of the above waveform complex for each selected location shown in the table. In the present embodiment, the ratio is calculated using the following equation:

$$R = E_{RR\text{-}II} \, / \, E_{RR\text{-}III.} \qquad (2)$$

As seen, this ratio employs total energy value ($E_{RR\text{-}II}$) of the waveform from the skin ECG signal (3570)...(4270) in order to provide a reference point for evaluating the endovascular total energy value of the waveform complex.

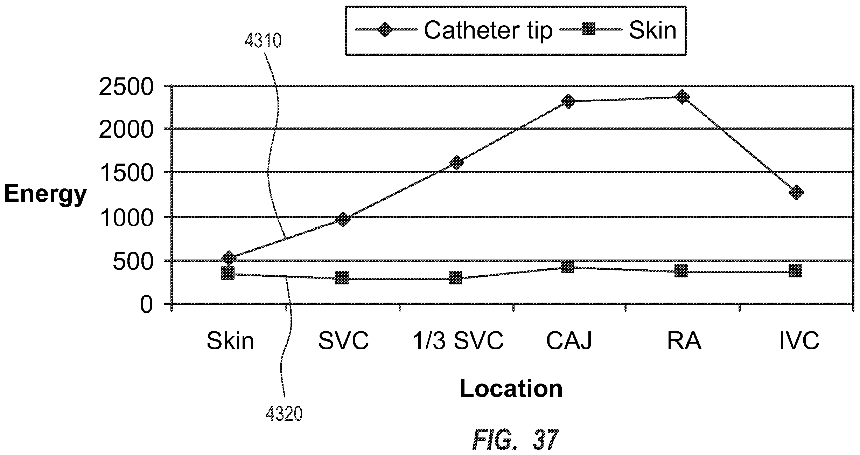

FIG. 37 shows a graph including an endovascular (catheter tip) ECG total energy plot (4310) and a skin ECG total energy plot (4320). In particular, the plot (4310) shows the total energy values of a waveform complex of the endovascular ECG signal (such as the ECG signal (3560) shown in FIG. 28) and the plot (4320) shows the total energy values of the waveform complex of the skin ECG signal (such as the ECG signal (3570) shown in FIG. 28) at selected possible locations of the catheter tip within the vasculature. The total energy values shown in the two plots (4310) and (4320) correspond to the total energy values shown in the fields (3540A)..(4240A) and (3540B)..(4240B) of FIGS. 28-35, respectively. As expected, the total energy reflected in the skin ECG signal as shown in plot (4320) remains substantially constant and independent of the catheter tip location during monitoring, with some possible light fluctuations being due to patient respiration during the catheter insertion procedure.

On the skin, as expected, the total energy as reflected in both plots (4310) and (4320) is of a similar value, with the endovascular energy value being slightly higher than that of the skin due to the particular orientation of lead II in the Einthoven triangle (FIG. 18A).

As the catheter tip advances from the insertion point towards the heart, the total energy detected at the tip thereof increases, as shown by the plot (4310). The total energy reaches a maximum value in the right atrium below the sino-atrial node and starts decreasing as the catheter tip advances beyond the right atrium and toward the inferior vena cava. It is therefore appreciated the total energy level measured by an endovascular electrode positioned proximate the tip of the catheter can be used to identify specific locations of the tip within the vasculature. It is thus seen that clear energy thresholds can be established between the different locations of interest.

Note that the total energy levels for each of the different locations shown in FIG. 37 are patient specific, and so comparison with a patient specific reference level is required in the present embodiment in order to accurately identify location of the catheter tip. For example, the energy level at the skin surface for the endovascular electrode could be used as a reference, with energy changes at the catheter tip being compared with this initial level. This in turn enables the same signal (from the endovascular lead II) to be used for reference and tip location estimation. However, this electrode is not operably connected to the apparatus (100) at the beginning of the procedure so as to be able to measure a skin-level measurement, and would be unable to measure the skin level energy after insertion into the vasculature, which skin energy level may change during the catheter insertion procedure due to patient respiration or movement.

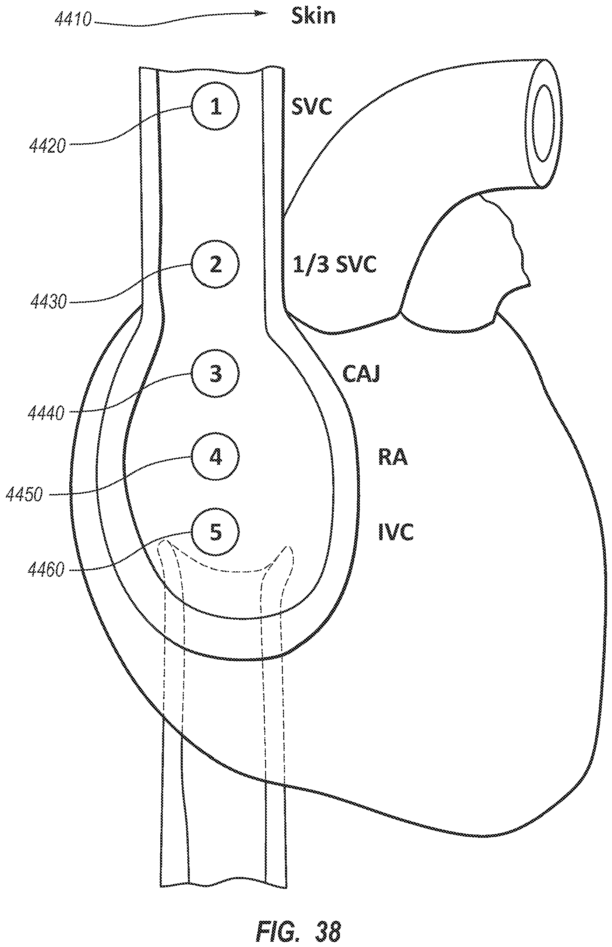

FIG. 38 graphically depicts selected possible locations for placement/location of the tip of a catheter or other vascular access device within the vasculature, including the skin at (4410), the SVC at (4420), the lower 1/3 of the SVC at (4430), the CAJ at (4440), the RA at (4450), the IVC at (4460). Of course, other locations can also be identified and used as desired placement locations for the methods described herein.

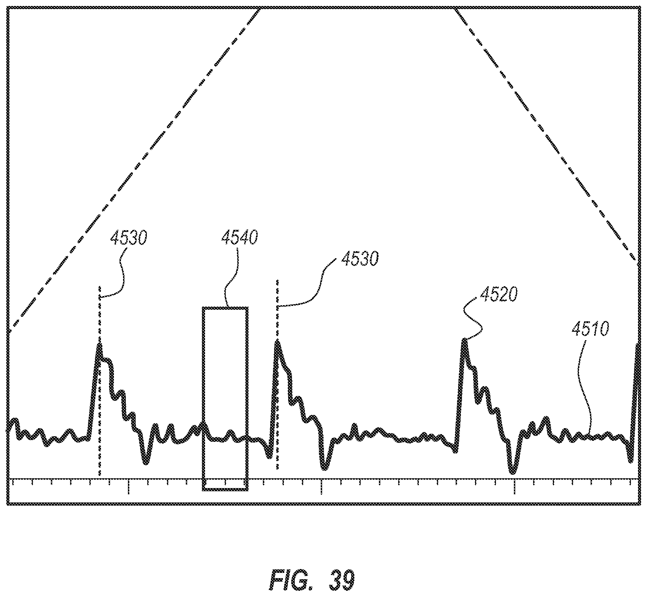

FIG. 39 shows various elements of the GUI not according to the invention, wherein an endovascular Doppler ultrasound signal is employed to locate a tip of a catheter or other medical device. As shown, an ultrasound signal (4510) is depicted, including the Doppler spectrum as measured at the tip of the catheter with a Doppler ultrasound probe or the like. Peaks (4530) represent a maximum in blood kinetic energy during a heart beat as measured by the Doppler ultrasound probe. The time interval between two designated peaks (4530) represents the time interval of a heart beat. Based on the peak activity of the heart cycle as detected by Doppler spectrum signal (4510), a region of interest (4540) can be defined corresponding to the atrial depolarization, i.e., a time interval during the heart beat equivalent to the P-wave. Based on energy calculations and changes in energy in the region (4540), the location of the catheter tip can be assessed. For example, a relatively high blood kinetic energy as detected by the Doppler ultrasound probe is indicative of location within the right atrium, in one embodiment. Note that in terms of the present disclosure dealing with ECG signals, that negative directional energy in the ultrasound example given here signifies the kinetic energy of blood flowing away from the catheter tip (i.e., a negative Doppler shift), and positive directional energy signifies the kinetic energy of blood flowing towards the catheter tip (i.e., a positive Doppler shift). Note further that the total energy, or non-directional energy, described further above in connection with ECG signals signifies in the ultrasound example here the kinetic energy measured by the Doppler ultrasound signal irrespective of the direction of the flow of the blood being measured.

In light of the above, it is therefore appreciated that in addition to ECG signals, other signal types, such as ultrasound (described above), or electromagnetic energy via magnetic or near-infrared signals can be employed in connection with the above-described energy mapping embodiments.

**Claims**

1. A computer implemented method for locating an indwelling medical device (110) within a vasculature of a patient, the method comprising:

    identifying an endovascular ECG waveform complex from an endovascular ECG signal associated with the indwelling medical device (110);
    calculating an absolute value of the energy ($E_{RR}$) of the endovascular ECG waveform complex of the endovascular ECG signal (3560) and the skin reference ECG signal (3570) over a predetermined segment thereof, using the equation

$$E_{RR} = Sum_{RR}\ A^2\ /\ \#\ of\ Samples_{RR},$$

    wherein the square of the amplitudes (A) of the complex at each discretely sampled portion of the complex between two R-peaks of the ECG waveform are summed together, then divided by the number of sampled portions of the complex; and
    determining a position of the medical device (110) within the vasculature by observation of the absolute value of the energy of the predetermined segment of the endovascular ECG waveform complex.

2. The method for locating as defined in claim 1,
    wherein the method is at least partially performed with an ECG signal monitoring system including a display for depicting the endovascular ECG signal, and wherein determining the position includes observing information relating to the absolute value of the energy of the endovascular ECG waveform complex as depicted on the display.

3. The method for locating as defined in claim 1,

    wherein the endovascular ECG waveform complex includes both positive energy values and negative energy values, and wherein identifying the endovascular ECG waveform complex includes identifying two successive peaks of an R-wave; and/or
    wherein the predetermined segment of the endovascular ECG waveform complex includes the entire complex corresponding to a complete single heartbeat of the patient.

4. The method for locating as defined in claim 1, wherein

    the endovascular ECG signal is detected by an electrode disposed proximate to a distal tip of the indwelling medical device; and/or

wherein the medical device is a catheter.

5. The method for locating as defined in claim 1, wherein calculating the absolute value of the energy further comprises:

    calculating the squares of the amplitudes of the endovascular ECG waveform complex at each discretely sampled portion of the complex over the predetermined segment;
    summing the squares of the amplitudes; and
    dividing the summed squares of the amplitudes by the number of discretely sampled portions of the complex.

6. The method for locating as defined in claim 1, wherein identifying the endovascular ECG waveform complex further comprises identifying an R-peak, a P-wave segment, and a P-peak of the endovascular ECG waveform complex.

7. The method for locating as defined in claim 1, further comprising:

    identifying a skin ECG waveform complex from a skin ECG signal sensed by an electrode on the skin of the patient; and
    calculating an absolute value of the energy of the skin ECG waveform complex over a predetermined segment time synchronized with the predetermined segment of the endovascular ECG waveform complex,
    wherein determining the position of the medical device includes determining the position of the medical device based on a ratio of the absolute value of the energy of the predetermined segment of the endovascular ECG waveform complex to the absolute value of the energy of the predetermined segment of the skin ECG waveform.

8. A system for tracking a location of a medical device within a vasculature of a patient, the system comprising:

    an endovascular electrode disposed on the medical device (110) and capable of detecting an endovascular ECG signal;
    a display for depicting an image of the endovascular ECG signal; **characterized by**
    a module (90, 140) including a processor capable of identifying an endovascular ECG waveform complex of the endovascular ECG signal and calculating an absolute value of the energy ($E_{RR}$) of at least a portion of the endovascular ECG waveform complex of the endovascular ECG signal (3560) and the skin reference ECG signal (3570), using the equation

$$E_{RR} = Sum_{RR} A^2 \; / \; \text{\# of Samples}_{RR,}$$

    wherein the square of the amplitudes (A) of the complex at each discretely sampled portion of the complex between two R-peaks of the ECG waveform are summed together, then divided by the number of sampled portions of the complex, in order to enable determination of a position of the medical device (110) within the vasculature.

9. The system for tracking as defined in claim 8 wherein the system includes a display for depicting the endovascular ECG signal and information relating to the absolute value the energy of the portion of the endovascular ECG waveform complex to enable a clinician to determine the location within the vasculature of the medical device.

10. The system as defined in claim 8, wherein the processor is further capable of identifying a P-wave segment in the endovascular ECG waveform complex, calculating a positive energy value relating to the amount of energy of the P-wave segment above a baseline of the endovascular ECG signal, and calculating a negative energy value relating to the amount of energy of the P-wave segment below a baseline of the endovascular ECG signal in order to assist in determining the location of the medical device within the vasculature.

11. The system for tracking as defined in claim 8, wherein the endovascular ECG signal is detected via a lead II configuration of Einthoven's triangle, and wherein a skin ECG signal is detected by the system via a lead III configuration of Einthoven's triangle.

**Patentansprüche**

1. Computerumgesetztes Verfahren zum Orten einer medizinischen Dauervorrichtung (110) innerhalb eines Gefäßsystems eines Patienten, wobei das Verfahren umfasst:

Identifizieren eines endovaskulären EKG-Wellenformkomplexes aus einem endovaskulären EKG-Signal, das mit der medizinischen Dauervorrichtung (110) assoziiert ist;

Berechnen eines Absolutwertes der Energie (ERR) des endovaskulären EKG-Wellenformkomplexes des endovaskulären EKG-Signals (3560) und des Hautreferenz-EKG-Signals (3570) während eines vorbestimmten Segments davon unter Verwenden der Gleichung

$$E_{RR} = \text{Sum}_{RR}\, A^2\, /\, \#\, \text{von Abtastungen}_{RR},$$

wobei das Quadrat der Amplituden (A) des Komplexes an jedem diskret abgetasteten Abschnitt des Komplexes zwischen zwei R-Spitzen der EKG-Wellenform summiert, dann durch die Anzahl abgetasteter Abschnitte des Komplexes dividiert wird; und

Bestimmen einer Position der medizinischen Vorrichtung (110) innerhalb des Gefäßsystems durch Beobachtung des Absolutwertes der Energie des vorbestimmten Segments des endovaskulären EKG-Wellenformkomplexes.

2. Verfahren zum Orten nach Anspruch 1,
wobei das Verfahren mindestens teilweise mit einem EKG-Signalüberwachungssystem ausgeführt wird, das eine Anzeige zum Abbilden des endovaskulären EKG-Signals beinhaltet, und wobei das Bestimmen der Position das Beobachten von Informationen hinsichtlich des Absolutwertes der Energie des endovaskulären EKG-Wellenformkomplexes, wie auf der Anzeige abgebildet, beinhaltet.

3. Verfahren zum Orten nach Anspruch 1,

wobei der endovaskuläre EKG-Wellenformkomplex sowohl positive Energiewerte als auch negative Energiewerte beinhaltet, und wobei das Identifizieren des endovaskulären EKG-Wellenformkomplexes das Identifizieren zweier aufeinanderfolgender Spitzen einer R-Welle beinhaltet; und/oder
wobei das vorbestimmte Segment des endovaskulären EKG-Wellenformkomplexes den gesamten Komplex beinhaltet, der einem vollständigen einzelnen Herzschlag des Patienten entspricht.

4. Verfahren zum Orten nach Anspruch 1,

wobei das endovaskuläre EKG-Signal von einer Elektrode erfasst wird, die nahe einer distalen Spitze der medizinischen Dauervorrichtung angeordnet ist; und/oder
wobei die medizinische Vorrichtung ein Katheter ist.

5. Verfahren zum Orten nach Anspruch 1, wobei das Berechnen des Absolutwertes der Energie weiter Folgendes umfasst:

Berechnen der Quadrate der Amplituden des endovaskulären EKG-Wellenformkomplexes an jedem diskret abgetasteten Abschnitt des Komplexes über das vorbestimmte Segment;
Summieren der Quadrate der Amplituden; und
Dividieren der summierten Quadrate der Amplituden durch die Anzahl diskret abgetasteter Abschnitte des Komplexes.

6. Verfahren zum Orten nach Anspruch 1, wobei das Identifizieren des endovaskulären EKG-Wellenformkomplexes weiter das Identifizieren einer R-Spitze, eines P-Wellensegments und einer P-Spitze des endovaskulären EKG-Wellenformkomplexes umfasst.

7. Verfahren zum Orten nach Anspruch 1, das weiter Folgendes umfasst:

Identifizieren eines Haut-EKG-Wellenformkomplexes aus einem Haut-EKG-Signal, das von einer Elektrode auf der Haut des Patienten erfasst wird; und
Berechnen eines Absolutwertes der Energie des Haut-EKG-Wellenformkomplexes über eine vorbestimmte Segmentzeit, die mit dem vorbestimmten Segment des endovaskulären EKG-Wellenformkomplexes synchronisiert ist,
wobei das Bestimmen der Position der medizinischen Vorrichtung das Bestimmen der Position der medizinischen Vorrichtung basierend auf einem Verhältnis des Absolutwertes der Energie des vorbestimmten Segments des endovaskulären EKG-Wellenformkomplexes zu dem Absolutwert der Energie des vorbestimmten Segments der Haut-EKG-Wellenform beinhaltet.

8. System zum Verfolgen eines Orts einer medizinischen Vorrichtung innerhalb eines Gefäßsystems eines Patienten, wobei das System umfasst:

eine endovaskuläre Elektrode, die auf der medizinischen Vorrichtung (110) angeordnet und dazu in der Lage ist, ein endovaskuläres EKG-Signal zu erfassen;
eine Anzeige zum Abbilden eines Bildes des endovaskulären EKG-Signals; **gekennzeichnet durch**
ein Modul (90, 140), das einen Prozessor beinhaltet, der in der Lage ist, einen endovaskulären EKG-Wellenformkomplex des endovaskulären EKG-Signals zu identifizieren, und Berechnen eines Absolutwertes der Energie (ERR) mindestens eines Abschnitts des endovaskulären EKG-Wellenformkomplexes des endovaskulären EKG-Signals (3560) und des Hautreferenz-EKG-Signals (3570) unter Verwenden der Gleichung $E_{RR} = Sum_{RR}\, A^2 / \#$ von Abtastungen$_{RR}$,
wobei das Quadrat der Amplituden (A) des Komplexes an jedem diskret abgetasteten Abschnitt des Komplexes zwischen zwei R-Spitzen der EKG-Wellenform summiert wird, dann durch die Anzahl abgetasteter Abschnitte des Komplexes dividiert wird, um das Bestimmen einer Position der medizinischen Vorrichtung (110) innerhalb des Gefäßsystems zu ermöglichen.

9. System zum Verfolgen nach Anspruch 8, wobei das System eine Anzeige zum Abbilden des endovaskulären EKG-Signals und von Informationen hinsichtlich des Absolutwertes der Energie des Abschnitts des endovaskulären EKG-Wellenformkomplexes beinhaltet, um es einem Mediziner zu ermöglichen, den Ort der medizinischen Vorrichtung innerhalb des Gefäßsystems zu bestimmen.

10. System nach Anspruch 8, wobei der Prozessor weiter in der Lage ist, ein P-Wellensegment in dem endovaskulären EKG-Wellenformkomplex zu identifizieren, einen positiven Energiewert hinsichtlich der Menge an Energie des P-Wellensegments oberhalb einer Basislinie des endovaskulären EKG-Signals zu berechnen, und einen negativen Energiewert hinsichtlich des P-Wellensegments unterhalb einer Basislinie des endovaskulären EKG-Signals zu berechnen, um das Bestimmen des Orts der medizinischen Vorrichtung innerhalb des Gefäßsystems zu unterstützen.

11. System zum Verfolgen nach Anspruch 8, wobei das endovaskuläre EKG-Signal über eine Konfiguration mit 2 Ableitungen des Einthoven-Dreiecks erfasst wird, und wobei ein Haut-EKG-Signal von dem System über eine Konfiguration mit 3 Ableitungen des Einthoven-Dreiecks erfasst wird.


**Revendications**

1. Procédé mis en oeuvre par ordinateur pour mettre en place un dispositif médical à demeure (110) dans une vasculature d'un patient, le procédé comprenant :

l'identification d'un complexe de forme d'onde d'ECG endovasculaire à partir d'un signal d'ECG endovasculaire associé au dispositif médical à demeure (110) ;
le calcul d'une valeur absolue de l'énergie (ERR) du complexe de forme d'onde d'ECG endovasculaire du signal d'ECG endovasculaire (3560) et du signal d'ECG de référence de peau (3570) sur un segment prédéterminé de celui-ci, à l'aide de l'équation

$$E_{RR} = Somme_{RR}\, A^2 / \#\ d'\text{Échantillons}_{RR},$$

dans lequel le carré des amplitudes (A) du complexe au niveau de chaque partie distinctement échantillonnée du complexe entre deux pics R de la forme d'onde d'ECG sont additionnés ensemble, puis divisés par le nombre de parties échantillonnées du complexe ; et
la détermination d'une position du dispositif médical (110) dans la vasculature par observation de la valeur absolue de l'énergie du segment prédéterminé du complexe de forme d'onde d'ECG endovasculaire.

2. Procédé de mise en place selon la revendication 1,

dans lequel le procédé est au moins partiellement effectué avec un système de surveillance de signal d'ECG incluant un afficheur pour représenter le signal d'ECG endovasculaire, et dans lequel la détermination de la position inclut l'observation d'informations concernant la valeur absolue de l'énergie du complexe de forme d'onde d'ECG endovasculaire tel que représenté sur l'afficheur.

3. Procédé de mise en place selon la revendication 1,

dans lequel le complexe de forme d'onde d'ECG endovasculaire inclut à la fois des valeurs d'énergie positives et des valeurs d'énergie négatives, et dans lequel l'identification du complexe de forme d'onde d'ECG endovasculaire inclut l'identification de deux pics successifs d'une onde R ; et/ou
dans lequel le segment prédéterminé du complexe de forme d'onde d'ECG endovasculaire inclut le complexe entier correspondant à un battement de coeur simple complet du patient.

4. Procédé de mise en place selon la revendication 1,

dans lequel le signal d'ECG endovasculaire est détecté par une électrode disposée à proximité d'une pointe distale du dispositif médical à demeure ; et/ou
dans lequel le dispositif médical est un cathéter.

5. Procédé de mise en place selon la revendication 1, dans lequel le calcul de la valeur absolue de l'énergie comprend en outre :

le calcul des carrés des amplitudes du complexe de forme d'onde d'ECG endovasculaire au niveau de chaque partie distinctement échantillonnée du complexe sur le segment prédéterminé ;
la somme des carrés des amplitudes ; et
la division des carrés totalisés des amplitudes par le nombre des parties distinctement échantillonnées du complexe.

6. Procédé de mise en place selon la revendication 1, dans lequel l'identification du complexe de forme d'onde d'ECG endovasculaire comprend en outre l'identification d'un pic R, d'un segment d'onde P et d'un pic P du complexe de forme d'onde d'ECG endovasculaire.

7. Procédé de mise en place selon la revendication 1, comprenant en outre :

l'identification d'un complexe de forme d'onde d'ECG de peau à partir d'un signal d'ECG de peau détecté par une électrode sur la peau du patient ; et
le calcul d'une valeur absolue de l'énergie du complexe de forme d'onde d'ECG de peau sur un temps de segment prédéterminé synchronisé avec le segment prédéterminé du complexe de forme d'onde d'ECG endovasculaire,
dans lequel la détermination de la position du dispositif médical inclut la détermination de la position du dispositif médical sur la base d'un rapport de la valeur absolue de l'énergie du segment prédéterminé du complexe de forme d'onde d'ECG endovasculaire sur la valeur absolue de l'énergie du segment prédéterminé de la forme d'onde d'ECG de peau.

8. Système de suivi de la mise en place d'un dispositif médical dans une vasculature d'un patient, le système comprenant :

une électrode endovasculaire disposée sur le dispositif médical (110) et capable de détecter un signal d'ECG endovasculaire ;
un afficheur pour représentée une image du signal d'ECG endovasculaire ; **caractérisé par**
un module (90, 140) incluant un processeur capable d'identifier un complexe de forme d'onde d'ECG endovasculaire du signal d'ECG endovasculaire et de calculer une valeur absolue de l'énergie (ERR) d'au moins une partie du complexe de forme d'onde d'ECG endovasculaire du signal d'ECG endovasculaire (3560) et du signal d'ECG de référence de peau (3570), à l'aide de l'équation

$$E_{RR} = \text{Somme}_{RR}\, A^2 \,/\, \#\, \text{d'Échantillons}_{RR,}$$

dans lequel le carré des amplitudes (A) du complexe au niveau de chaque partie distinctement échantillonnée du complexe entre deux pics R de la forme d'onde d'ECG sont additionnés ensemble, puis divisés par le nombre de parties échantillonnées du complexe, afin de permettre la détermination d'une position du dispositif médical (110) dans la vasculature.

9. Système de suivi selon la revendication 8 dans lequel le système inclut un afficheur pour représenter le signal d'ECG endovasculaire et des informations concernant la valeur absolue de l'énergie de la partie du complexe de forme d'onde d'ECG endovasculaire pour permettre à un médecin de déterminer l'emplacement dans la vasculature du dispositif médical.

10. Système selon la revendication 8, dans lequel le processeur est en outre capable d'identifier un segment d'onde P dans le complexe de forme d'onde d'ECG endovasculaire, de calculer une valeur d'énergie positive concernant la quantité d'énergie du segment d'onde P au-dessus d'une ligne de base du signal d'ECG endovasculaire, et de calculer une valeur d'énergie négative concernant la quantité d'énergie du segment d'onde P sous une ligne de base du signal d'ECG endovasculaire afin d'aider à la détermination de l'emplacement du dispositif médical dans la vasculature.

11. Système de suivi selon la revendication 8, dans lequel le signal d'ECG endovasculaire est détecté via une configuration de dérivation II de triangle d'Einthoven, et dans lequel un signal d'ECG de peau est détecté par le système via une configuration de dérivation III de triangle d'Einthoven.

*100*

VASCULAR
ACCESS
DEVICE — *110*

*115*

ADAPTOR

*120*

ELECTRONIC
MODULE — *130*

*131*

*132*

(WIRELESS)
CONNECTIVITY

*150*

COMPUTER
MODULE — *140*

PERIPHERALS

*160*

ALGORITHMS — *170*

GRAPHICAL
USER
INTERFACE — *180*

**FIG. 1A**

**FIG. 1B**

**FIG. 2**

FIG. 3

**FIG. 4A**

**FIG. 4B**

**FIG. 4C**

**FIG. 4D**

FIG. 5

FIG. 6

**FIG. 7**

FIG. 8

FIG. 9

FIG. 10A

FIG. 10B

INPUT SIGNAL 1100

DETECTION OF ERROR CONDITIONS
- Defibrillation detection
- Pacemaker detection
- Lead-off detection          1105

Error Handling

1110

PRE-PROCESSING          1115
- Scalable amplification
- Baseline correction (high-pass filter)
- Wideband noise suppression (wavelet transform, selective filter)
- Interference suppression (notch filter, narrow band, 50/60 Hz)
- High frequency noise suppression (low-pass filter, averaging)

Adaptive Filtering

1120

Fast Fourier Transform

1125

TIME DOMAIN
PATTERN RECOGNITION          1130
- R-Peak detection
- QRS detection (first derivative)
- R-R interval (auto-correlation)
- P-Peak and P pp detection
- T-Peak detection
- Calc PR and ST intervals
- Calc ratio P/R
- Determine P-wave polarity and type
- Calculate temporal averages of P-peak,
  R-peak, P/R ratio, R-R interval
- Determine P max, P pp max over time
- Determine ST elevation and T alternans
- P-wave analysis
- ST segment analysis
- Baseline subtraction
- Waveform averaging
- Signal energy computation
- First derivative computation
- Integral of first derivative

FREQUENCY DOMAIN
PATTERN RECOGNITION          1140
- Principal components analysis
- Determine if single, bi or multiple frequency
  spectrum
- Calculate signal energy E
- Calculate spectral power Pwr
- Determine maximum frequency
- Determine the frequency of last maximum
- Calculate average of s
  power, highest frequency over time
- Determine max energy E max and maximum
  spectral power Pwr max over time
- Calculate preload from energy at R-time
- P-wave analysis
- ST segment analysis
- DC component removal
- Selective filtering of noise/artifacts
- Signal reconstruction in time domain
- Inverse Fourier transform

DECISION SUPPORT ALGORITHMS 1-Based on P-Max at caval-atrial junction as reference
A: Upper SVC
    Ppp / Ppp max < T1 AND Pwr/Pwr max<T1 and unipolar P-wave, e.g., T1=0.4
B: Lower third of SVC
    T1<Ppp / Ppp max < T2 AND T1 < Pwr/Pwr max < T2 AND unipolar P-wave, e.g., T2=0.6
C: Caval-atrial junction
    Ppp>T3*Ppp max AND Pwr>T3*Pwr max AND P>R, .g., T3=0.9
D: Right Atrium
    T2<Pwr / Pwr max < T3 AND bipolar P-Wave          1150
E: Upper third of the IVC
    T1<Ppp / Pwr max < T2 AND T1 < Pwr / Pwr max < T2 ND unipolar P-wave WITH reversed polarity

OUTPUT LOCATION ON HEART MAP          1160

*FIG. 11*

EP 2 618 727 B1

**1250**

DECISION SUPPORT ALGORITHMS 1 - Based on skin ECG as reference

A: Upper SVC
$T1 < P\,pp / P\,pp\,skin < T2$ AND $T1 < Pwr / Pwr\,skin < T2$ and unipolar P-wave, e.g., $T1 = 0.9$, $T2 = 1.2$

B: Lower third of SVC
$T3 < P\,pp / P\,pp\,skin < T4$ AND $T3 < Pwr / Pwr\,skin < T4$ AND unipolar P-wave, e.g., $T3 = 1.5$, $T4 = 2$

C: Caval-atrial junction
$P\,pp > T5 * P\,pp\,skin$ AND $Pwr > T5 * Pwr\,skin$ AND $P > R$, e.g., $T5 = 2.5$

D: Right atrium
$T6 < Pwr / Pwr\,skin < T7$ AND bipolar P-wave, e.g., $T6 = 2$, $T7 = 2.5$

E: Upper third of the IVC
$T1 < P\,pp / P\,pp\,skin < T2$ AND $T1 < Pwr / Pwr\,skin < T2$ AND unipolar P-wave WITH reversed polarity

*FIG. 12*

FIG. 13

*FIG. 14*

*FIG. 15*

FIG. 16

FIG. 17

**FIG. 18A**

FIG. 18B

FIG. 18C

*FIG. 18D*

*FIG. 18E*

**FIG. 18F**

**FIG. 19A**

**FIG. 19B**

*FIG. 20A*

*FIG. 20B*

**FIG. 20C**

**FIG. 20D**

FIG. 20E

FIG. 20F

2510

2520

**FIG. 21A**

2510

2530

**FIG. 21B**

FIG. 22A

FIG. 22B

FIG. 22C

FIG. 22D

FIG. 23A

FIG. 23B

*FIG. 24A*

*FIG. 24B*

FIG. 24C

FIG. 24D

FIG. 24E

FIG. 24F

**FIG. 25A**

**FIG. 25B**

*FIG. 26*

*FIG. 27A*

*FIG. 27B*

EP 2 618 727 B1

FIG. 28

FIG. 29

FIG. 30

FIG. 31

FIG. 32

*FIG. 33*

**FIG. 34**

**FIG. 35**

| Location<br>Energy type | Skin | Upper SVC | Lower 1/3 SVC | CAJ | RA | IVC |
|---|---|---|---|---|---|---|
| Lead II R-R Energy $E_{RR-II}$ | 525 | 969 | 1627 | 2325 | 2371 | 1285 |
| Lead III R-R Energy $E_{RR-III}$ | 344 | 299 | 297 | 415 | 360 | 363 |
| $R = E_{RR-II} / E_{RR-III}$ | 1.53 | 3.24 | 5.48 | 5.60 | 6.59 | 3.54 |

*FIG. 36*

FIG. 37

*FIG. 38*

*FIG. 39*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009100158 A1
- US 20100036227
- US 20090259124
- US 20100222664
- US 20110015533 A